# EUROPEAN PATENT SPECIFICATION

(11) **EP 4 279 609 B1**
(45) Date of publication and mention of the grant of the patent: **08.04.2026**
(21) Application number: 21913962.3
(22) Date of filing: 17.12.2021
(51) Int. Cl.: C12Q 1/6806, C12Q 1/6869, C12N 15/10

(54) **METHOD AND KIT FOR LABELING NUCLEIC ACID MOLECULES**
VERFAHREN UND KIT ZUR MARKIERUNG VON NUKLEINSÄUREMOLEKÜLEN
PROCÉDÉ ET KIT DE MARQUAGE DE MOLÉCULES D'ACIDE NUCLÉIQUE

(30) Priority: 31.12.2020 CN 202011639159
(43) Date of publication of application: 22.11.2023
(73) Proprietor: Beijing Institute of Genomics, Chinese Academy of Sciences (China National Center for Bioinformation), Beijing 100101 (CN)
(72) Inventor: JIANG, Lan, Beijing 100101 (CN); LI, Yun, Beijing 100101 (CN)
(74) Representative: Plavsa, Olga
(86) International application number: PCT/CN2021/139123
(87) International publication number: WO 2022/143221

(56) References cited:
- WO-A2-2020/112604
- CN-A- 102 264 914
- CN-A- 109 207 572
- CN-A- 109 526 228
- US-A1- 2014 093 916
- CHARLES COLE ET AL: "Tn5Prime, a Tn5 based 5' capture method for single cell RNA-seq", NUCLEIC ACIDS RESEARCH, vol. 46, no. 10, 14 March 2018 (2018-03-14), GB, pages e62 - e62, XP055637367, ISSN: 0305-1048, DOI: 10.1093/nar/gky182
- DANIEL ALPERN ET AL: "Time- and cost-efficient high-throughput transcriptomics enabled by Bulk RNA Barcoding and sequencing", BIORXIV, 8 January 2019 (2019-01-08), XP055719233, Retrieved from the Internet <URL:https://www.biorxiv.org/content/10.1101/256594v2.full.pdf> [retrieved on 20200730], DOI: 10.1101/256594
- LU BO ET AL: "Transposase-assisted tagmentation of RNA/DNA hybrid duplexes", ELIFE, vol. 9, 23 July 2020 (2020-07-23), XP055892345, DOI: 10.7554/eLife.54919
- LAFZI ATEFEH ET AL: "Tutorial: guidelines for the experimental design of single-cell RNA sequencing studies", NATURE PROTOCOLS, NATURE PUBLISHING GROUP, GB, vol. 13, no. 12, 16 November 2018 (2018-11-16), pages 2742 - 2757, XP036643359, ISSN: 1754-2189, [retrieved on 20181116], DOI: 10.1038/S41596-018-0073-Y
- GEORGIA GIANNOUKOS ET AL: "UDiTaS(TM), a genome editing detection method for indels and genome rearrangements", BMC GENOMICS, BIOMED CENTRAL LTD, LONDON, UK, vol. 19, no. 1, 21 March 2018 (2018-03-21), pages 1 - 10, XP021254923, DOI: 10.1186/S12864-018-4561-9
- CHARLES COLE, ASHLEY BYRNE, ANNA E BEAUDIN, E CAMILLA FORSBERG, CHRISTOPHER VOLLMERS: "Tn5Prime, a Tn5 based 5′ capture method for single cell RNA-seq", NUCLEIC ACIDS RESEARCH, OXFORD UNIVERSITY PRESS, GB, vol. 46, no. 10, 1 June 2018 (2018-06-01), GB , pages e62 - e62, XP055637367, ISSN: 0305-1048, DOI: 10.1093/nar/gky182
- TANG JIANGTAO, ET AL.: "Research development of Tn5transposition mechanism", JI YIN ZU XUE YU YING YONG SHENG WU XUE [GENOMICS AND APPLIED BIOLOGY], CHINA, vol. 22, no. 4, 31 December 2003 (2003-12-31), China , pages 316 - 321, XP055947312, ISSN: 1008-3464
- SIMONE PICELLI, ÅSA K. BJÖRKLUND, BJÖRN REINIUS, SVEN SAGASSER, GÖSTA WINBERG, RICKARD SANDBERG: "Tn5 transposase and tagmentation procedures for massively scaled sequencing projects", GENOME RESEARCH, COLD SPRING HARBOR LABORATORY PRESS, US, vol. 24, no. 12, 1 December 2014 (2014-12-01), US , pages 2033 - 2040, XP055236186, ISSN: 1088-9051, DOI: 10.1101/gr.177881.114

## Description

### Field of the invention

This invention relates to transcriptome sequencing, in particular high-throughput single-cell transcriptome sequencing. in particular, the invention relates to a method for generating a pool of nucleic acid fragments by treating cells or cell nuclei, and using the generated nucleic acid fragments to create labeled nucleic acid molecules for constructing nucleic acid libraries for transcriptome sequencing or high-throughput sequencing of single-cell transcriptomes. Additionally, the invention relates to nucleic acid libraries constructed using the described method and reagent kits for implementing the method.

### Background of the invention

Cells, as the fundamental units of structure and function in living organisms, have posed a significant challenge in the study of their functionality and heterogeneity in the field of biology. The development of single-cell omics sequencing technologies has revolutionized our understanding of cellular diversity and heterogeneity, playing a crucial role in the advancement of various fields such as developmental biology, cancer research, assisted reproduction, immunology, neuroscience, and microbiology. Single-cell sequencing encompasses various omics techniques including single-cell genomics, transcriptomics, methylation sequencing, chromatin accessibility sequencing, and multi-omics sequencing that integrates the aforementioned information. Essentially, these techniques involve the analysis of DNA and RNA sequences, copy numbers, modifications, and interactions within individual cells, revealing genomic, transcriptomic, epigenomic, and chromatin accessibility changes in single cells.

Currently, single-cell transcriptome sequencing is the most widely applied technique for obtaining transcriptome information from individual cells at a specific moment. In summary, this method involves reverse transcription of the transcriptome within individual cells at a specific moment to obtain cDNA, amplifying the cDNA, constructing sequencing libraries, and performing sequencing to obtain the transcriptome information of specific cells. Single-cell transcriptome technology enables researchers to study cellular characteristics at the single-cell or subpopulation level, allowing for precise investigations into cell development, tumor microenvironments, and single-cell mapping. Since the establishment of the first single-cell transcriptome library construction technology in 2009, numerous methods for single-cell transcriptome library construction have emerged, evolving from low-throughput methods with low detectable gene numbers to methods with improved data quality and higher throughput. Researchers are striving for high-throughput and cost-effective single-cell sequencing techniques to enable more detailed characterization of cellular heterogeneity, analysis of single-cell gene regulatory networks, and comprehensive profiling of cell populations.

Currently, high-throughput single-cell transcriptome library construction techniques mainly include the following:
(1) The cell barcoding in microfluidic droplets is used in high-throughput single-cell transcriptome library construction. This category of library construction technology is represented by the in Drop system developed by David Weitz et al. from Harvard University in 2015 (Cell, 2015, 161(5): p. 1187-1201), the Drop-seq system (Cell, 2015, 161(5): p. 1202-1214), and the Chromium platform by 10X Genomics. The basic principle of these technologies is the generation of nanoliter-scale water in oil emulsions reaction systems in microfluidic systems, where individual cells are encapsulated with microbeads. Each microbead surface carries millions of barcoded polythymidine (PolyT) primers. The barcodes include cell-specific barcodes (cell barcodes) and unique molecular barcodes (UMIs) specific to each molecule on the microbeads. In a single operation, reverse transcription reactions can be performed simultaneously on thousands of cells, enabling single-cell and single-molecule labeling. The resulting cDNA is pooled and subjected to library construction and sequencing. The main disadvantages of this library construction technology are low cell throughput, high empty droplet rate in the micro-reaction system, low sample throughput, and high library construction cost.
(2) High-throughput single-cell transcriptome library construction technology using cell barcoding in microwell plates. This category of library construction technology is represented by the Seq-Well technology developed by Alex K Shalek et al. in 2017 (Nature Methods, 2017, 14(7): p. 752-752) and the Microwell-seq platform established by the Guo Guoji team at Zhejiang University in 2018 (Cell, 2018, 172(5): p. 1091-1107). The basic principle of these technologies involves the use of chips or microwell plates with hundreds of thousands of microwells as carriers, combined with magnetic beads carrying polythymidine (PolyT) and cell barcodes for single-cell capture. After adding the single-cell suspension to the microwell plate, the cells settle into the microwells. After washing away the unbound single-cell samples, magnetic beads with polythymidine and cell barcodes are added. Each microwell acts as an independent reaction chamber, and after cell lysis in the microwells, the RNA released from different cells in different wells is captured by the primers on the magnetic beads, labeled with different cell barcodes. Subsequently, the RNA-captured magnetic beads are transferred to an EP tube for reverse transcription, enabling simultaneous labeling of RNA molecules from thousands of cells, followed by cDNA amplification and library construction. The main disadvantages of this library construction technology are low cell throughput, low sample throughput, cumbersome operation process, and high library construction cost.
(3) High-throughput single-cell transcriptome library construction technology based on split-pool ligation-based transcriptome sequencing using the combination of separation and pooling of multiple rounds of labeling. This category of library construction technology is represented by the SPLiT-seq technology developed by A. B. Rosenberg's team in 2018 (Nature Methods, 2018, 15(12): p. 1126-1126). This technology does not rely on expensive microfluidic or microwell preparation equipment. Instead, it employs multiple rounds of ligation reactions to attach different combinations of barcodes to cells, enabling simultaneous library construction of tens of thousands of cells. The main disadvantages of this library construction technology are low cell throughput, cumbersome operation process, high library construction cost, and it has not been commercially applied.

After years of development, high-throughput single-cell transcriptome sequencing technology has made significant progress.

For example, early single-cell transcriptome studies primarily utilized mRNA captured from intact live cells, which had strict time requirements from sampling to library construction of fresh samples. This greatly limited the application of single-cell transcriptome sequencing technology. The emergence of single-cell nuclear transcriptome sequencing technology overcame this limitation. This technology enables the direct extraction of nuclei from frozen tissues and captures RNA from individual cell nuclei for sequencing, producing single-cell transcriptome data that is comparable to that obtained from intact cells. Single-cell nuclear transcriptome sequencing technology overcomes sample limitations and enables single-cell transcriptome studies on frozen samples, especially frozen clinical samples.

Additionally, in order to increase sample throughput (i.e., to perform transcriptome library construction and sequencing on cells from multiple sample sources in a single experiment), multiplexing strategies based on additional labels have been developed. In such methods, sample barcodes can be added to the samples in advance, and then the multiple samples can be mixed for library construction and sequencing. After sequencing, with the help of sample barcodes, the single-cell transcriptome information of multiple samples can be extracted from the sequencing data. Representative examples of high-throughput transcriptome library construction technology based on additional labels and microfluidic droplets include the Feature Barcoding technology developed by 10X Genomics using BioLegend's TotalSeq^{™} antibodies (Single Cell 3' Feature Barcode Library Kit, #PN-1000079; Single Cell 5' Feature Barcode Kit, #PN-1000256), as well as the MULTI-seq technology reported by Zev J. Gartner's team in 2019 (McGinnis, C.S., et al., Nature Methods, 2019, 16(7): p. 619-626). These technologies are based on microfluidic platforms such as 10X Genomics Chromium and Fluidigm C1. Before preparing single-cell droplets labeled with cell barcodes, each cell sample is individually subjected to a specific labeling round using different tags, and then the samples labeled with different tags are mixed to prepare the droplets. For example, in the library construction approach based on Feature Barcoding technology, TotalSeq^{™} antibodies that can specifically bind to different proteins on the cell membrane are coupled with oligonucleotides containing specific labels and sequences complementary to the bead barcode sequences of 10X Genomics. In this way, different cell samples can be prelabeled with different TotalSeq^{™} antibodies. These labeled samples can be mixed and subjected to standard 10X Genomics transcriptome library construction, followed by library enrichment using the Feature Barcoding kit and sequencing. With the specific labels introduced by TotalSeq^{™} antibodies, it is possible to determine the source of single-cell transcriptome data from each sample. In the library construction approach based on MULTI-seq technology, liposomes carrying specific oligonucleotide sequences that can selectively bind to the cell membrane are used to label different samples. These labeled samples are then mixed and subjected to transcriptome library construction and sequencing. With the Feature Barcoding technology or MULTI-seq technology, high-throughput transcriptome library construction and sequencing can be performed on multiple cells from different samples.

Furthermore, for high-throughput single-cell transcriptome library construction technologies that involve cell barcode labeling in microfluidic droplets or microwell plates, if a single droplet or microwell contains two or more cells, the sequencing results obtained from that droplet or microwell will not accurately reflect the transcriptome information of a single cell and cannot be used. Therefore, in the process of single-cell transcriptome library construction, it is necessary to avoid the occurrence of doublets or multiplets (i.e., avoiding the presence of two or more cells in a single droplet or microwell) as much as possible. Taking the standard technical approach of the 10X Genomics Chromium platform as an example, in order to control the multiplet rate within an acceptable range (e.g., 5%), it is typically recommended to limit the cell throughput to less than 10,000 cells per reaction. This means that the number of droplets containing reagents and beads produced in a single reaction is approximately 100,000, with an effective utilization rate of less than 10%. The majority of droplets are empty, without cells, resulting in significant waste. To address this issue, Christoph Bock's team developed the scifi-RNA-seq method (bioRxiv, 2019: p. 2019.12.17.879304). The library construction process of this method involves dividing the cell samples into multiple portions and performing reverse transcription on each portion of the cell sample separately using reverse transcription primers labeled with specific oligonucleotide sequences, thereby achieving the first-round labeling of nucleic acid molecules in each portion. Then, these samples are mixed and library construction is performed using the 10X Genomics scATAC kit, resulting in the second-round labeling of nucleic acid molecules in the library (including cell barcodes and unique molecular identifiers). With the combination of the first and second-round labels, this method can increase the cell throughput of the standard technical approach of the 10X Genomics Chromium platform by approximately 15 times. However, this method can only be used for 3'-end library construction, capturing information about the 3'-end of mRNA molecules in the transcriptome and cannot obtain information about the 5'-end of mRNA molecules.

Based on the difference in RNA information enriched in the library, high-throughput single-cell transcriptome sequencing library construction technology can be classified into two types: 5'-end library construction technology and 3'-end library construction technology used for transcriptome sequencing. Both library construction technologies can be used for non-full-length mRNA end sequencing, but they are two different techniques: the 3'-end library construction technology is used for enrichment and determination of the 3'-end information of transcriptome mRNA molecules, while the 5'-end library construction technology is used for enrichment and determination of the 5'-end information of transcriptome mRNA molecules, providing information about transcription start sites. These two technologies have different goals and are suitable for different scenarios. Currently, 10X Genomics has introduced different kits for these two library construction technologies: Chromium Next GEM Single Cell 3' GEM, Library & Gel Bead Kit, #PN-1000075; and Chromium Single Cell 5' Library & Gel Bead Kit, # PN-1000006.

T lymphocytes (T cells) and B lymphocytes (B cells) are primarily responsible for adaptive immune responses, relying on the T cell receptor (TCR) and B cell receptor (BCR) to recognize antigens. A common feature of these cell types is their diversity, allowing them to recognize a wide range of antigen molecules. The heavy chain of BCR and TCRβ chain consist of V, D, J, and C gene segments, while the light chain of BCR and TCRα chain consist of V, J, and C gene segments. These gene segments undergo genetic recombination and rearrangement during development, resulting in different combinations that ensure receptor diversity. VDJ sequencing can be used to explore immune mechanisms and uncover the relationship between immune repertoire and diseases. Since the VDJ region is located at the 5'-end of mRNA, the use of 5'-end library construction technology facilitates the enrichment of sequences spanning the full-length V(D)J region of T cell receptors (TCRs) and B cell receptors (BCRs).

Currently, the main drawbacks of commercial methods for 5'-end library construction technology used in transcriptome sequencing (such as the 5'-end library construction scheme developed by 10X Genomics) are as follows: low cell throughput, high empty droplet rate in micro-reaction systems, low sample throughput, and high library construction costs. For example, the 5'-end library construction scheme based on 10X Genomics' Feature Barcoding technology allows for the labeling of multiple samples in a single reaction but requires additional expensive Feature Barcoding kits and TotalSeq^{™} antibodies. Additionally, the barcode labels introduced by TotalSeq^{™} antibodies cannot differentiate the transcriptomes of different cells originating from the same droplet. Therefore, when analyzing sequencing data, sequencing results can only be classified as "pseudo-single cells (doublets or multiplets)" and discarded. The more cells loaded onto the system, the more "pseudo-single cells (doublets or multiplets)" data needs to be removed, resulting in higher wasted sequencing costs. Consequently, this 5'-end library construction scheme still fails to address the issues of low cell throughput, high empty droplet rate in micro-reaction systems, and high transcriptome sequencing costs.

In conclusion, existing high-throughput single-cell transcriptome library construction technologies (especially 5'-end library construction technologies) still suffer from the following limitations: low cell throughput, high empty droplet rate in micro-reaction systems, and high library construction costs. Therefore, there is an urgent need to develop new high-throughput single-cell transcriptome library construction technologies (especially 5'-end library construction technologies).

### Related prior art documents:

1. Daniel Alpern ET AL: "Time- and cost-efficient high-throughput transcriptomics enabled by Bulk RNA Barcoding and sequencing", bioRxiv, 8 January 2019 (2019-01-08), XP055719233, DOl: 10.1101/256594
2. LU BO ET AL: "Transposase-assisted tagmentation of RNA/DNA hybrid duplexes", ELIFE, vol. 9,23 July 2020 (2020-07-23), XP055892345 DOl:10.7554/eLife.54919
3. LAFZI ATEFEH ET AL: "Tutorial: guidelines for the experimental design of single-cell RNA sequencing studies", NATURE PROTOCOLS, NATURE PUBLISHING GROUP, GB, vol. 13, no.12, 16 November 2018 (2018-11-16), pages 2742-2757, XP036643359

### THE TECHNICAL TERMS

In this invention, unless otherwise specified, the scientific and technical terms used herein have the meanings commonly understood by those skilled in the art. Furthermore, the nucleic acid laboratory operations described in this document are routine procedures widely used in the respective field. Additionally, to facilitate a better understanding of the present invention, definitions and explanations of relevant terms are provided below. Unless specifically limited or described differently elsewhere in this document, the terms and descriptions related to the present invention should be interpreted according to the definitions provided below.

When the terms "for example," "such as," "including," "comprising," "containing," or variations thereof are used in this document, these terms should not be interpreted as limiting, but rather as indicating "but not limited to" or "including, but not limited to."

Unless otherwise indicated or contradicted by the context, the terms "a" and "an," as well as "the" and similar terms, when used in the context of describing the present invention (particularly in the context of the following claims), should be interpreted to cover both the singular and the plural.

As used herein, the term "pseudo-single cells (doublets or multiplets)" refers to a situation in single-cell transcriptomic experiments where a micro-reaction system (e.g., an oil droplet or a microwell) contains two or more cells. In the case of "pseudo-single cells (doublets or multiplets)," two or more cells within the same micro-reaction system (e.g., the same droplet or microwell) will be labeled with the same cell-specific tag. Consequently, using only the cell-specific tag introduced by the micro-reaction system cannot provide a "one-to-one" identification of individual cells within the micro-reaction system. Accordingly, sequencing data generated from the "pseudo-single cells (doublets or multiplets)" micro-reaction system, due to containing sequencing results from two or more cells, cannot be used to analyze the transcriptomic information of single cells. Therefore, in traditional high-throughput single-cell transcriptome sequencing methods, it is necessary to filter or remove sequencing data generated from the "pseudo-single cells (doublets or multiplets)" micro-reaction system from the final sequencing data. Furthermore, to avoid significant waste of sequencing data, it is desirable to minimize or control the quantity or ratio of "pseudo-single cells (doublets or multiplets)" micro-reaction systems. As used herein, the term "pseudo-single cells (doublets or multiplets) rate" refers to the ratio of "pseudo-single cells (doublets or multiplets)" micro-reaction systems (quantity) to all micro-reaction systems (quantity) containing cells.

As used herein, "cell throughput" refers to the number of cells that can be simultaneously labeled in a single library preparation reaction for a given single-cell library construction method.

As used herein, "sample throughput" refers to the number of samples that can be simultaneously labeled in a single library preparation reaction for a given single-cell library construction method.

As used herein, the cells or their nuclei (e.g., cells or nuclei capable of being processed using the methods of the present invention to generate a population of nucleic acid fragments) that can be used in the present invention method may be any cells or their nuclei of interest, such as cancer cells, stem cells, neuronal cells, fetal cells, and immune cells or their nuclei involved in immune responses. The cells can be a single cell or multiple cells. The cells can be a mixture of cells of the same type or a completely heterogeneous mixture of different cell types. Different cell types can include cells derived from different tissues of an individual (e.g., epithelial tissue, connective tissue, muscle tissue, nervous tissue), cells derived from body fluids (e.g., blood), or cells derived from the same tissues of different individuals (e.g., epithelial tissue, connective tissue, muscle tissue, nervous tissue), cells derived from body fluids (e.g., blood), or cells derived from microorganisms of different genera, species, strains, variants, or any combination thereof. For example, different cell types may include normal cells and cancer cells of an individual; various types of immune cells obtained from human subjects; diverse bacterial species, strains, and/or variants from the environmental, forensic, microbiome, or other samples; or any other mixture of various cell types.

For example, non-limiting examples of cancer cells that can be processed or analyzed using the methods described in this document include cancer cells such as squamous cell carcinoma, acne cell carcinoma, acoustic neuroma, acral melanoma, acral eccrine adenocarcinoma, acute eosinophilic leukemia, acute lymphoblastic leukemia, acute megakaryoblastic leukemia, acute monocytic leukemia, acute promyelocytic leukemia, acute myeloid leukemia with maturation, acute myelomonocytic leukemia, acute myeloid leukemia, acute undifferentiated leukemia, adamantinoma, adenocarcinoma, adenoid cystic carcinoma, adenoma, adenomatoid odontogenic tumor, adrenocortical carcinoma, adult T-cell leukemia, aggressive NK-cell leukemia, AIDS-related cancer, AIDS-related lymphoma, alveolar soft part sarcoma, ameloblastic fibroma, anal carcinoma, anaplastic large cell lymphoma, anaplastic thyroid carcinoma, angioimmunoblastic T-cell lymphoma, angiomyolipoma, angiosarcoma, appendiceal carcinoma, astrocytoma, atypical teratoid/rhabdoid tumor, basal cell carcinoma, basaloid carcinoma, B-cell leukemia, B-cell lymphoma, Bellini duct carcinoma, bile duct carcinoma, bladder cancer, embryonal carcinoma, bone cancer, bone tumor, brainstem glioma, brain tumor, breast cancer, Brenner tumor, bronchial tumor, bronchioloalveolar carcinoma, brown tumor, Burkitt lymphoma, Carcinoma of unknown primary origin, carcinoma, carcinoma in situ, penile cancer, , Castleman disease, central nervous system embryonal tumor, cerebellar astrocytoma, cerebral astrocytoma, cervical cancer, cholangiocarcinoma, chondroma, chondrosarcoma, chordoma, choriocarcinoma, choroid plexus papilloma, chronic lymphocytic leukemia, chronic monocytic leukemia, chronic myeloid leukemia, chronic myeloproliferative disorder, chronic neutrophilic leukemia, clear cell tumor, colon cancer, colorectal cancer, craniopharyngioma, cutaneous T-cell lymphoma, De Quervain disease, dermoid cyst, dermoid cyst, desmoplastic small round cell tumor, diffuse large B-cell lymphoma, Neuroepithelial Tumor of Embryonal Origin, Embryonal Carcinoma, Endodermal Sinus Tumor, Endometrial Cancer, Endometrioid Tumor, Enteropathy-Associated T-Cell Lymphoma, Epithelioid Malignant Mesothelioma, Epithelioid Mesothelioma, Epithelioid Sarcoma, Erythroleukemia, Esophageal Cancer, Nasal Cavity Neuroglial Tumor, Ewing Family of Tumors, Ewing Sarcoma, Ewing's Sarcoma, Extracranial Germ Cell Tumor, Extragonadal Germ Cell Tumor, Extrahepatic Cholangiocarcinoma, Extramammary Paget's Disease, Fallopian Tube Cancer, Fetus in Fetu, Fibroma, Fibrosarcoma, Follicular Lymphoma, Follicular Thyroid Cancer, Gallbladder Cancer, Gallbladder Cancer, Glioma, Ganglioneuroblastoma, Gastric Cancer, Gastric Lymphoma, Gastrointestinal Cancer, Gastrointestinal Carcinoid Tumor, Gastrointestinal Stromal Tumor, Germ Cell Tumor, Germ Cell Tumor, Gestational Trophoblastic Tumor, Gestational Nourishment Cell Tumor, Giant Cell Tumor of Bone, Pleomorphic Glioblastoma, Glioma, Glioma Disease, Glomus Tumor, Glucagonoma, Gonadal Stromal Cell Tumor, Granulosa Cell Tumor, Hairy Cell Leukemia, Head and Neck Cancer, Heart Cancer, Hemangioblastoma, Hemangiopericytoma, Hemangiosarcoma, Hematological Malignancies, Hepatocellular Carcinoma, Hepatosplenic T-Cell Lymphoma, Hereditary Breast Cancer Syndrome, Hodgkin Lymphoma, Hypopharyngeal Cancer, Hypothalamic Neuroglial Tumor, Inflammatory Breast Cancer, Intraocular Melanoma, Islet Cell Carcinoma, Islet Cell Tumor, Juvenile Myelomonocytic Leukemia, Kaposi's Sarcoma, Kaposi's Sarcoma, Kidney Cancer, Klatskin Tumor, Krukenberg Tumor, Laryngeal Cancer, Malignant Melanoma of Unknown Primary with Squamous Cell Carcinoma, Metastatic Urothelial Carcinoma, Mixed Mullerian Tumor, Monocytic Leukemia, Oral Cancer, Mucinous Tumor, Multiple Endocrine Neoplasia Syndrome, Multiple Myeloma, Multiple Myeloma, Myeloproliferative Neoplasm, Myelodysplastic Syndrome, Myeloid Leukemia, Myeloma, Myeloproliferative Disorders, Mucinous Tumor, Nasal Cavity Cancer, Nasopharyngeal Cancer, Neoplasm, Neuroma, Neuroblastoma, Neurofibroma, Neuroma, Nodular Melanoma, Non-Hodgkin Lymphoma, Non-Melanoma Skin Cancer, Non-Small Cell Lung Cancer, Ocular Tumor, Oligodendroglioma, Oligodendrocyte-like Tumor, Optic Nerve Sheath Meningioma, Oropharyngeal Cancer, Osteosarcoma, Ovarian Cancer, Ovarian Epithelial Cancer, Ovarian Germ Cell Tumor, Ovarian Low Malignant Potential Tumor, Paget's Disease, Pancoast Tumor, Gastric acidophilic granulocytic tumors, optic nerve sheath tumors, oral pharyngeal cancers, osteosarcomas, ovarian cancers, ovarian epithelial cancers, ovarian germ cell tumors, ovarian low malignant potential tumors, Paget's disease, pulmonary sulcus tumors, pancreatic cancers, papillary thyroid carcinomas, papillomatosis, paragangliomas, sinus cancers, parathyroid carcinomas, penile cancers, perivascular epithelioid cell tumors, pharyngeal cancers, chromaffin cell tumors, intermediate pineal parenchymal tumors, pineocytomas, pituitary cell tumors, pituitary adenomas, pituitary tumors, plasma cell tumors, pleural pulmonary blastomas, teratomas, precursor T-cell lymphoblastic lymphomas, primary central nervous system lymphomas, primary effusion lymphomas, primary hepatocellular carcinomas, primary liver cancers, primary peritoneal carcinomas, primitive neuroectodermal tumors, prostate cancers, pseudomyxoma peritonei, rectal cancers, renal cell carcinomas, respiratory tract cancers involving the NUT gene on chromosome 15, retinoblastomas, thabdomyomas, rhabdomyosarcomas, Richter transformation, sacrococcygeal teratomas, salivary gland cancers, sarcomas, neurofibromatosis, sebaceous gland cancers, secondary tumors, seminomas, serous tumors, sex cord-stromal tumors, Sjögren's syndrome, signet ring cell carcinomas, skin cancers, small blue round cell tumors, small cell carcinomas, small cell lung cancers, small lymphocytic lymphomas, small intestine cancers, soft tissue sarcomas, somatostatinomas, squamous papillomas, spinal cord tumors, spinal tumors, splenic marginal zone lymphomas, squamous cell carcinomas, superficial spreading melanomas, suprasellar primitive neuroectodermal tumors, surface epithelial-stromal tumors, synovial sarcomas, T-cell acute lymphoblastic leukemias, T-cell large granular lymphocytic leukemias, T-cell leukemias, T-cell lymphomas, T-cell lymphoblastic leukemias, teratomas, terminal lymphomas, testicular cancers, thecoma, thymic carcinomas, thymomas, thyroid cancers, renal pelvis and ureter transitional cell carcinomas, transitional cell carcinomas, urachal cancers, urethral cancers, urogenital tumors, uterine sarcomas, uveal melanomas, vaginal cancers, von Willebrand's disease, lymphoepithelial cystadenomas, Waldenström's macroglobulinemia, Wilms tumors, and combinations thereof.

The unlimited examples of immune cells that can be processed or analyzed using the methods described in this document include B cells, T cells (e.g., cytotoxic T cells, natural killer T cells, regulatory T cells, and T helper cells), natural killer cells, cytokine-induced killer (CIK) cells, bone marrow cells (e.g., granulocytes, including eosinophils, basophils, neutrophils/hypersegmented neutrophils), monocytes/macrophages, mast cells, platelets/megakaryocytes, dendritic cells, and combinations thereof.

Similarly, the cell nuclei of the aforementioned cells can also be processed or analyzed using the methods described in this document.

As used in this document, "long non-coding RNA" (lncRNA) has the meaning commonly understood by those skilled in the art and is interchangeable with "lncRNA." Long non-coding RNA refers to a class of RNA molecules with a transcript length exceeding 200 nucleotides that typically do not encode proteins but instead regulate the expression levels of target genes in the form of RNA.

As used in this document, "enhancer RNA" (eRNA) has the meaning commonly understood by those skilled in the art and represents a class of RNAs transcribed from enhancer regions by RNA polymerase II.

As used in this document, "nucleic acid fragment pool" refers to a collection or group of nucleic acid fragments derived, for example, from a target nucleic acid molecule (such as DNA double-stranded molecules, RNA/cDNA hybrid double-stranded molecules, DNA single-stranded molecules, or RNA single-stranded molecules). In some embodiments, the nucleic acid fragment pool includes a nucleic acid fragment library that contains sequences that represent the target nucleic acid molecule in terms of properties and/or quantities. In other embodiments, the nucleic acid fragment pool comprises a subset of the nucleic acid fragment library.

As used in this document, "nucleic acid molecule library" represents a collection or group of labeled nucleic acid fragments generated from a target nucleic acid molecule (e.g., labeled DNA double-stranded molecule fragments, labeled RNA/cDNA hybrid double-stranded molecule fragments, labeled DNA single-stranded molecule fragments, or labeled RNA single-stranded molecule fragments) wherein the combination of labeled nucleic acid fragments in the collection or group displays sequences that represent the sequence of the target nucleic acid molecule from which the labeled nucleic acid fragments were generated, in terms of properties and/or quantities. In preferred embodiments, for a nucleic acid molecule library, there has been no intentional selection or exclusion of labeled nucleic acid fragments in the collection or group by purposefully using nucleotides or sequence compositions based on the target nucleic acid molecule.

As used in this document, "cDNA," "cDNA strand," or "cDNA molecule" refers to a "complementary DNA" synthesized by extending a primer annealed to at least a portion of the RNA molecule of interest using an RNA-dependent DNA polymerase or reverse transcriptase, using the RNA molecule of interest as a template (this process is also known as "reverse transcription"). The synthesized cDNA molecule is "complementary" or "base-paired" or "forming a complex" with at least a portion of the template.

As used in this document, "transposase" refers to an enzyme capable of forming a functional complex with a complex containing transposon ends (e.g., transposon, transposon end, transposon end complex) and catalyzing the insertion or transposition of the complex containing transposon ends into double-stranded nucleic acid molecules (e.g., DNA double-strands, RNA/cDNA hybrid double-strands) incubated with the enzyme in a transposition reaction (e.g., in vitro transposition). Examples of non-limiting transposases include Tn5 transposase, MuA transposase, Sleeping Beauty transposase, Mariner transposase, Tn7 transposase, Tn10 transposase, Ty1 transposase, Tn552 transposase, and variants, modified products, and derivatives thereof having transposase activity (e.g., higher transposition activity) of the aforementioned transposases.

As used herein, the terms "transposon end" or "transposase recognition sequence" refer to the double-stranded nucleic acid molecules containing the nucleotide sequence necessary for the formation of a functional complex with the transposase in a transposition reaction. In this document, "transposon end" and "transposase recognition sequence" have the same meaning and are interchangeable. The transposon end forms a "transposase complex" or "transpososome complex" or "transpososome assembly" with the transposase that recognizes and binds to the transposon end, and this complex is capable of inserting or transposing the transposon end into the target double-stranded nucleic acid molecule incubated with it in an in vitro transposition reaction. The transposon end comprises two complementary sequences, namely the "transferred transposon end sequence" and the "non-transferred transposon end sequence." The nucleic acid chain containing the transferred transposon end sequence is called the "transferred strand," while the nucleic acid chain containing the non-transferred transposon end sequence is called the "non-transferred strand."

In an in vitro transposition reaction, the 3' end of the transferred strand is ligated to or transferred to the target nucleic acid molecule (e.g., DNA molecule, RNA molecule). In an in vitro transposition reaction, the 5' end of the non-transferred strand (i.e., the transposon end sequence complementary to the transferred transposon end sequence) does not ligate to or transfer to the target nucleic acid molecule.

In some embodiments, the transferred strand and non-transferred strand are non-covalently joined (e.g., connected by hydrogen bonds formed between bases). In some embodiments, the transferred strand and non-transferred strand are covalently joined. For example, in some embodiments, the transferred strand sequence and non-transferred strand sequence are provided on a single oligonucleotide, such as in a hairpin configuration. Thus, although the free end (5' end) of the non-transferred strand is not directly ligated to the target DNA through the transposition reaction, the non-transferred strand is indirectly linked to the DNA fragment because it is connected to the transferred strand through the loop of the hairpin structure.

"Transposon end complex" or "transposon sequence" refers to a complex comprising the transposon end (i.e., the smallest double-stranded DNA fragment capable of undergoing transposition with the transposase) optionally combined with additional sequences at the 5' end of the transferred transposon end sequence and/or the 3' end of the non-transferred transposon end sequence. In this document, "transposon end complex" and "transposon sequence" have the same meaning and are interchangeable. For example, the transposon end (transposase recognition sequence) linked to a first-label sequence and/or a first common sequence is referred to as a "transposon end complex" or "transposon sequence." In some embodiments, the transposon end complex includes two transposon end oligonucleotides or is composed of two transposon end oligonucleotides, wherein the transposon end oligonucleotides contain a sequence displaying the transposon end in combination, and one or both strands include additional sequences, forming the "transferred transposon end oligonucleotide" or "transferred strand" and the "non-transferred strand oligonucleotide" or "non-transferred strand," respectively.

The term "transferred strand" refers to the transferred portion of both the "transposon end" and the "transposon end complex," disregarding whether the transposon end is connected to a label sequence or other parts. Similarly, the term "non-transferred strand" refers to the non-transferred portion of both the "transposon end" and the "transposon end complex." In some embodiments, the transposon end complex or transposon sequence is provided as a linear double-stranded structure formed by the non-covalent hydrogen bonding between two single oligonucleotide strands. In some embodiments, the 5' end of the non-transferred strand in the transposon end complex or transposon sequence is phosphorylated. In some embodiments, the 3' end nucleotide of the non-transferred strand in the transposon end complex or transposon sequence is blocked (e.g., by a dideoxynucleotide). In some embodiments, the transposon end complex is a "hairpin transposon end complex," which refers to a transposon end complex composed of a single deoxyribo-oligonucleotide, wherein the deoxyribo-oligonucleotide displays the non-transferred transposon end sequence at its 5' end, the transferred transposon end sequence at its 3' end, and an arbitrary sequence long enough to allow stem-loop formation between the non-transferred transposon end sequence and the transferred transposon end sequence, enabling the transposon end portion to function in the transposition reaction. In some embodiments, the 5' end of the hairpin transposon end complex has a phosphate group at the 5' position of the nucleotide. In some embodiments, a specific-purpose or application-specific label sequence is provided in the arbitrary sequence inserted between the non-transferred transposon end sequence and the transferred transposon end sequence of the hairpin transposon end complex.

In the present invention, the term "upstream" is used to describe the relative positional relationship of two nucleic acid sequences (or two nucleic acid molecules) and has the meaning understood by those skilled in the art. For example, the expression "a nucleic acid sequence is located upstream of another nucleic acid sequence" means that, when arranged in the 5' to 3' direction, the former is positioned ahead (i.e., closer to the 5' end) compared to the latter. The term "downstream," as used herein, has the opposite meaning to "upstream."

As used herein, the term "label sequence" (such as "first label sequence," "second label sequence," "third label sequence," "fourth label sequence," "unique molecular label sequence," "first common sequence," "second common sequence," "first primer sequence," "second primer sequence," "template switching sequence," etc.) refers to an oligonucleotide that provides identification, recognition, and/or molecular manipulation or biochemical manipulation means (such as by providing oligonucleotides for annealing oligonucleotide sites, such as primers for DNA polymerase extension or oligonucleotides for capturing reactions or ligation reactions) to a nucleic acid fragment to which it is annealed or its annealed nucleic acid fragment derivative (e.g., a complementary fragment of the nucleic acid fragment, a fragmented short segment of the nucleic acid fragment, etc.). A label sequence may consist of at least two consecutive nucleotides (preferably about 6 to 100 nucleotides, but there is no specific limitation on the length of the oligonucleotide, as the exact size depends on many factors, which in turn depends on the final function or purpose of the oligonucleotide), or it may be composed of multiple segments of oligonucleotides arranged in a continuous or non-continuous manner. A label sequence can be unique for each nucleic acid fragment it is annealed to or unique for a certain class of nucleic acid fragments it is annealed to. A label sequence can be reversibly or irreversibly annealed to the nucleic acid sequence to be labeled using any suitable method, including ligation, hybridization, or other methods. The process of annealing a labeled sequence to a nucleic acid molecule is sometimes referred to as "labeling" in this disclosure, and a nucleic acid molecule that undergoes labeling or contains a labeled sequence is referred to as a "labeled nucleic acid molecule."

For various reasons, the nucleic acid or oligonucleotide of the present invention (such as a label sequence, transposase recognition sequence, first primer, second primer, third primer, or fourth primer) may include one or more modified nucleic acid bases, sugar portions, or nucleoside linkages. Some reasons for using nucleic acids or oligonucleotides containing modified bases, sugar portions, or nucleoside linkages include, but are not limited to: (1) altering the Tm (melting temperature); (2) altering the susceptibility of the nucleic acid or oligonucleotide to one or more nucleases; (3) providing a portion for attaching a label; (4) providing a label or quencher; or (5) providing a portion for attaching another molecule in solution or bound to a surface, such as biotin. For example, in some embodiments, oligonucleotides such as primers containing nucleosides can be synthesized such that the random portion includes one or more conformationally restricted nucleic acid analogs, such as one or more locked nucleic acid analogs where the ribose ring is locked by a methylene bridge connecting the 2'-O atom and the 4'-C atom; these modified nucleotides result in an increase in Tm or melting temperature of each nucleotide monomer by about 2 to 8 degrees Celsius. For example, in some embodiments using oligonucleotide primers containing ribonucleotides, an indicator of the use of a modified nucleotide in the method may be that the oligonucleotide containing the modification can be digested by a single-strand specific RNAse.

In the method of the present invention, for example, nucleotide bases in one or more positions of a polynucleotide or oligonucleotide may include adenine, guanine, cytosine, thymine, or uracil. Alternatively, one or more nucleotide bases in the nucleotide bases may optionally include modified bases, such as but not limited to hypoxanthine, allylamine-uracil, allylamine-thymine nucleoside, pseudouridine, 2-aminoadenine, 5-propynyluracil, 5-propynylcytosine, 4-thiouracil, 6-thioguanine, nitrogenous uracil, and deazauracil, thymidine, cytosine, adenine, or guanine. Additionally, they may include nucleotide bases derived from the following portions: a biotin moiety, a digoxigenin moiety, a fluorescent moiety or chemiluminescent moiety, a quencher moiety, or some other moiety. The present invention is not limited to the listed nucleotide bases; the provided list exemplifies a wide range of bases that can be used in the methods of the present invention.

For the nucleic acid or oligonucleotide of the present invention, one or more sugar portions in the sugar moiety may include 2'-deoxyribose, or optionally, one or more sugar portions in the sugar moiety may include some other sugar portions, such as but not limited to: ribose or 2'-fluoro-2'-deoxyribose or 2'-O-methyl-ribose that provide resistance to certain nucleases, or 2'-amino-2'-deoxyribose or 2'-azido-2'-deoxyribose that can be labeled by reacting with a visible, fluorescent, infrared fluorescent, or other detectable dye or a chemical substance having an electrophilic, photoresponsive, acetylene, or other reactive chemical moiety.

The nucleoside linkage between nucleosides in the nucleic acid or oligonucleotide of the present invention can be a phosphodiester linkage or optionally, one or more nucleoside linkages may include modified linkages, such as but not limited to: phosphorothioate, dithiophosphate, phosphoroselenate, or phosphorodiselenate linkages that provide resistance to certain nucleases.

As used herein, the term "reverse transcriptase with terminal transferase activity" refers to a reverse transcriptase that catalyzes the addition (or "tail") of one or more deoxyribonucleoside triphosphates (dNTPs) or single dideoxyribonucleoside triphosphate independently of a template to the 3' end of cDNA. Examples of such reverse transcriptases include but are not limited to, M-MLV reverse transcriptase, HIV-1 reverse transcriptase, AMV reverse transcriptase, telomerase reverse transcriptase, as well as variants, modified products, and derivatives thereof having the reverse transcriptase and terminal transferase activities. In preferred embodiments, the reverse transcriptase used for reverse transcribing RNA to generate cDNA does not have or has reduced RNase activity (particularly RNase H activity) to avoid degradation of RNA. Thus, in preferred embodiments, the reverse transcriptase used for reverse transcribing RNA to generate cDNA has terminal transferase activity and does not have or has reduced RNase activity (particularly RNase H activity). Examples of such reverse transcriptases include, but are not limited to, M-MLV reverse transcriptase,HIV-1 reverse transcriptase, AMV reverse transcriptase, and telomerase reverse transcriptase, modified or mutated to remove RNase activity (particularly RNase H activity). As used herein, the expression "reduced RNase activity" refers to a decrease in RNase activity of the modified or mutated reverse transcriptase compared to the wild-type native reverse transcriptase.

As used herein, the term "nucleic acid polymerase with strand displacement activity" refers to a nucleic acid polymerase that, during the process of elongating a new nucleic acid strand, can continue the extension reaction and dissociate (rather than degrade) the nucleic acid strand complementary to the template strand downstream.

As used herein, the term "high-fidelity nucleic acid polymerase" (or DNA polymerase) refers to a nucleic acid polymerase (or DNA polymerase) used in the amplification of nucleic acids, where the probability of introducing erroneous nucleotides (i.e., error rate) is lower than that of the wild-type Taq enzyme.

As used herein, the terms "annealing" or "hybridizing" and "annealing" or "hybridization" refer to the formation of a complex between nucleotide sequences that have sufficient complementarity to form a complex via Watson-Crick base pairing. In the context of the present invention, nucleic acid sequences that are "complementary" or "hybridize" or "anneal" to each other should be able to form or form a sufficiently stable "hybrid" or "complex" that serves the intended purpose. It is not required that every nucleotide base within a sequence displayed by one nucleic acid molecule be able to base pair or hybridize or complex with every nucleotide base within a sequence displayed by a second nucleic acid molecule in order for the two nucleic acid molecules or the respective sequences displayed by them to be "complementary" or "anneal" or "hybridize" with each other. As used herein, the terms "complementary" or "complementarity" are used when referring to the pairing of nucleotide sequences according to base pairing rules. For example, the sequence 5'-A-G-T-3' is complementary to the sequence 3'-T-C-A-5'. Complementarity can be "partial," where only some of the nucleotide bases in the nucleic acid are matched according to base pairing rules. Alternatively, complementarity between nucleic acids can be "complete" or "total." The degree of complementarity between nucleic acid strands significantly affects the efficiency and strength of hybridization between the strands. This is particularly important in amplification reactions and nucleic acid-based detection methods. The terms "annealing" or "hybridizing" are used when referring to the pairing of complementary nucleic acid strands. Hybridization and hybridization strength (i.e., the binding strength between nucleic acid strands) are influenced by many factors known in the art, including the degree of complementarity between the nucleic acids, the stringency of conditions influenced by factors such as salt concentration, the Tm (melting temperature) of the formed hybrid, the presence of other components (such as the presence or absence of polyethylene glycol or betaine), the molar concentration of the hybridizing strands, and the G:C content of the nucleic acid strands. Low stringency conditions, medium stringency conditions, or high stringency conditions can be used for annealing or hybridization, as known in the art.

As used herein, the term "bead" generally refers to a particle. Beads can be porous, non-porous, solid, semi-solid, semi-fluid, or fluid. Beads can be magnetic or non-magnetic. In some embodiments, beads can be soluble, breakable, or degradable. In some cases, beads can be non-degradable. In some embodiments, beads can be gel beads. Gel beads can be hydrogel beads. Hydrogel beads can be formed from precursor molecules such as polymers or monomeric substances. Semi-solid beads can be liposomal beads. Solid beads can contain metals, including iron oxide, gold, and silver. In some cases, beads are silica beads. In some cases, beads are rigid. In some cases, beads can be flexible and/or compressible.

In some embodiments, the beads may contain molecular precursors (e.g., monomers or polymers) that can form a polymer network through the polymerization of the precursors. In some cases, the precursors can be pre-polymerized substances that can undergo further polymerization, for example, through chemical cross-linking. In some cases, the precursors include acrylamide or methacrylamide monomers, oligomers, or polymers. In some cases, the beads may contain prepolymers that are capable of further polymerization, such as prepolymerized polyurethane beads. In some cases, the beads may contain individual polymers that can polymerize together. In some cases, the beads can be generated by polymerizing different precursors, resulting in beads that contain mixed polymers, copolymers, and/or block copolymers.

The beads can be composed of natural and/or synthetic materials. For example, the polymers can be natural polymers or synthetic polymers. In some cases, the beads contain both natural and synthetic polymers. Examples of natural polymers include proteins and sugars, such as deoxyribonucleic acid (DNA), rubber, cellulose, starch, proteins, enzymes, polysaccharides, silk, polyhydroxyalkanoates, chitosan, dextran, collagen, carrageenan, agar, gelatin, chitin, guar gum, xanthan gum, alginic acid, alginates, or their natural polymers. Examples of synthetic polymers include acrylics, nylon, siloxanes, spandex, viscose rayon, polycarboxylic acids, polyvinyl acetate, polyacrylamide, polyacrylates, polyethylene glycol, polyurethane, polylactic acid, silica, polystyrene, polyacrylonitrile, polybutadiene, polycarbonate, polyethylene, polyethylene terephthalate, poly(tetrafluoroethylene), poly(ethylene oxide), poly(butylene terephthalate), poly(trifluorochloroethylene), poly(epichlorohydrin), poly(ethylene terephthalate), polyisobutylene, poly(methyl methacrylate), poly(formaldehyde), polyacetal, polypropylene, poly(styrene-co-acrylonitrile), poly(vinyl acetate), poly(vinyl alcohol), poly(chloroethylene), poly(vinylidene chloride), poly(vinylidene fluoride), poly(vinyl fluoride), and any combination thereof (e.g., copolymers). The beads can also be formed from materials other than polymers, including lipids, micelles, ceramics, glass ceramics, material composites, metals, other inorganic materials, etc.

The beads can have uniform or non-uniform sizes. In some cases, the diameter of the beads can be approximately 1µm, 5µm, 10µm, 20µm, 30µm, 40µm, 50µm, 60µm, 70µm, 80µm, 90µm, 100µm, 250µm, 500µm, or 1mm. In some cases, the diameter of the beads can be at least approximately 1µm, 5µm, 10µm, 20µm, 30µm, 40µm, 50µm, 60µm, 70µm, 80µm, 90µm, 100µm, 250µm, 500µm, 1mm, or larger. In some cases, the diameter of the beads can be less than approximately 1µm, 5µm, 10µm, 20µm, 30µm, 40µm, 50µm, 60µm, 70µm, 80µm, 90µm, 100µm, 250µm, 500µm, or 1mm. In some cases, the diameter of the beads can be within a range of approximately 40-75µm, 30-75µm, 20-75µm, 40-85µm, 40-95µm, 20-100µm, 10-100µm, 1-100µm, 20-250µm, or 20-500µm.

In some embodiments, beads are provided as a group of beads or multiple beads with relatively monodisperse size distributions. Maintaining consistent bead characteristics, such as size, can contribute to overall uniformity, particularly when a relatively consistent amount of reagent needs to be provided within a partition. Specifically, the beads described herein can have a coefficient of variation of their cross-sectional size of less than 50%, less than 40%, less than 30%, less than 20%, and in some cases less than 15%, less than 10%, or even less than 5% in size distribution.

The beads can have any suitable shape. Examples of bead shapes include, but are not limited to, spherical, non-spherical, elliptical, elongated, amorphous, circular, cylindrical, and variations thereof.

As described herein, oligonucleotide molecules containing a labeling sequence can be coupled to the surface and/or enclosed within the beads. Functionalization of the beads for linking oligonucleotides can be achieved through various methods, including chemical groups within activated polymers, incorporation of active or activatable functional groups into the polymer structure, or linking during the precursor or monomer stage of bead production. For example, the precursors (e.g., monomers, cross-linking agents) involved in the polymerization to form the beads may include acrylamide phosphoramidite portions, such that the resulting beads also contain acrylamide phosphoramidite portions when formed. The acrylamide phosphoramidite portion can be attached to the oligonucleotide.

As described herein, the beads can release the oligonucleotides spontaneously or upon exposure to one or more stimuli, such as temperature changes, pH changes, exposure to specific chemicals or phases, exposure to light, reducing agents, etc. Adding multiple types of unstable bonds to the gel beads can result in the generation of beads capable of responding to different stimuli. Each type of unstable bond can be sensitive to a specific stimulus (e.g., chemical stimulus, light, temperature) so that the release of the substance connected to the beads through each unstable bond can be controlled by applying the appropriate stimulus. These functional groups can be used to release substances from the gel beads in a controlled manner. In some cases, another substance containing unstable bonds can be connected to the gel beads after the formation of the gel beads by using, for example, the activated functional groups of the gel beads as described above. It should be understood that the oligonucleotide released, cleaved, or reversibly connected to the beads described herein can include barcode or labeling sequences that are released or can be released by the cleavage of the bonds between the oligonucleotide molecules and the beads or by the degradation of the beads themselves, or a combination thereof. The barcode or labeling sequence allows for proximity or accessibility to other reagents.

In addition to cleavable bonds, disulfide bonds, and UV-sensitive bonds, other non-limiting examples of unstable bonds that can be coupled to precursors or beads include ester bonds (e.g., cleavable by acid, base, or hydroxylamine), orthoester bonds (e.g., cleavable by periodic acid), Diels-Alder bonds (e.g., cleavable by heat), sulfonyl bonds (e.g., cleavable by base), methylsilane ether bonds (e.g., cleavable by acid), glycosidic bonds (e.g., cleavable by amylase), peptide bonds (e.g., cleavable by protease), or phosphodiester bonds (e.g., cleavable by nucleases such as DNAase).

In addition to or as an alternative to the cleavable bonds between the beads and oligonucleotides described above, the beads can be degradable, destructible, or soluble spontaneously or upon exposure to one or more stimuli, such as temperature changes, pH changes, exposure to specific chemicals or phases, exposure to light, reducing agents, etc. In some cases, the beads can be soluble, allowing the material components of the beads to dissolve when exposed to specific chemicals or environmental changes (e.g., temperature or pH changes). In some cases, the gel beads degrade or dissolve under elevated temperature and/or alkaline conditions. In some cases, the beads can be thermally degradable, such that they degrade when exposed to appropriate temperature changes (e.g., heating). The degradation or dissolution of the beads containing substances (e.g., oligonucleotides, such as barcode-tagged oligonucleotides) can result in the release of the substances from the beads.

Additionally, substances that are not involved in the polymerization can also be encapsulated within the beads during bead formation (e.g., during the polymerization of precursors). Such substances can be introduced into the polymerization reaction mixture, resulting in the generation of beads that contain the respective substances when formed. In some cases, such substances can be added to the gel beads after their formation. Such substances can include, for example, oligonucleotides, reagents for nucleic acid amplification reactions (e.g., primers, polymerases, dNTPs, cofactors such as ion cofactors), reagents for enzyme-catalyzed reactions (e.g., enzymes, cofactors, substrates), or reagents for nucleic acid modification reactions such as polymerization, ligation, or digestion. The capture of such substances can be controlled by the polymer network density generated during the precursor's polymerization, control of ion charges within the gel beads (e.g., through ion substances linked to the polymer), or through the release of other substances. Encapsulated substances can be released from the beads during degradation of the beads and/or upon application of stimuli capable of releasing substances from the beads.

As used herein, the terms "transposase," "reverse transcriptase," and "nucleic acid polymerase" refer to protein molecules or protein complexes responsible for catalyzing specific chemical and biological reactions. Generally, the methods, compositions, or kits of the present invention are not limited to the use of specific transposases, reverse transcriptases, or nucleic acid polymerases from particular sources. Conversely, the methods, compositions, or kits of the present invention encompass any transposase, reverse transcriptase, or nucleic acid polymerase from any source that exhibits equivalent enzymatic activity as the specific enzymes disclosed in the present specification, methods, compositions, or kits. Furthermore, the methods of the present invention include the following embodiments: wherein any specific enzyme provided and used in the steps of the method is replaced by a combination of two or more enzymes, and the result obtained from the reaction mixture produced by the combination of two or more enzymes, whether used sequentially or simultaneously, is the same as the result obtained using the specific enzyme. The methods, buffer solutions, and reaction conditions provided in this disclosure, including those in the examples, are preferred embodiments for implementing the methods, compositions, and kits of the present invention. However, other enzyme storage buffers, reaction buffers, and reaction conditions known in the art for using some of the enzymes of the present invention may also be suitable for use in the present invention and are encompassed within this disclosure.

### SUMMARY OF THE INVENTION

The inventors of the present application have developed a novel method for labeling nucleic acid molecules. The labeled nucleic acid molecules generated by the method of the present invention can be conveniently used for constructing nucleic acid libraries, particularly transcriptome sequencing libraries, wherein the nucleic acid libraries contain information about the 5' end sequences of RNA molecules (e.g., mRNA molecules), which can be used to analyze the abundance, 5' end sequences, and transcription start sites of RNA molecules in the transcriptome. Furthermore, the nucleic acid libraries constructed using the present application method have dual cellular labels (e.g., a first label and a second label), thereby significantly reducing the adverse effects of "pseudo-single cells (doublets or multiplets)s" on the sequencing process and sequencing data. Therefore, the method of the present application can greatly reduce the empty rate of micro-reaction systems in the library construction process, increase the cell throughput and sample throughput of a single library preparation reaction, and significantly reduce library construction and sequencing costs.

In addition, the method of labeling nucleic acid molecules of the present invention: 1) is compatible with current major transcriptome library construction technologies and platforms (including high-throughput single-cell transcriptome library construction technologies based on microfluidic droplets, high-throughput single-cell transcriptome library construction technologies based on microplates, etc.), enabling convenient commercial applications; 2) is compatible with library construction schemes based on cells or cell nuclei, overcoming sample limitations (e.g., enabling the construction of single-cell and single-nucleus transcriptome libraries from frozen samples).

Therefore, in one aspect, the present inveniton provides a method for generating a pool of nucleic acid fragments from cells or cell nuclei, comprising the following steps:
(1) Providing one or more cells or cell nuclei;
(2) Treating the RNA (e.g., mRNA, long non-coding RNA, eRNA) within the cells or cell nuclei, including a reverse transcription step, to form double-stranded nucleic acid containing cDNA chains (e.g., hybrid double-stranded nucleic acid containing RNA (e.g., mRNA, long non-coding RNA, eRNA) chains and cDNA chains);
(3) Incubating the double-stranded nucleic acid (e.g., the hybrid double-stranded nucleic acid) with a transposase complex, wherein the transposase complex comprises a transposase and a transposon sequence that the transposase can recognize and bind to, and the transposase can cleave or break the double-stranded nucleic acid (e.g., the hybrid double-stranded nucleic acid containing RNA and DNA); and wherein the transposon sequence comprises a transposase recognition sequence, a first label sequence, and a first common sequence; wherein the first label sequence is located upstream (e.g., 5' end) of the transposase recognition sequence, and the first common sequence is located upstream (e.g., 5' end) of the first label sequence; and wherein the incubation is performed under conditions that allow the double-stranded nucleic acid (e.g., the hybrid double-stranded nucleic acid) to be fragmented by the transposase complex into nucleic acid fragments, and the transposon sequence is ligated to the end (e.g., 5' end) of the nucleic acid fragments, thereby forming a pool of nucleic acid fragments, wherein the nucleic acid fragments include cDNA fragments and the sequence of the transposon chain connected to the 5' end of the cDNA fragments.

In some preferred embodiments, described the nucleic acid fragments from the 5 'to 3' end contains the first sequence, the first label sequence, identify sequences of the translocation and cDNA fragments.

In some preferred embodiments, cells are permeabilized and/or fixed prior to proceeding with step (2). In some embodiments, the cells may be treated with methanol and/or formaldehyde before proceeding to step (2).

Regardless of theoretical limitations, cells can be permeabilized using a variety of known methods in the proposed method Such permeabilization enables a variety of reaction reagents (including, for example, enzymes such as reverse transcriptase and transposase, and nucleic acid molecules such as reverse transcription primers and transposable sequences) to penetrate the cell membrane, enter the cell, and function. In some exemplary embodiments, cells may be permeated with methanol.

Without theoretical restrictions, in the method of the present invention, cells can be fixed using various known methods. In some embodiments, cells may be fixed using formaldehyde.

The method may be used to treat one or more cells or nuclei. In some preferred embodiments at least 2 (for example, at least 10, at least 102, at least 103, at least 104, at least 105, at least 106, at least 107, or more) cells or nuclei are provided in step (1).

When the invention methods are used to handle multiple cell or cell nucleus, processed or nucleus of cells could be grouped, and for each cell or cell nucleus, can use the same or different hopping sequence for processing, thus, can be derived from the different groups of cells or cell nucleus of nucleic acid molecular markers on the same or different sequences (e.g., a first label sequence).

Therefore, in some preferred embodiments, prior to step (3) (e.g., before step (2), or after step (2) and before step (3)), the cells or cell nuclei are divided into at least 2 (e.g., at least 3, at least 4, at least 5, at least 8, at least 10, at least 12, at least 20, at least 24, at least 50, at least 96, at least 100, at least 200, at least 384, at least 400, or more) subpopulations, wherein each subpopulation contains at least one cell or cell nucleus.

In some preferred embodiments, in step (3), the double-stranded nucleic acid (e.g., hybrid double-stranded nucleic acid) within the cells or cell nuclei of each subpopulation is incubated with a transposase complex separately.

In some preferred embodiments, for each subpopulation, the transposase complex has different first label sequences, resulting in nucleic acid fragments generated from cells or cell nuclei of each subpopulation having different first label sequences.

In some preferred embodiments, for each subpopulation, the transposase complex has the same transposase, the same transposase recognition sequence, the same first common sequence, and/or the same non-transferred chain.

In some preferred embodiments, for each subpopulation, in addition to the first label sequence, the transposase complex has the same transposase, the same transposase recognition sequence, the same first common sequence, and the same non-transferred chain. In such embodiments, the nucleic acid fragments generated from cells or cell nuclei of each subpopulation have the same first common sequence and transposase recognition sequence, and the nucleic acid fragments generated from the same subpopulation have the same first label sequence, while the nucleic acid fragments generated from different subpopulations have different first label sequences.

In some preferred embodiments, the nucleic acid fragments generated from each subpopulation have the same first common sequence, and the nucleic acid fragments generated from the same subpopulation have the same first label sequence, while the nucleic acid fragments generated from different subpopulations have different first label sequences.

In some embodiments utilizing multiple first label sequences, it is easily understood that the first label sequences can be used to determine the subpopulation from which cells or cell nuclei originate and to distinguish cells or cell nuclei originating from different subpopulations. Therefore, after transposition, cells or cell nuclei from different subpopulations can be merged, and the first label sequences can be utilized to distinguish cells or cell nuclei from different subpopulations. Thus, in some preferred embodiments, after step (3), cells or cell nuclei from at least two subpopulations are merged. In some preferred embodiments, after step (3), cells or cell nuclei from each subpopulation are merged.

It is easily understood that the method of the present invention is applicable to any cells or cell nuclei, including but not limited to cancer cells, stem cells, neuronal cells, fetal cells, and immune cells or their nuclei involved in immune responses. Detailed descriptions of such cells are provided in the preceding section defining terms, but are not limited to the specific examples listed therein. In some preferred embodiments, the cells are cells or cell lines derived from animals, plants, microorganisms, or any combination thereof. In some preferred embodiments, the cells are cells or cell lines derived from mammals (e.g., humans), or any combination thereof. In some preferred embodiments, the cells are cancer cells, stem cells, neuronal cells, fetal cells, immune cells, or any combination thereof. In some preferred embodiments, the cells are immune cells, such as B cells or T cells. Accordingly, cell nuclei derived from the cells are applicable in the methods of the present application. In some preferred embodiments, the cell nuclei are derived from immune cells, such as B cells or T cells. In some preferred embodiments, the pool of nucleic acid fragments includes T cell receptor genes or gene products, or B cell receptor genes or gene products.

Without being limited by theory, various reverse transcriptases can be used to perform the reverse transcription reaction. In some preferred embodiments, a reverse transcriptase is used to reverse transcribe the RNA (e.g., mRNA, long non-coding RNA, eRNA), forming hybrid double-stranded nucleic acid containing RNA (e.g., mRNA, long non-coding RNA, eRNA) chains and cDNA chains.

In certain cases, it is advantageous to form or add overhangs at the 3' end of the cDNA strand in hybrid double-stranded nucleic acids, for example, for subsequent nucleic acid manipulations. Therefore, in some preferred embodiments, the hybrid double-stranded nucleic acid has overhangs at the 3' end of the cDNA strand. In some preferred embodiments, the overhangs have a length of at least 1, at least 2, at least 3, at least 4, at least 5, at least 6, at least 7, at least 8, at least 9, at least 10, or more nucleotides. In some preferred embodiments, the overhangs have 2-5 pyrimidine nucleotides (e.g., CCC overhangs).

Various suitable methods can be used to form or add overhangs at the 3' end of the cDNA strand. In some embodiments, overhangs can be formed or added at the 3' end of the cDNA strand by using a reverse transcriptase with terminal transferase activity. Therefore, in some preferred embodiments, the reverse transcriptase has terminal transferase activity. In some preferred embodiments, the reverse transcriptase is capable of synthesizing the cDNA strand using RNA (e.g., mRNA, long non-coding RNA, eRNA) as a template and adding overhangs at the 3' end of the cDNA strand. In some preferred embodiments, the reverse transcriptase is capable of adding overhangs of at least 1, at least 2, at least 3, at least 4, at least 5, at least 6, at least 7, at least 8, at least 9, at least 10, or more nucleotides at the 3' end of the cDNA strand. In some preferred embodiments, the reverse transcriptase is capable of adding overhangs of 2-5 pyrimidine nucleotides (e.g., CCC overhangs) at the 3' end of the cDNA strand.

It is easily understood that any reverse transcriptase (i.e., a reverse transcriptase with terminal transferase activity) capable of synthesizing a cDNA strand using an RNA molecule as a template and adding overhangs at the 3' end of the cDNA strand is applicable in this method. Examples of reverse transcriptases with terminal transferase activity include, but are not limited to, M-MLV reverse transcriptase, HIV-1 reverse transcriptase, AMV reverse transcriptase, and telomerase reverse transcriptase. Additionally, to avoid unnecessary degradation of RNA, the reverse transcriptase used preferably lacks or has reduced RNase activity (particularly RNase H activity). Therefore, in some preferred embodiments, the reverse transcriptase is selected from modified or mutated M-MLV reverse transcriptase, HIV-1 reverse transcriptase, AMV reverse transcriptase, and telomerase reverse transcriptase (e.g., M-MLV reverse transcriptase without RNase H activity). These reverse transcriptases are modified or mutated to remove RNase activity, particularly RNase H activity.

In some preferred embodiments, reverse transcription of the RNA (e.g., mRNA, long non-coding RNA, eRNA) is performed using primers containing a poly(T) sequence and/or primers containing random oligonucleotide sequences. In some preferred embodiments, the poly(T) sequence and/or random oligonucleotide sequence is located at the 3' end of the primer. In some preferred embodiments, the poly(T) sequence contains at least 5 (e.g., at least 10, at least 15, or at least 20) thymidine nucleotide residues. In some preferred embodiments, the random oligonucleotide sequence has a length of 5-30 nt (e.g., 5-10 nt, 10-20 nt, 20-30 nt). In some preferred embodiments, the primers do not contain modifications, or they contain modified nucleotides.

It is easily understood that any transposase capable of forming a functional complex with a composition containing a transposase recognition sequence and catalyzing the transposition of all or part of the composition containing the transposase recognition sequence into a double-stranded nucleic acid molecule incubated with the enzyme in a transposition reaction is applicable in the present application. In some preferred embodiments, the transposase complex is capable of randomly cleaving or breaking hybrid double-stranded nucleic acids containing RNA and DNA.

In some preferred embodiments, the transposase is selected from Tn5 transposase, MuA transposase, Sleeping Beauty transposase, Mariner transposase, Tn7 transposase, Tn10 transposase, Ty1 transposase, Tn552 transposase, as well as variants, modified products, and derivatives of the aforementioned transposases having transposition activity. In some preferred embodiments, the transposase is Tn5 transposase.

In the method of this invention, the first tag sequence is not limited by its composition or length as long as it can serve as an identifier. In some preferred embodiments, the first tag sequence has a length of at least 2, at least 3, at least 4, at least 5, at least 6, at least 7, at least 8, at least 9, at least 10, or more nucleotides. For example, the length of the first tag sequence is 4-8 nucleotides. In some preferred embodiments, the first tag sequence is connected (e.g., directly connected) to the 5' end of the transposase recognition sequence.

In the method of this invention, the first common sequence is not limited by its composition or length. In some preferred embodiments, the first common sequence has a length of at least 5, at least 6, at least 7, at least 8, at least 9, at least 10, at least 12, at least 15, at least 18, at least 20, at least 25, or more nucleotides. For example, the length of the first common sequence is 12-25 nucleotides. In some preferred embodiments, the first common sequence is connected (e.g., directly connected) to the 5' end of the first tag sequence.

In some preferred embodiments, the transferred strand from the 5' end to the 3' end contains the first common sequence, the first label sequence, and the transposase recognition sequence. In some preferred embodiments, the transposase recognition sequence has a sequence as shown in SEQ ID NO: 99. This sequence includes the recognition sequence of the Tn5 transposase.

In some preferred embodiments, the non-transfer strand is capable of annealing or hybridizing with the transferred strand to form a duplex. In some preferred embodiments, the non-transfer strand contains a sequence complementary to the transposase recognition sequence in the transferred strand. In some preferred embodiments, the non-transfer strand has a sequence as shown in SEQ ID NO: 1.

Transfer or non-transfer chains can be modified or not modified as needed. Thus, in some preferred embodiments, the transfer chain does not contain modifications, or contains modified nucleotides; And/or, the non-transferable chain does not contain a modification, or contains a modified nucleotide. In some preferred embodiments, the 5 'end of the non-transfer chain is modified with a phosphoric acid group; And/or, the 3 'terminal of the non-transferable chain is closed (e.g., the 3' terminal nucleotide of the non-transferable chain is a dideoxynucleotide).

In some preferred embodiments, in step (3), the nucleic acid fragments are formed in the cell or nucleus.

In some preferred embodiments, the nucleic acid fragments are used to construct a transcriptome library (e.g., 5' end transcriptome library) or for transcriptome sequencing (e.g., 5' end transcriptome sequencing).

In some preferred embodiments, the nucleic acid fragments are used to construct a library of target nucleic acids (e.g., V(D)J sequences) or for sequencing of target nucleic acids (e.g., V(D)J sequences). In some preferred embodiments, the target nucleic acids contain the sequences of the desired nucleic acids generated by cellular transcription or their complementary sequences. In some preferred embodiments, the target nucleic acids contain either (1) nucleotide sequences or partial sequences (e.g., V(D)J sequences) encoding T-cell receptor (TCR) or B-cell receptor (BCR), or (2) the complementary sequences of (1). In some preferred embodiments, the target nucleic acids contain sequences or complementary sequences of V(D)J genes.

In one respect, this invention provides a method for generating labeled nucleic acid molecules, which consists of the following steps:
(a) Providing:
   - One or more cells or nuclei that have been treated according to the method described above in this invention and that contain groups of nucleic acid fragments; and
   - One or more beads conjugated with oligonucleotide molecules containing a labeled sequence;
(b) Use the nucleic acid fragment and the oligonucleotide molecule to generate a labeled nucleic acid molecule containing a sequence of the nucleic acid fragment and a complementary sequence of the labeled sequence from the 5' end to the 3' end, or a complementary sequence containing the labeled sequence and the nucleic acid fragment.

It should be understood that the method can be implemented using multiple cells or nuclei and multiple beads. In some preferred embodiments, in step (a), at least 2 (e.g., at least 10, at least 102, at least 103, at least 104, at least 105, at least 106, at least 107, or more) cells or nuclei are provided, and/or at least 2 (e.g., at least 10, at least 102, at least 103, at least 104, at least 105, at least 106, at least 107, at least 108, or more) beads are provided.

The cells or nuclei and beads can be provided in various suitable reaction systems. In some preferred embodiments, in step (a), the cells or nuclei, as well as the beads, are provided in microwells or droplets (e.g., in multiple microwells or droplets). In some preferred embodiments, the droplets are oil-encapsulated water droplets. Various methods can be used to prepare oil-encapsulated water droplets containing cell nuclei or cells and oligonucleotide-conjugated beads. For example, in some illustrative embodiments, the 10X GENOMICS Chromium platform or controller can be used for the preparation of oil-encapsulated water droplets.

It is advantageous to conjugate multiple oligonucleotide molecules to the beads, which can be used to capture multiple nucleic acid fragments within the cells or nuclei. In some preferred embodiments, the beads are conjugated with multiple (e.g., at least 10, at least 102, at least 103, at least 104, at least 105, at least 106, at least 107, at least 108, or more) oligonucleotide molecules.

Various known methods can be used to conjugate oligonucleotide molecules to the beads. Such methods are described in detail in the definitions section above and are not limited to the specific examples listed therein. Additionally, the oligonucleotide molecules can be conjugated to the surface of the beads or encapsulated within the beads. In some preferred embodiments, the oligonucleotide molecules are conjugated to the surface of the beads and/or encapsulated within the beads.

In some preferred embodiments, the beads are capable of spontaneously or upon exposure to one or more stimuli (such as temperature changes, pH changes, exposure to specific chemicals or phases, exposure to light, reducing agents, etc.) releasing the oligonucleotides.

Any suitable material can be used to prepare the beads, and the beads can have any desired size, shape, particle size distribution, and/or modifications, as described in detail in the definitions section above. In some preferred embodiments, the beads are gel beads.

Various labeling sequences can be designed and used as needed. In some preferred embodiments, the labeling sequence includes one or more of the following elements: a first amplification primer sequence, a second common sequence, a second labeling sequence, a unique molecular tag sequence, a template-switching sequence, or any combination thereof. In some preferred embodiments, the labeling sequence includes the second common sequence, the second labeling sequence, the unique molecular tag sequence, and the template switching sequence. In some preferred embodiments, the labeling sequence also includes the first amplification primer sequence.

The template switching sequence can be designed to facilitate the capture (e.g., annealing or hybridization to) nucleic acid fragments within the cells or nuclei by the oligonucleotide molecules (labeling sequences). In some preferred embodiments, the template switching sequence includes a sequence that is complementary to the 3' overhang of the cDNA chain. In some preferred embodiments, the overhang is a 2-5 nucleotide overhang of pyrimidine nucleotides (e.g., CCC overhang), and the 3' end of the template-switching sequence contains a 2-5 nucleotide overhang of purine nucleotides (e.g., GGG). With the template switching sequence designed in this way, the nucleic acid fragments with the cDNA chain's 3' end within the cell nucleus or cell can be captured by the oligonucleotide molecules, allowing annealing or hybridization between the two.

It should be understood that the template switching sequence is not limited by its length. In some preferred embodiments, the template switching sequence has a length of at least 5, at least 6, at least 7, at least 8, at least 9, at least 10, at least 15, at least 20, at least 25, or more nucleotides. In some preferred embodiments, the template switching sequence does not contain modifications or contains modified nucleotides (such as locked nucleic acids). The use of modified nucleotides can be advantageous in certain cases. For example, modified nucleotides (such as locked nucleic acids) can help enhance the binding (base complementarity) between the template switching sequence and the nucleic acid fragment.

In the method of the present invention, the unique molecular tag sequence is not limited by its composition or length as long as it can serve as an identifier. In some preferred embodiments, the unique molecular tag sequence has a length of at least 5, at least 6, at least 7, at least 8, at least 9, at least 10, at least 15, at least 20, at least 25, or more nucleotides. In some preferred embodiments, the unique molecular tag sequence does not contain modifications or contains modified nucleotides.

In the method of the present invention, the second labeling sequence is not limited by its composition or length as long as it can serve as an identifier. In some preferred embodiments, the second labeling sequence has a length of at least 3, at least 4, at least 5, at least 6, at least 7, at least 8, at least 9, at least 10, at least 15, at least 20, at least 25, or more nucleotides. In some preferred embodiments, the second labeling sequence does not contain modifications or contains modified nucleotides.

In the method of the presentinvention the second common sequence is not limited by its composition or length. In some preferred embodiments, the second common sequence has a length of at least 3, at least 4, at least 5, at least 6, at least 7, at least 8, at least 9, at least 10, at least 15, at least 20, at least 25, or more nucleotides. In some preferred embodiments, the second common sequence does not contain modifications or contains modified nucleotides.

In some preferred embodiments, the beads are coupled with multiple oligonucleotide molecules, and each oligonucleotide molecule has a different unique molecular tag sequence. In some preferred embodiments, each oligonucleotide molecule has the same second labeling sequence and/or the same second common sequence.

In some preferred embodiments, the method uses more than one bead, and each bead has more than one oligonucleotide molecule; Moreover, the multiple oligonucleotide molecules on the same bead have the same second tag sequence, and the oligonucleotide molecules on different beads have different second tag sequences from each other. By means of this design of the labeling sequence, labeled nucleic acid molecules generated from nucleic acid fragments in the same droplet can carry the same second tag sequence or its complementary sequence, as well as distinct molecular tag sequences or its complementary sequence (used to label different nucleic acid fragments in the same droplet). Labeled nucleic acid molecules generated from nucleic acid fragments in different droplets may carry different second label sequences or complementary sequences.

Furthermore, for ease of subsequent nucleic acid manipulations, each oligonucleotide molecule on each bead can have the same second common sequence and/or the same first amplification primer sequence. As a result, the labeled nucleic acid molecules generated from nucleic acid fragments within each droplet can carry the same second common sequence or its complementary sequence and/or the same first amplification primer sequence or its complementary sequence. Therefore, in some preferred embodiments, the oligonucleotide molecules on each bead have the same second common sequence. In some preferred embodiments, the oligonucleotide molecules on each bead also have the same first amplification primer sequence.

The arrangement order of each component can be set according to the desired functionality of each component. For example, a template-switching sequence can be used to capture the desired nucleic acid fragments and initiate extension reactions. Accordingly, the template switching sequence can be positioned at the 3' end of the labeling sequence. For example, the second common sequence and/or the first amplification primer sequence can be used to provide primer binding sites. Accordingly, the second common sequence and/or the first amplification primer sequence can be positioned at the 5' end of the labeling sequence. Therefore, in some preferred embodiments, the template switching sequence is located at the 3' end of the labeling sequence. In some preferred embodiments, the second common sequence is located upstream of the second labeling sequence, unique molecular tag sequence, and/or template switching sequence. In some preferred embodiments, the first amplification primer sequence is located upstream of the second common sequence. In some preferred embodiments, the labeling sequence from the 5' end to the 3' end includes an optional first amplification primer sequence, second common sequence, second labeling sequence, unique molecular tag sequence, and template switching sequence. In some preferred embodiments, the labeling sequence from the 5' end to the 3' end includes an optional first amplification primer sequence, second common sequence, unique molecular tag sequence, second labeling sequence, and template switching sequence.

In some preferred embodiments, in step (b), the nucleic acid fragments and the oligonucleotide molecules are brought into contact by any of the following methods:
(b1) Cell or nuclear lysis to release nucleic acid fragments;
(b2) Release of oligonucleotide molecules from beads; or
(b3) A combination of (b1) and (b2).

In some preferred embodiments, in step (b), the oligonucleotide molecules anneal or hybridize with the nucleic acid fragments containing a 3' overhang complementary to the template switching sequence, where the template switching sequence contains a sequence complementary to the 3' overhang of the cDNA chain. The nucleic acid fragments (or the oligonucleotide molecules), under the action of a nucleic acid polymerase (e.g., DNA polymerase or reverse transcriptase), are extended using the oligonucleotide molecules (or the nucleic acid fragments) as templates, resulting in labeled nucleic acid molecules. Without being limited by theory, various suitable nucleic acid polymerases (e.g., DNA polymerase or reverse transcriptase) can be used for the extension reaction as long as they can extend the captured nucleic acid fragments (or oligonucleotide molecules) using the oligonucleotide molecules (or captured nucleic acid fragments) as templates. In some preferred embodiments, the nucleic acid polymerase used in step (b) is the same as the reverse transcriptase used in step (2).

In step (b), typically only the nucleic acid fragments with cDNA chain 3' ends can be captured by the oligonucleotide molecules through the 3' overhang of the cDNA chain. Therefore, the resulting labeled nucleic acid molecules usually contain a sequence corresponding to the 3' end of the cDNA chain (corresponding to the 5' end of RNA, such as mRNA, long non-coding RNA, eRNA) or its complementary sequence. Thus, sequencing the resulting labeled nucleic acid molecules or their derivatives can provide sequence information about the 5' end of RNA (e.g., mRNA, long non-coding RNA, eRNA) in cells or cell nuclei.

In some preferred embodiment, the labeled nucleic acid molecule from the 5' terminal to the 3' terminal contains the labeled sequence as well as a complementary sequence of the nucleic acid fragment, where the nucleic acid fragment contains a sequence complementary to the 5' terminal sequence of RNA (e.g., mRNA, long non-coding RNA, eRNA).

In some preferred embodiments, the labeled nucleic acid molecules from the 5' end to the 3' end comprise the sequence of the first common sequence, the first labeling sequence, the transposase recognition sequence, the sequence of the cDNA fragment, the complementary sequence of the template-switching sequence, the complementary sequence of the unique molecular tag sequence, the complementary sequence of the second labeling sequence, the complementary sequence of the second common sequence, and the complementary sequence of the optional first amplification primer sequence. In some preferred embodiments, the cDNA fragment contains a sequence complementary to the 5' end sequence of RNA (e.g., mRNA, long non-coding RNA, eRNA).

In some preferred embodiments, the method further comprises: (c) recovering and purifying the labeled nucleic acid molecules.

In some preferred embodiments, the labeled nucleic acid molecules are used for constructing transcriptome libraries (e.g., 5' end transcriptome libraries) or for transcriptome sequencing (e.g., 5' end transcriptome sequencing).

In some preferred embodiments, the group of nucleic acid fragments is used for constructing libraries of target nucleic acids (e.g., V(D)J sequences) or for sequencing target nucleic acids (e.g., V(D)J sequences). In some preferred embodiments, the target nucleic acids contain the sequences of the desired nucleic acids generated by cellular transcription or their complementary sequences. In some preferred embodiments, the target nucleic acids comprise (1) nucleotide sequences or partial sequences (e.g., V(D)J sequences) encoding T cell receptors (TCRs) or B cell receptors (BCRs), or (2) the complementary sequences of (1). In some preferred embodiments, the target nucleic acids comprise the sequences or complementary sequences of V(D)J genes.

In another aspect, the present invention provides a method for constructing a nucleic acid molecule library comprising:
(i) generating multiple labeled nucleic acid molecules according to the method described above in the present application, and
(ii) recovering and/or combining multiple labeled nucleic acid molecules to obtain a nucleic acid molecule library.

In some preferred embodiments, in step (ii), the labeled nucleic acid molecules derived from multiple beads are recovered and/or combined.

The labeled nucleic acid molecules may be enriched as desired. For example, nucleic acid amplification reactions can be performed on the labeled nucleic acid molecules to generate enrichment products. Therefore, in some preferred embodiments, the method further comprises (iii) enriching the labeled nucleic acid molecules.

In some preferred embodiments, in step (iii), the labeled nucleic acid molecules are subjected to nucleic acid amplification reactions to generate enrichment products. In some preferred embodiments, the nucleic acid amplification reactions use at least a first primer, wherein the first primer can hybridize or anneal to the complementary sequence of the first amplification primer sequence and/or the complementary sequence of the second common sequence. Optionally, the nucleic acid amplification reactions also use a second primer that can hybridize or anneal to the complementary sequence of the first common sequence.

In some preferred embodiments, the first primer comprises: (1) the sequence of the first amplification primer or a partial sequence thereof, or (2) the sequence of the second common sequence or a partial sequence thereof, or (3) a combination of (1) and (2).

In some preferred embodiments, the second primer comprises the sequence of the first common sequence or a partial sequence thereof.

Without being limited by theory, various suitable nucleic acid polymerases (e.g., DNA polymerases) can be used for nucleic acid amplification reactions to enrich the labeled nucleic acid molecules as long as they can amplify the labeled nucleic acid molecules as templates. In some exemplary embodiments, a nucleic acid polymerase with strand displacement activity (e.g., a DNA polymerase with strand displacement activity) can be used for the nucleic acid amplification reactions. In some exemplary embodiments, a high-fidelity nucleic acid polymerase (e.g., a high-fidelity DNA polymerase) can be used for the nucleic acid amplification reactions. In some preferred embodiments, the nucleic acid amplification reactions in step (iii) are performed using a nucleic acid polymerase (e.g., a DNA polymerase, such as a DNA polymerase with strand displacement activity and/or high fidelity).

In some embodiments, particularly in embodiments where the labeled nucleic acid molecules comprise the sequence of the nucleic acid fragment and the complementary sequence of the labeling sequence, it is preferred to include a step of degrading the oligonucleotide molecules or template switching sequences before performing step (iii). It is understood that degrading the oligonucleotide molecules or template switching sequences in some cases is advantageous, as it can prevent hindrance of the nucleic acid amplification reactions by the oligonucleotide molecules or template switching sequences.

In some preferred embodiments, the annealing temperature of the first primer to the labeled nucleic acid molecules is higher than the annealing temperature of the oligonucleotide molecules to the labeled nucleic acid molecules.

In some preferred embodiments, the method further comprises (iv) recovering and purifying the enrichment products of step (iii).

For ease of recovery and purification of the enrichment products of step (iii) in step (iv), optionally, in step (iii), the labeled first primer and/or the labeled second primer can be used for nucleic acid amplification of the labeled nucleic acid molecules. Thus, in step (iv), binding molecules capable of interacting with the labeling molecules can be used to recover and purify the enrichment products of step (iii).

In some embodiments, the binding molecules can interact with the labeling molecules through specific or non-specific interactions.

In some embodiments, the binding molecules interact with the labeling molecules through interactions selected from the following: electrostatic interactions (e.g., polylysine-glycoprotein), affinity interactions (e.g., biotin-avidin, biotin-streptavidin, antigen-antibody, receptor-ligand, enzyme-cofactor), click chemistry reactions (e.g., alkyne-containing group-azide compound), or any combination thereof.

For example, the labeling molecules can be polylysine and the binding molecules can be glycoproteins. Alternatively, the labeling molecules can be antibodies and the binding molecules can be antigens that can bind to the antibodies. Alternatively, the labeling molecules can be biotin and the binding molecules can be streptavidin.

In some embodiments, the binding molecules can be polylysine and the labeling molecules can be glycoproteins. Alternatively, the binding molecules can be antibodies and the labeling molecules can be antigens that can bind to the antibodies. Alternatively, the binding molecules can be biotin and the labeling molecules can be streptavidin.

In some embodiments, in step (iii), the first primer is conjugated with a first labeling molecule that can interact with a first binding molecule. In some embodiments, in step (iv), the first enrichment product of step (iii) is recovered and purified using the first binding molecule.

In some embodiments, in step (iii), the labeled nucleic acid molecules are subjected to nucleic acid amplification using at least the first primer and the second primer to generate enrichment products. The first primer is conjugated with a first labeling molecule and/or the second primer is conjugated with a second labeling molecule. The first labeling molecule can interact with a first binding molecule, and the second labeling molecule can interact with a second binding molecule. In some embodiments, in step (iv), the enrichment products of step (iii) are recovered and purified using the first binding molecule and/or the second binding molecule. In some embodiments, the first labeling molecule and the second labeling molecule can be the same or different, and the first binding molecule and the second binding molecule can be the same or different.

To improve the efficiency of nucleic acid amplification, in some embodiments, in step (iii), the labeled nucleic acid molecules can be subjected to nucleic acid amplification using the first primer without the labeling molecule and/or the second primer without the labeling molecule; then, additional nucleic acid amplification can be performed on the labeled nucleic acid molecules using the first primer conjugated with the first labeling molecule and/or the second primer conjugated with the second labeling molecule.

It is understood that the detailed description and definition of binding molecules provided above are equally applicable to the first binding molecule and the second binding molecule. Similarly, the detailed description and definition of labeling molecules provided above are equally applicable to the first labeling molecule and the second labeling molecule.

In the method of the present invention, nucleic acid amplification can be performed on the recovered labeled nucleic acid molecules or the recovered enrichment products to generate amplification products for sequencing. Therefore, in some preferred embodiments, the method further comprises the following steps:
(v) performing nucleic acid amplification on the recovered labeled nucleic acid molecules of step (ii) or the recovered enrichment products of step (iv) to generate amplification products.

In some preferred embodiments, in step (v), the nucleic acid amplification is performed using at least a third primer and a fourth primer. The third primer can hybridize or anneal to the complementary sequence of the first amplification primer sequence and/or the complementary sequence of the second shared sequence, optionally containing a third label sequence. The fourth primer can hybridize or anneal to the complementary sequence of the first shared sequence, optionally containing a second amplification primer sequence and/or a fourth label sequence.

In some embodiment, the third and fourth label sequences may not be used. In some embodiments, the third label sequence may be introduced in the third primer without introducing the fourth label sequence in the fourth primer. In some embodiments, the fourth label sequence may be introduced in the fourth primer without introducing the third label sequence in the third primer. In some embodiments, both the third and fourth label sequences may be introduced in the third and fourth primers, respectively. Without being limited by theory, the third and/or fourth label sequences can be used, for example, to distinguish labeled nucleic acid molecules from different libraries.

Therefore, in some preferred embodiments, the third primer contains the first amplification primer sequence or a partial sequence thereof, an optional third label sequence, and an optional second shared sequence or a partial sequence thereof.

For example, the third primer contains: (1) the first amplification primer sequence or a partial sequence thereof; or (2) the first amplification primer sequence or a partial sequence thereof, the second shared sequence or a partial sequence thereof; or (3) the first amplification primer sequence or a partial sequence thereof, a third label sequence, and the second shared sequence or a partial sequence thereof.

In some preferred embodiments, the fourth primer contains the second amplification primer sequence, an optional fourth label sequence, and the first shared sequence or a partial sequence thereof.

For example, the fourth primer contains: (1) the second amplification primer sequence and the first shared sequence or a partial sequence thereof; or (2) the second amplification primer sequence, the fourth label sequence, and the first shared sequence or a partial sequence thereof.

The third label sequence in the method of the present application is not limited by its composition or length, as long as it can serve as an identifier. In some preferred embodiments, the third label sequence has a length of at least 3, at least 4, at least 5, at least 6, at least 7, at least 8, at least 9, at least 10, or more nucleotides. In some preferred embodiments, the third label sequence does not contain modifications or contains nucleotides with modifications.

In the method of the present invention, the composition or length of the fourth label sequence is not limited as long as it can serve as an identifier. In some preferred embodiments, the fourth label sequence has a length of at least 3, at least 4, at least 5, at least 6, at least 7, at least 8, at least 9, at least 10, or more nucleotides. In some preferred embodiments, the fourth label sequence does not contain modifications or contains nucleotides with modifications.

Without being limited by theory, various suitable nucleic acid polymerases (e.g., DNA polymerases) can be used to perform nucleic acid amplification reactions to generate amplification products for sequencing, as long as they can perform nucleic acid amplification reactions using the labeled nucleic acid molecules or enrichment products as templates (e.g., extending the third primer and the fourth primer). In some embodiments, a nucleic acid polymerase with strand displacement activity (e.g., a DNA polymerase with strand displacement activity) can be used for the nucleic acid amplification reaction. In some embodiments, a high-fidelity nucleic acid polymerase (e.g., a high-fidelity DNA polymerase) can be used for the nucleic acid amplification reaction. In some preferred embodiments, the nucleic acid amplification reaction in step (v) uses a nucleic acid polymerase (e.g., a DNA polymerase; e.g., a DNA polymerase with strand displacement activity and/or high fidelity) to perform the reaction.

Nucleic acid amplification reactions for enriching labeled nucleic acid molecules and for generating nucleic acid molecules for sequencing can be performed using the same or different nucleic acid polymerases (e.g., DNA polymerases). Therefore, in some preferred embodiments, the nucleic acid polymerase (e.g., DNA polymerase) used in step (v) is the same as or different from the one used in step (iii).

In some preferred embodiments, the nucleic acid molecule library contains the amplification products from step (v).

In some preferred embodiments, the nucleic acid strand of the amplification product contains, from the 5' end to the 3' end, the second amplification primer sequence, an optional fourth label sequence, the first shared sequence, the first label sequence, a transposase recognition sequence, the sequence of the cDNA fragment, the complementary sequence of the template switching sequence, the complementary sequence of the unique molecular identifier sequence, the complementary sequence of the second label sequence, the complementary sequence of the second shared sequence, the complementary sequence of an optional third label sequence, and the complementary sequence of the first amplification primer sequence. In some preferred embodiments, the cDNA fragment contains a sequence complementary to the 5' end sequence of RNA (e.g., mRNA, long non-coding RNA, eRNA).

In some preferred embodiments, the nucleic acid molecule library is used for transcriptome sequencing (e.g., 5' end transcriptome sequencing) or for sequencing of target nucleic acids (e.g., V(D)J sequences). In some preferred embodiments, the target nucleic acid contains the sequence or its complementary sequence of the desired nucleic acid produced by cellular transcription. In some preferred embodiments, the target nucleic acid contains (1) the nucleotide sequence or a partial sequence (e.g., V(D)J sequence) encoding T cell receptor (TCR) or B cell receptor (BCR), or (2) the complementary sequence of (1). In some preferred embodiments, the target nucleic acid contains the sequence or the complementary sequence of V(D)J genes.

In some embodiments, the method of invention further comprises a step of enriching the target nucleic acid molecules.

Understandably, the step of enriching the target nucleic acid molecules can be performed at any point after step (i) of the method.

In some embodiments of the method, the enrichment of the target nucleic acid molecules occurs after step (ii), after step (iii), or after step (v).

The enrichment and recovery of the target nucleic acid molecules can be performed using various known methods. For example, oligonucleotide probes can be used to selectively enrich the target nucleic acid molecules from the pool of multiple labeled nucleic acid molecules. In some embodiments, the oligonucleotide probes contain oligonucleotide sequences that can specifically bind or anneal to the target nucleic acid molecules. In some embodiments, the oligonucleotide probes are labeled, and one or more binding molecules can be used to selectively recover and purify the target nucleic acid molecules that specifically bind or anneal to the oligonucleotide probes. The binding molecules can interact specifically or non-specifically with the labeled molecules.

In some embodiments, the target nucleic acid molecules include: (i) nucleotide sequences or partial sequences (e.g., V(D)J sequences) encoding T cell receptors (TCR), and/or (ii) the complementary sequences of (i).

In some embodiments, the specific enrichment of the target nucleic acid molecules from the pool of multiple labeled nucleic acid molecules can be achieved using a set of oligonucleotide probes comprising a first oligonucleotide probe and a second oligonucleotide probe. The first oligonucleotide probe contains a first specific oligonucleotide sequence that can specifically bind or anneal to the nucleotide sequence or its complementary sequence of the constant region of the α chain of the TCR, encoding a TCR, and a first label molecule. The second oligonucleotide probe contains a second specific oligonucleotide sequence that can specifically bind or anneal to the nucleotide sequence or its complementary sequence of the constant region of the β chain of the TCR, encoding a TCR, and a second label molecule. First binding molecules capable of interacting with the first label molecule and/or second binding molecules capable of interacting with the second label molecule can be used to recover and purify the target nucleic acid molecules annealed to the oligonucleotide probes. In some embodiments, the first label molecule is the same as the second label molecule. In some embodiments, the first label molecule is different from the label molecule.

In some embodiments, the enrichment of the target nucleic acid molecules can be performed according to the method described in Tu, A.A. et al., "TCR sequencing paired with massively parallel 3' RNA-seq reveals clonotypic T cell signatures," Nat Immunol 20, 1692-1699 (2019).

In some embodiments, the target nucleic acid molecules include: (i) nucleotide sequences or partial sequences (e.g., V(D)J sequences) encoding B cell receptors (BCR), and/or (ii) the complementary sequences of (i).

In some embodiments, the specific enrichment of the target nucleic acid molecules from the pool of multiple labeled nucleic acid molecules can be achieved using a set of oligonucleotide probes comprising a third oligonucleotide probe and a fourth oligonucleotide probe. The third oligonucleotide probe contains a third specific oligonucleotide sequence that can specifically bind or anneal to the nucleotide sequence or its complementary sequence of the constant region of the light chain of the BCR, encoding a BCR, and a third label molecule. The fourth oligonucleotide probe contains a fourth specific oligonucleotide sequence that can specifically bind or anneal to the nucleotide sequence or its complementary sequence of the constant region of the heavy chain of the BCR, encoding a BCR, and a fourth label molecule. Third binding molecules capable of interacting with the third label molecule and/or fourth binding molecules capable of interacting with the fourth label molecule can be used to recover and purify the target nucleic acid molecules annealed to the oligonucleotide probes. In some embodiments, the third label molecule is the same as the fourth label molecule. In some embodiments, the third label molecule is different from the fourth label molecule.

Understandably, the detailed descriptions and definitions regarding the label molecules and binding molecules provided earlier apply to this context as well.

In one aspect, the present invention also provides a method for sequencing nucleic acids in cells or cell nuclei, comprising:
constructing a nucleic acid library according to the method described above in the present application; and
sequencing the nucleic acid library.

In some preferred embodiments, before sequencing, at least 2, at least 3, at least 4, at least 5, at least 8, at least 10, at least 12, at least 15, at least 18, at least 20, at least 25, or more nucleic acid libraries are merged, each nucleic acid library having multiple nucleic acid molecules (i.e., amplification products), and the multiple nucleic acid molecules in the same library have the same third label sequence or the same fourth label sequence, while the nucleic acid molecules originating from different libraries have different third label sequences or different fourth label sequences.

In one aspect, the present invention also provides a nucleic acid library comprising multiple nucleic acid molecules, wherein:
the nucleic acid molecules have one strand of the nucleic acid chain containing, from the 5' end to the 3' end, a first common sequence, a first label sequence, a transposase recognition sequence, a sequence of cDNA fragments, the complementary sequence of the template switching sequence, the complementary sequence of a unique molecular identifier (UMI) sequence, the complementary sequence of a second label sequence, and the complementary sequence of a second common sequence. The cDNA fragment contains a sequence complementary to the 5' end sequence of RNA (e.g., mRNA, long non-coding RNA, eRNA).

In some preferred embodiments, the nucleic acid chain of each nucleic acid molecule has the same first common sequence, the same transposase recognition sequence, the same complementary sequence of the template switching sequence, and the same complementary sequence of the second common sequence.

In some preferred embodiments, the nucleic acid chains of the nucleic acid molecules derived from the same cell have the same first label sequence and the complementary sequence of the same second label sequence.

In some preferred embodiments, the nucleic acid chain further includes an optional second amplification primer sequence located upstream of the first common sequence and an optional fourth label sequence.

In some preferred embodiments, the nucleic acid chain further includes the complementary sequence of an optional third label sequence located downstream of the second common sequence and the complementary sequence of the first amplification primer sequence.

Understandably, the nucleic acid library can be constructed using the method provided in the present application for constructing a nucleic acid library. Therefore, the detailed descriptions and definitions of each component (including but not limited to the second amplification primer sequence, fourth label sequence, first common sequence, first label sequence, transposase recognition sequence, cDNA fragment, template switching sequence, unique molecular identifier sequence, second label sequence, second common sequence, third label sequence, and/or first amplification primer sequence) provided earlier are equally applicable to this aspect.

In some preferred embodiments, the nucleic acid library is a transcriptome library.

In some preferred embodiments, the nucleic acid molecules in the nucleic acid library are derived from immune cells.

In some preferred embodiments, the immune cells are selected from B cells and T cells.

In some preferred embodiments, the nucleic acid library is constructed using the method provided in the present application.

In one aspect, the present invention also provides a reagent kit comprising: reverse transcriptase, transposase, and one or more transposase recognition sequences that the transposase can recognize and bind to, wherein:
the transposase and transposase recognition sequences can form a transposase complex, and the transposase complex can cleave or break double-stranded nucleic acids (e.g., hybrid double-stranded nucleic acids containing RNA and DNA); and
the transposase recognition sequence contains a transfer chain and a non-transfer chain. The transfer chain contains a transposase recognition sequence, a first label sequence, and a first common sequence. The first label sequence is located upstream (e.g., 5' end) of the transposase recognition sequence, and the first common sequence is located upstream (e.g., 5' end) of the first label sequence.

In some preferred embodiments, the reagent kit contains at least 2 (e.g., at least 3, at least 4, at least 5, at least 8, at least 10, at least 20, at least 50, at least 100, at least 200, or more) transposase recognition sequences. The various transposase recognition sequences have different first label sequences. In some preferred embodiments, the various transposase recognition sequences have the same transposase recognition sequence, the same first common sequence, and/or the same non-transfer chain.

In some preferred embodiments, the reverse transcriptase has terminal transfer activity. In some preferred embodiments, the reverse transcriptase can synthesize a cDNA chain using RNA (e.g., mRNA, long non-coding RNA, eRNA) as a template and add a dangling end at the 3' end of the cDNA chain. In some preferred embodiments, the reverse transcriptase can add a dangling end consisting of at least 1, at least 2, at least 3, at least 4, at least 5, at least 6, at least 7, at least 8, at least 9, at least 10, or more nucleotides at the 3' end of the cDNA chain. In some preferred embodiments, the reverse transcriptase can add a dangling end consisting of 2-5 pyrimidine nucleotides (e.g., CCC dangling end) at the 3' end of the cDNA chain. In some preferred embodiments, the reverse transcriptase does not have or has reduced RNase activity (particularly RNase H activity). In some preferred embodiments, the reverse transcriptase is selected from modified or mutated M-MLV reverse transcriptase, HIV-1 reverse transcriptase, AMV reverse transcriptase, and telomerase reverse transcriptase (e.g., M-MLV reverse transcriptase lacking RNase H activity).

In some preferred embodiments, the transposase is selected from Tn5 transposase, MuA transposase, Sleeping Beauty transposase, Mariner transposase, Tn7 transposase, Tn10 transposase, Ty1 transposase, Tn552 transposase, and variants, modified products, and derivatives thereof with transposase activity. In some preferred embodiments, the transposase is Tn5 transposase.

In some preferred embodiments, the first label sequence is directly connected to the 5' end of the transposase recognition sequence.

In some preferred embodiments, the first common sequence is directly connected to the 5' end of the first label sequence.

In some preferred embodiments, the transfer chain comprises the first common sequence, the first label sequence, and the transposase recognition sequence from the 5' end to the 3' end. In some preferred embodiments, the transposase recognition sequence has a sequence as shown in SEQ ID NO: 99.

In some preferred embodiments, the non-transfer chain can anneal or hybridize with the transfer chain to form a double-stranded structure. In some preferred embodiments, the non-transfer chain contains a sequence complementary to the transposase recognition sequence in the transfer chain. In some preferred embodiments, the non-transfer chain has a sequence as shown in SEQ ID NO: 1.

In some preferred embodiments, the transfer chain does not contain modifications, or contains modified nucleotides; And/or, the non-transferable chain does not contain a modification, or contains a modified nucleotide. In some preferred embodiments, the 5 'end of the non-transfer chain is modified with a phosphoric acid group; And/or, the 3 'terminal of the non-transferable chain is closed (e.g., the 3' terminal nucleotide of the non-transferable chain is a dideoxynucleotide).

In some preferred embodiments, the reagent kit further comprises reverse transcription primers, such as primers containing a poly(T) sequence and/or primers containing a random oligonucleotide sequence. In some preferred embodiments, the poly(T) sequence or the random oligonucleotide sequence is located at the 3' end of the primer. In some preferred embodiments, the poly(T) sequence contains at least 5 (e.g., at least 10, at least 15, or at least 20) thymidine nucleotide residues. In some preferred embodiments, the random oligonucleotide sequence has a length of 5-30 nt (e.g., 5-10 nt, 10-20 nt, 20-30 nt). In some preferred embodiments, the primer does not contain modified nucleotides or contains modified nucleotides.

In some preferred embodiments, the reagent kit described in the present application further comprises reagents for constructing transcriptome sequencing libraries.

In some preferred embodiments, the reagents for constructing transcriptome sequencing libraries include beads coupled with oligonucleotide molecules, wherein the oligonucleotide molecules contain a labeling sequence.

In some preferred embodiments, the oligonucleotide molecules are coupled to the surface of the beads and/or encapsulated within the beads.

In some preferred embodiments, the beads can spontaneously release the oligonucleotides when exposed to one or more stimuli (e.g., temperature changes, pH changes, exposure to specific chemicals or phases, exposure to light, reducing agents, etc.).

In some preferred embodiments, the beads are gel beads.

In some preferred embodiments, the labeling sequence comprises elements selected from the following: the first amplification primer sequence, the second common sequence, the second label sequence, unique molecular identifier (UMI) sequence, template switching sequence, or any combination thereof.

In some preferred embodiments, the labeling sequence comprises the second common sequence, the second label sequence, the unique molecular identifier (UMI) sequence, and the template switching sequence. In some preferred embodiments, the labeling sequence further comprises the first amplification primer sequence.

In some preferred embodiments, the template switching sequence contains a blunt-end complementary sequence added to the 3' end of the cDNA chain by the reverse transcriptase. In some preferred embodiments, the blunt end is a blunt end of 2-5 pyrimidine nucleotides (e.g., CCC), and the 3' end of the template switching sequence contains 2-5 guanine nucleotides (e.g., GGG). In some preferred embodiments, the template switching sequence does not contain modified nucleotides or contains modified nucleotides (e.g., locked nucleic acids).

In some preferred embodiments, the unique molecular identifier (UMI) sequence has a length of at least 5, at least 6, at least 7, at least 8, at least 9, at least 10, or more nucleotides. In some preferred embodiments, the UMI sequence does not contain modified nucleotides or contains modified nucleotides.

In some preferred embodiments, the second label sequence has a length of at least 3, at least 4, at least 5, at least 6, at least 7, at least 8, at least 9, at least 10, or more nucleotides. In some preferred embodiments, the second label sequence does not contain modified nucleotides or contains modified nucleotides.

In some preferred embodiments, the second common sequence has a length of at least 3, at least 4, at least 5, at least 6, at least 7, at least 8, at least 9, at least 10, or more nucleotides. In some preferred embodiments, the second common sequence does not contain modified nucleotides or contains modified nucleotides.

In some preferred embodiments, the beads are coupled with multiple oligonucleotide molecules, and each oligonucleotide molecule has a unique molecular identifier (UMI) sequence that is different from the others. In some preferred embodiments, the oligonucleotide molecules have the same second label sequence and/or the same second common sequence.

In some preferred embodiments, the reagent kit contains multiple beads, and each bead has multiple oligonucleotide molecules, and the oligonucleotide molecules on the same bead have the same second label sequence, while the oligonucleotide molecules on different beads have different second label sequences. In some preferred embodiments, the oligonucleotide molecules on each bead have the same second common sequence. In some preferred embodiments, the oligonucleotide molecules on each bead also have the same first amplification primer sequence.

In some preferred embodiments, the template switching sequence is located at the 3' end of the labeling sequence.

In some preferred embodiments, the second common sequence is located upstream of the second label sequence, the unique molecular identifier (UMI) sequence, and/or the template switching sequence.

In some preferred embodiments, the first amplification primer sequence is located upstream of the second common sequence.

In some preferred embodiments, the labeling sequence from the 5' end to the 3' end comprises an optional first amplification primer sequence, the second common sequence, the second label sequence, the unique molecular identifier (UMI) sequence, and the template switching sequence.

In certain preferred embodiments, the reagent kit further comprises mineral oil, a buffer, dNTP, one or more nucleic acid polymerases (such as DNA polymerase; for example, a DNA polymerase with strand displacement activity and/or high fidelity), reagents for nucleic acid recovery or purification (such as magnetic beads), primers for amplifying nucleic acids (such as the first primer, second primer, third primer, fourth primer as defined above, or any combination thereof), or any combination thereof.

In certain preferred embodiments, the reagent kit further comprises reagents for sequencing, such as reagents for second-generation sequencing.

It will be understood that the reagent kit described herein can be used to perform the methods provided in this invention (e.g., methods for processing cells or cell nuclei to generate a population of nucleic acid fragments as described above; methods for generating labeled nucleic acid molecules; methods for constructing nucleic acid libraries; and/or methods for transcriptome sequencing of cells or cell nuclei). Therefore, the detailed descriptions and definitions provided above for various components and elements (including, but not limited to, reverse transcriptase, transposase, transposon sequences, oligonucleotide molecules, beads, tag sequences, nucleic acid polymerases, first, second, third, and fourth primers, and elements thereof (e.g., second amplification primer sequences, fourth tag sequences, first common sequences, first tag sequences, transposase recognition sequences, cDNA fragments, template switching sequences, unique molecular identifier (UMI)sequences, second tag sequences, second common sequences, third tag sequences, and/or first amplification primer sequences)) are equally applicable to this aspect.

In one aspect, the present invention further provides the methods described herein (e.g., methods for processing cells or cell nuclei to generate a population of nucleic acid fragments as described herein; methods for generating labeled nucleic acid molecules; and/or methods for constructing nucleic acid libraries) or the reagent kit for constructing nucleic acid libraries or for performing transcriptome sequencing.

In certain preferred embodiments, the nucleic acid library is used for transcriptome sequencing (e.g., single-cell transcriptome sequencing).

In certain preferred embodiments, the methods or reagent kit described herein are used for single-cell transcriptome sequencing. In certain preferred embodiments, the methods or reagent kit described herein are used to analyze gene expression levels, transcription start sites, and/or 5' end sequences of RNA molecules (such as mRNA, long non-coding RNA, eRNA) in cells or cell nuclei (e.g., immune cells or their nuclei).

In certain preferred embodiments, the methods or reagent kit described herein are used for constructing transcriptome libraries of cells or cell nuclei (e.g., immune cells or their nuclei) or for performing transcriptome sequencing of cells or cell nuclei (e.g., immune cells or their nuclei).

In certain preferred embodiments, the immune cells are from B cells and T cells.

### Advantages of the Invention

The present invention provides a novel method for labeling nucleic acid molecules (e.g., RNA molecules, such as mRNA molecules, long non-coding RNA, eRNA), wherein the resulting labeled nucleic acid molecules can be conveniently used for constructing nucleic acid libraries (particularly transcriptome sequencing libraries) and can be readily employed for high-throughput sequencing (especially high-throughput single-cell transcriptome sequencing). The method of the present invention exhibits one or more beneficial technical effects selected from the following:
(1) The nucleic acid libraries constructed using the method of the present invention contain information on the 5' end sequences of RNA molecules (e.g., mRNA molecules, long non-coding RNA, eRNA). Accordingly, the high-throughput sequencing data obtained from the nucleic acid libraries can be used not only for analyzing the abundance of RNA molecules (e.g., mRNA molecules, long non-coding RNA, eRNA) in the transcriptome but also for analyzing the transcription start sites and 5' end sequences of RNA molecules (e.g., mRNA molecules). Therefore, the method of the invention can be conveniently employed for analyzing the sequences of TCR and BCR, compatible with V(D)J sequencing methods.
(2) The method for labeling nucleic acid molecules (e.g., RNA molecules, such as mRNA molecules, long non-coding RNA, eRNA) of the present invention is compatible with the current major transcriptome library construction technologies and platforms (including high-throughput single-cell transcriptome library construction technologies based on microfluidic droplets, microplate-based high-throughput single-cell transcriptome library construction technologies, etc.), enabling convenient commercial applications.
(3) The nucleic acid libraries constructed using the method of the present invention can significantly reduce the adverse impact of "pseudo-single cells (doublets or multiplets)" on the sequencing process and sequencing data. Currently, the major library construction and sequencing methods for transcriptome sequencing (especially 5' end library construction and sequencing methods) suffer from the severe limitations imposed by "pseudo-single cells (doublets or multiplets)" phenomena. Sequencing data generated from "pseudo-single cells (doublets or multiplets)" are typically filtered and discarded due to their inability to accurately reflect the transcriptome information of individual cells. This leads to waste of sequencing data and increased sequencing costs. The nucleic acid libraries constructed using the method of the present invention possess dual cell labels (e.g., first label and second label), enabling the segregation of sequencing data generated from "pseudo-single cells (doublets or multiplets)" and accurate tracking and determination of the cell origin of the sequencing data. In other words, even sequencing data generated from "pseudo-single cells (doublets or multiplets)" can be utilized through the use of the method of the present invention, thereby greatly reducing the negative impact of the "pseudo-single cells (doublets or multiplets)" phenomenon during library construction. Consequently, the utilization rate of sequencing data is greatly improved, and sequencing costs are significantly reduced.
(4) The nucleic acid libraries constructed using the method of the present invention can substantially reduce the empty reaction rate in the microreaction system during library construction. Due to the limitations imposed by the "pseudo-single cells (doublets or multiplets)" phenomenon, the microreaction systems required by the current major transcriptome library construction and sequencing methods (particularly 5' end library construction and sequencing methods) have a very high empty reaction rate (close to 99%). In other words, out of a hundred microreaction systems, only one microreaction is loaded with a cell. This leads to significant waste of reaction systems and reagents, resulting in high library construction costs. The method of the present invention greatly eliminates the negative impact of the "pseudo-single cells (doublets or multiplets)" phenomenon, allowing the use of higher cell throughput during library construction (e.g., cell throughput can be increased by at least 5 times, at least 10 times, or even 100 times), substantially reducing the empty reaction rate in the microreaction system, and significantly lowering library construction costs. For example, in certain exemplary embodiments, with the aid of the method of the present application, the cell throughput for a single library construction can be increased to 100,000-1,000,000 cells.
(5) The nucleic acid libraries constructed through a single reaction using the method of the present invention can originate from different samples (e.g., cells from 2, 94, or 384 individuals). The number of samples in a single reaction depends on the type of the first label and is easily scalable, theoretically without any upper limit. The method of the present invention significantly increases the sample throughput of a single reaction at a lower cost, making it applicable for single-cell transcriptome sequencing of large population samples, immune repertoire analysis of large population samples, drug screening under various conditions based on cell lines and organoids, and large-scale parallel single-cell interpretation of gene editing results, among other invention scenarios.
(6) The method of the present invention is compatible with library construction schemes based on cells or nuclei and can perform multiplex labeling of RNA molecules (e.g., mRNA molecules, long non-coding RNA, eRNA) within individual cells or nuclei, enabling more flexible and extensive applications.

The following detailed description of the embodiments of the present invention will be given in conjunction with the accompanying drawings and exemplary embodiments. However, those skilled in the art will understand that the following drawings and exemplary embodiments are for illustrative purposes only and do not limit the scope of the present invention. Based on the detailed description of the following drawings and preferred embodiments, various objectives and advantageous aspects of the present invention will become apparent to those skilled in the art.

### BRIEF DESCRIPTION OF THE DRAWINGS

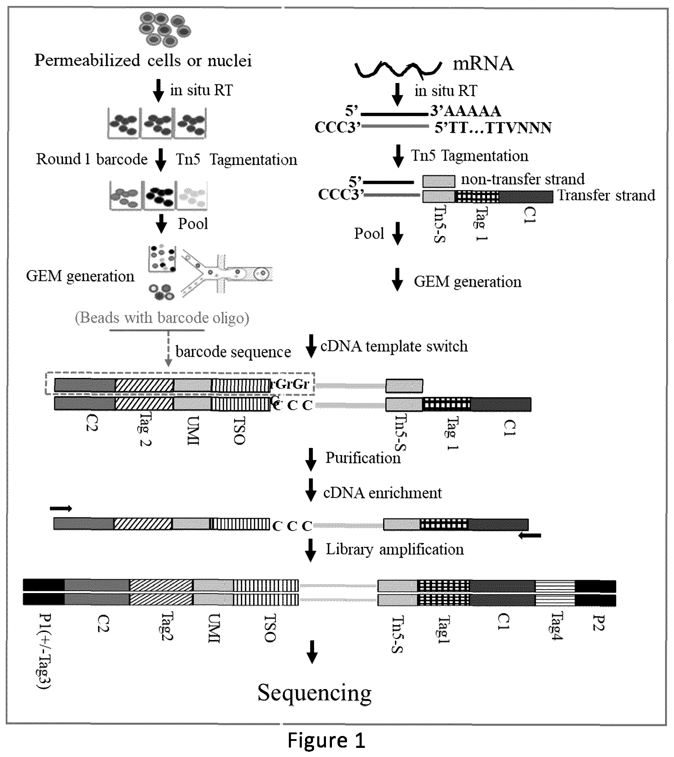

Figure 1 illustrates an exemplary scheme for constructing libraries for single-cell transcriptome sequencing using the method of the present invention, as well as the exemplary structures of nucleic acid molecules used for sequencing in the library. The exemplary scheme includes the following steps.

First, the permeabilized cells or nuclei are divided into one or more subsets (e.g., at least 1, at least 2, at least 3, at least 4, at least 5, at least 8, at least 10, at least 20, at least 50, at least 100, at least 200, or more subsets). The RNA molecules (e.g., mRNA molecules, long non-coding RNA, eRNA) within the nuclei/permeabilized cells are reverse transcribed using a reverse transcriptase (e.g., a reverse transcriptase with terminal transfer activity) and reverse transcription primers (e.g., reverse transcription primers carrying a poly(T) sequence or random oligonucleotide sequence at the 3' end) to generate cDNA with a dangling end (e.g., a dangling end containing three cytidine nucleotides). The RNA (e.g., mRNA, long non-coding RNA, eRNA) within the cells or nuclei hybridizes with the generated cDNA, forming a hybrid double-stranded nucleic acid. In this step, it is also possible to first reverse transcribe the RNA molecules (e.g., mRNA molecules, long non-coding RNA, eRNA) within the nuclei/permeabilized cells using a reverse transcriptase (e.g., a reverse transcriptase with terminal transfer activity, e.g., M-MLV reverse transcriptase) and reverse transcription primers (e.g., reverse transcription primers carrying a poly(T) sequence or random oligonucleotide sequence at the 3' end) to generate a hybrid double-stranded nucleic acid containing the RNA (e.g., mRNA, long non-coding RNA, eRNA) and cDNA, followed by dividing the permeabilized cells or nuclei into multiple subsets. Various reverse transcriptases with terminal transfer activity can be used for the reverse transcription reaction. In some preferred embodiments, the reverse transcriptase used does not have or has reduced RNase activity (particularly RNase H activity).

Next, a transposase complex capable of cutting or breaking the hybrid double-stranded nucleic acid (e.g., Tn5 transposase complex) is used to transpose the hybrid double-stranded nucleic acid containing RNA (e.g., mRNA, long non-coding RNA, eRNA) and cDNA, causing random fragmentation of the hybrid double-stranded nucleic acid. In this step, the transposase complex used contains a transposase (e.g., Tn5 transposase) and a transposon sequence that the transposase can recognize and bind to (e.g., a transposon sequence containing the Tn5 transposase recognition sequence). The transposon sequence includes a transposed strand and a non-transposed strand. The transposed strand includes the transposase recognition sequence (Tn5 transposase recognition sequence; Tn5-S), a first label sequence (Tag1), and a first common sequence (C1). The first label sequence is located upstream (e.g., at the 5' end) of the transposase recognition sequence, and the first common sequence is located upstream (e.g., at the 5' end) of the first label sequence. The non-transposed strand contains a sequence complementary to the transposase recognition sequence in the transposed strand. Consequently, after transposition, the transposase complex randomly breaks the hybrid double-stranded nucleic acid into nucleic acid fragments, and the transposed strands carrying the first label sequence and the first common sequence are connected to the 5' end of the fragmented cDNA strand.

Furthermore, in this step, the transposase complexes used for each subset have different first label sequences. Consequently, the nucleic acid fragments generated from cells or nuclei of each subset contain different first label sequences. In preferred embodiments, besides the first label sequence, the transposase complexes used for each subset may have the same transposase, the same transposase recognition sequence, the same first common sequence, and the same non-transposed strand. As a result, the nucleic acid fragments generated from cells or cell nuclei of each subset have the same first common sequence and transposase recognition sequence, and the nucleic acid fragments generated from cells or cell nuclei of the same subset have the same first label sequence, while the nucleic acid fragments generated from cells or nuclei of different subsets have different first label sequences.
Subsequently, the nuclei or cells of multiple subsets (e.g., all subsets) are combined and brought into contact with beads conjugated with multiple oligonucleotides, generating labeled nucleic acid molecules. In preferred embodiments, in droplets (e.g., water-in-oil droplets), the nuclei or cells are brought into contact with beads conjugated with oligonucleotides (e.g., beads provided by 10X Genomics for transcriptome library construction), wherein the oligonucleotides contain a labeling sequence. Exemplary labeling sequences may include a first amplification primer sequence (P1), a second common sequence (C2), a second label sequence (Tag2), a unique molecular identifier (UMI), a template switching sequence (TSO), or any combination thereof. In preferred embodiments, the labeling sequence may include the first amplification primer sequence, the second common sequence, the second label sequence, the unique molecular identifier, and the template switching sequence. The template switching sequence is typically located at the 3' end of the labeling sequence. The first amplification primer sequence and/or the second common sequence are typically located at the 5' end of the labeling sequence. Various methods can be used to prepare water-in-oil droplets containing nuclei or cells and beads conjugated with oligonucleotides. For example, in some exemplary embodiments, the 10X GENOMICS Chromium platform or controller can be used for the preparation of water-in-oil droplets.

In exemplary embodiments, the template switching sequence may contain a sequence that is complementary to the 3' overhang of the cDNA strand. For example, when the 3' end of the cDNA strand contains a 3-nucleotide overhang of thymines, the template switching sequence may contain GGG at its 3' end. Additionally, modifications can be made to the nucleotides of the template switching sequence (e.g., using locked nucleic acids) to enhance the complementary pairing between the template switching sequence and the 3' overhang of the cDNA strand. With the designed template switching sequence, nucleic acid fragments containing the 3' end of the cDNA strand within nuclei or cells can be captured by oligonucleotide molecules and undergo annealing or hybridization. Subsequently, the captured nucleic acid fragments can be extended by nucleic acid polymerase using the oligonucleotide molecules as templates, adding the complementary sequence of the labeling sequence to the 3' end of the cDNA strand, thereby generating labeled nucleic acid molecules carrying the first label sequence, the first common sequence, and the complementary sequence of the labeling sequence at the 5' end and carrying the complementary sequence of the labeling sequence at the 3' end. It is not limited in theory, various suitable nucleic acid polymerases (e.g., DNA polymerase or reverse transcriptase) can be used for the extension reaction, as long as they can extend the captured nucleic acid fragments using the oligonucleotide molecules as templates. In some exemplary embodiments, the same reverse transcriptase used in the aforementioned reverse transcription step can be used to extend the captured nucleic acid fragments. In this process, typically only the nucleic acid fragments containing the 3' end of the cDNA strand can be captured by the oligonucleotide molecules through the 3' overhang of the cDNA strand. Therefore, the resulting labeled nucleic acid molecules typically contain the sequence corresponding to the 5' end of RNA (e.g., mRNA, long non-coding RNA, eRNA) at the 3' end of the cDNA strand. Thus, sequencing the resulting labeled nucleic acid molecules or their derivatives can provide sequence information for the 5' end of RNA (e.g., mRNA) within cells or nuclei.

In exemplary embodiments, each bead is individually coupled with multiple oligonucleotide molecules. Moreover, the oligonucleotide molecules on the same bead can have distinct unique molecular identifier sequences, while the oligonucleotide molecules on the same bead can have the same second label sequence. Additionally, oligonucleotide molecules on different beads can have distinct second label sequences. With this design of labeling sequences, labeled nucleic acid molecules generated from nucleic acid fragments within the same droplet can carry the complementary sequences of the same second label sequence and the distinct complementary sequences of unique molecular identifier sequences (for labeling different nucleic acid fragments within the same droplet). Labeled nucleic acid molecules generated from nucleic acid fragments within different droplets can carry distinct complementary sequences of second label sequences.

Furthermore, to facilitate subsequent nucleic acid manipulations, the oligonucleotide molecules on each bead can have the same second common sequence and/or the same first amplification primer sequence. Consequently, labeled nucleic acid molecules generated from nucleic acid fragments within different droplets can carry the complementary sequences of the same second common sequence and/or the complementary sequences of the same first amplification primer sequence. For example, the first amplification primer sequence may include a library adapter sequence (e.g., P5 adapter sequence). This allows the addition of library adapters to the ends of the labeled nucleic acid molecules, facilitating subsequent sequencing. Subsequently, the generated multiple labeled nucleic acid molecules can be recovered and pooled.

As needed, the labeled nucleic acid molecules can be enriched. For example, nucleic acid amplification reactions can be performed on the labeled nucleic acid molecules to generate enrichment products. In exemplary embodiments, the nucleic acid amplification reaction can be performed using at least a first primer. In some cases, the first primer can be designed to hybridize or anneal to the complementary sequence of the first amplification primer sequence and/or the complementary sequence of the second common sequence. An exemplary first primer can include: the first amplification primer sequence or a partial sequence thereof, or the second common sequence or a partial sequence thereof, or a combination of both. It is not limited in theory, various suitable nucleic acid polymerases (e.g., DNA polymerase) can be used for the nucleic acid amplification reaction to enrich the labeled nucleic acid molecules, as long as they can extend the first primer using the labeled nucleic acid molecules as templates. In some exemplary embodiments, a nucleic acid polymerase with strand displacement activity (e.g., a DNA polymerase with strand displacement activity) can be used for the nucleic acid amplification reaction. In some exemplary embodiments, a high-fidelity nucleic acid polymerase (e.g., a high-fidelity DNA polymerase) can be used for the nucleic acid amplification reaction. Subsequently, the enriched products can be recovered and purified. It is understood that the enrichment step is not necessary and can be performed based on practical considerations.

Subsequently, the recovered labeled nucleic acid molecules or the recovered enrichment products can be subjected to nucleic acid amplification reactions to generate amplification products for sequencing. In exemplary embodiments, the nucleic acid amplification reaction can be performed using at least a third primer and a fourth primer. The third primer can be designed to hybridize or anneal to the complementary sequence of the first amplification primer sequence and/or the complementary sequence of the second common sequence, optionally containing a third label sequence (Tag3). Additionally, the fourth primer can be designed to hybridize or anneal to the complementary sequence of the first common sequence, optionally containing the second amplification primer sequence (P2) and/or the fourth label sequence (Tag4).

In some cases, the third and fourth labels may not be used. In some cases, the third label can be introduced in the third primer without introducing the fourth label in the fourth primer. In some cases, the fourth label can be introduced in the fourth primer without introducing the third label in the third primer. In some cases, the third and fourth labels can be respectively introduced in the third and fourth primers. It is not limited in theory, the third and/or fourth labels can be used, for example, to distinguish labeled nucleic acid molecules from different libraries.

Therefore, in exemplary embodiments, the third primer may contain: ① the first amplification primer sequence or a partial sequence thereof, or ② the first amplification primer sequence or a partial sequence thereof, and the second common sequence or a partial sequence thereof, or ③ the first amplification primer sequence or a partial sequence thereof, a third label sequence, and the second common sequence or a partial sequence thereof. In exemplary embodiments, the fourth primer may contain: ① the second amplification primer sequence and the first common sequence or a partial sequence thereof, or ② the second amplification primer sequence, a fourth label sequence, and the first common sequence or a partial sequence thereof.

It is not limited in theory, various suitable nucleic acid polymerases (e.g., DNA polymerase) can be used to perform nucleic acid amplification reactions to generate amplification products for sequencing, as long as they can extend the third primer and the fourth primer using the labeled nucleic acid molecules or the enrichment products as templates. In some exemplary embodiments, a nucleic acid polymerase with strand displacement activity (e.g., a DNA polymerase with strand displacement activity) can be used for the nucleic acid amplification reaction. In some exemplary embodiments, a high-fidelity nucleic acid polymerase (e.g., a high-fidelity DNA polymerase) can be used for the nucleic acid amplification reaction. The nucleic acid amplification reaction for enriching the labeled nucleic acid molecules and the nucleic acid amplification reaction for generating nucleic acid molecules for sequencing can use the same or different nucleic acid polymerases (e.g., DNA polymerase).

In exemplary embodiments, the second amplification primer sequence can include a library adapter sequence (e.g., P7 adapter sequence). Consequently, the generated amplification products can contain library adapter sequences (e.g., P5 adapter sequence and P7 adapter sequence) at both ends and can be used for subsequent sequencing (e.g., next-generation sequencing).

Figure 1 also illustrates an exemplary structure of one nucleic acid strand of the nucleic acid molecules (amplification products) to be sequenced in the library constructed by the aforementioned exemplary embodiments. The structure includes: the second amplification primer sequence (e.g., P7 adapter sequence), the fourth label sequence, the first common sequence, the first label sequence, the transposase recognition sequence, the sequence of the cDNA fragment, the complementary sequence of the template switching sequence, the complementary sequence of the unique molecular identifier sequence, the complementary sequence of the second label sequence, the complementary sequence of the second common sequence, and the complementary sequence of the first amplification primer sequence (e.g., P5 adapter sequence), wherein the cDNA fragment contains a sequence complementary to the 5' end sequence of RNA (e.g., mRNA).

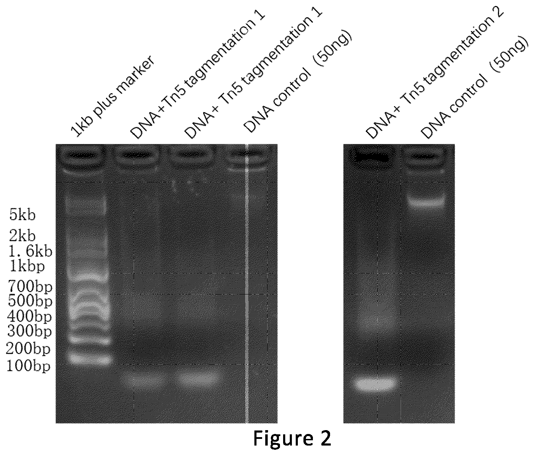

Figure 2 shows the gel electrophoresis results of the products obtained by transposing mouse genomic DNA using Tn5 transposase complex. The experimental results indicate that the Tn5 transposase complex can fragment mouse genomic DNA (23 kb in full length) into bands of 300-600 bp.

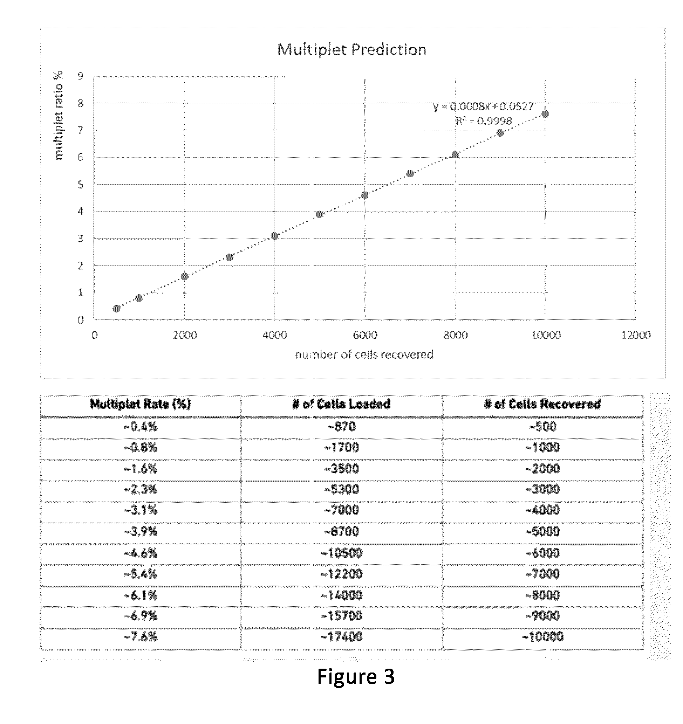

Figure 3 shows the relationship between the captured (recovered) cell count and the "doublets or multiplets" rate and the number of cells loaded for 5' end transcriptome sequencing library construction using the 10X Genomics Chromium platform and reagent kit. The results show that the doublets or multiplets rate is linearly correlated with the number of cells loaded: the higher the number of cells loaded, the higher the "doublets or multiplets rate".

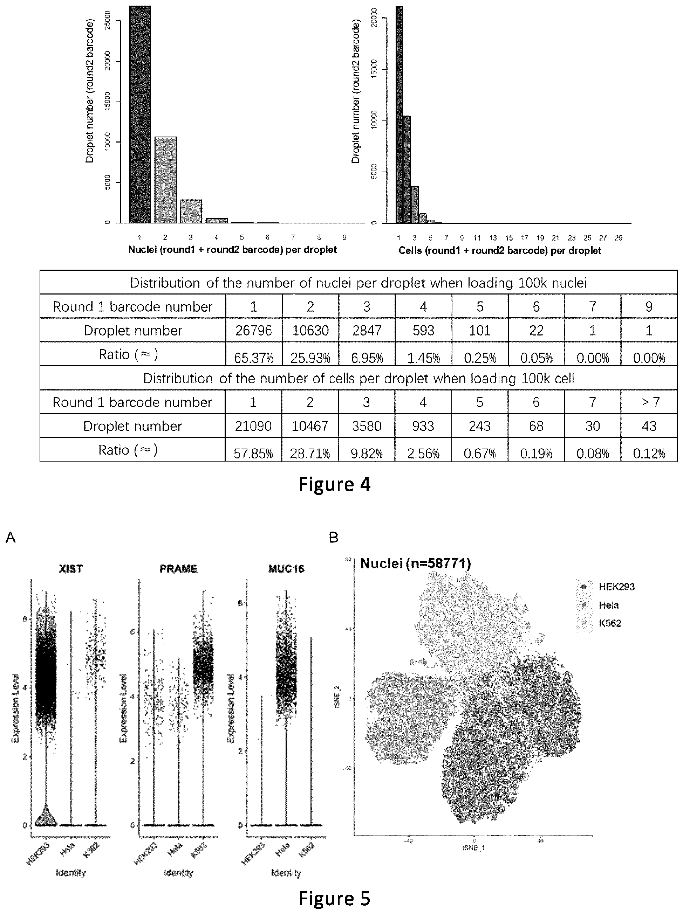

Figure 4 illustrates the number and ratio of water-in-oil droplets containing different numbers of the first label (1-9 labels) prepared using permeabilized cells or nuclei in Example 4. The results show that the ratio of droplets containing two or more types of the first label is 34.63% (nuclear sample) or 42.15% (cell sample).

Figure 5 shows the results of single-cell transcriptome analysis obtained from 5' end transcriptome library construction and sequencing using cell nuclei from HEK293T cells, Hela cells, and K562 cells in Example 4. Figure 5A displays the expression of marker genes in each cell line, and Figure 5B presents the clustering visualization results of each cell line.

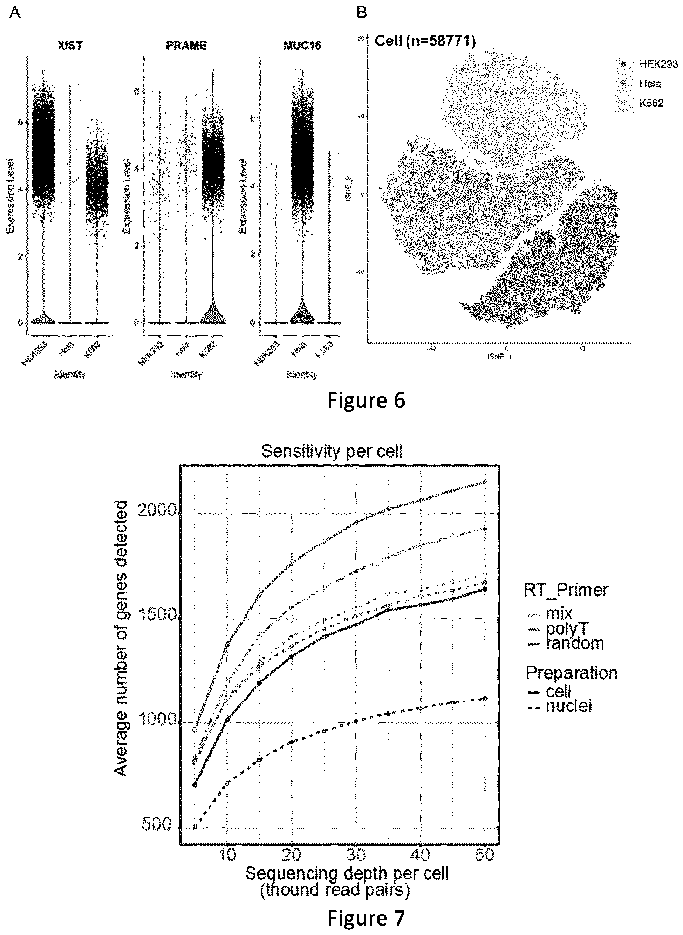

Figure 6 displays the results of single-cell transcriptome analysis obtained from 5' end transcriptome library construction and sequencing using permeabilized HEK293T cells, Hela cells, and K562 cells in Example 4. Figure 6A shows the expression of marker genes in each cell line, and Figure 6B presents the clustering visualization results of each cell line.

Figure 7 shows the results of single-cell transcriptome analysis obtained from 5' end transcriptome library construction and sequencing using permeabilized cell samples and nuclear samples from Hela cells in Example 5, using three different reverse transcription primers. The x-axis represents sequencing depth, and the y-axis represents the number of detected genes.

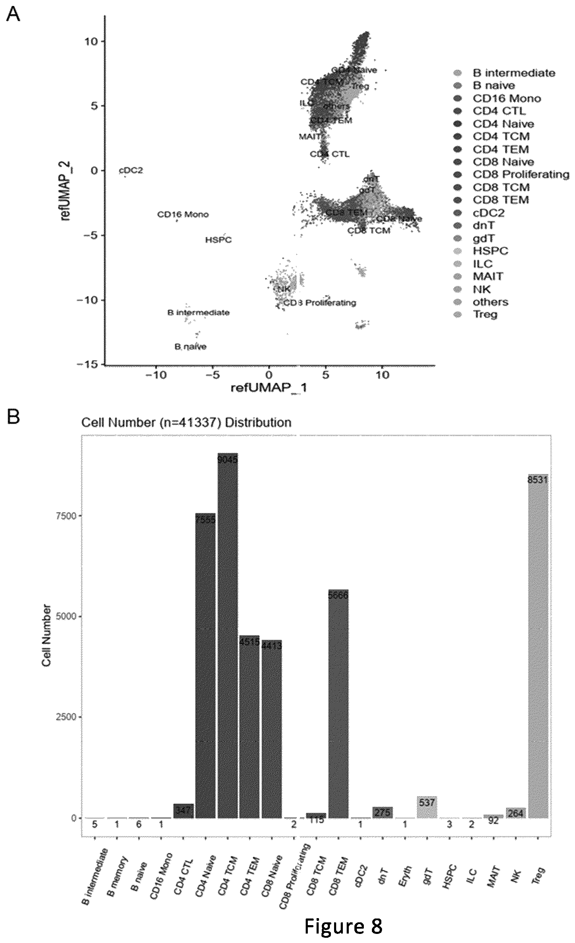

Figure 8 displays the results of data analysis from single-cell transcriptomes of enriched T cells from peripheral blood samples of 14 individuals in Example 6. Figure 8A shows the visualization results of different cell clusters, and Figure 8B shows the number of cells in each cell cluster.

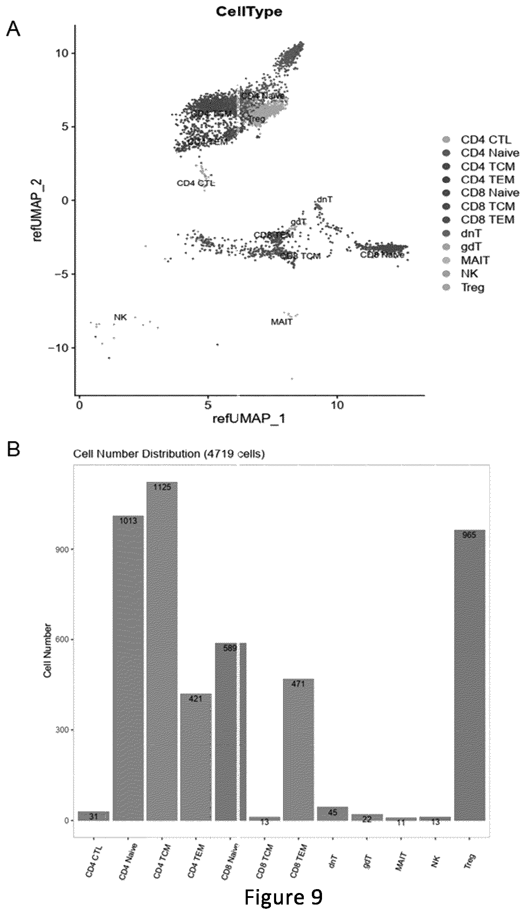

Figure 9 displays the results of data analysis of the TCR VDJ region from enriched T cells from peripheral blood samples of 14 individuals in Example 6. Figure 9A shows the visualization results of the distribution of different TCR clones detected in T cells, and Figure 9B shows the number of cells in each cluster of detected TCR clones.

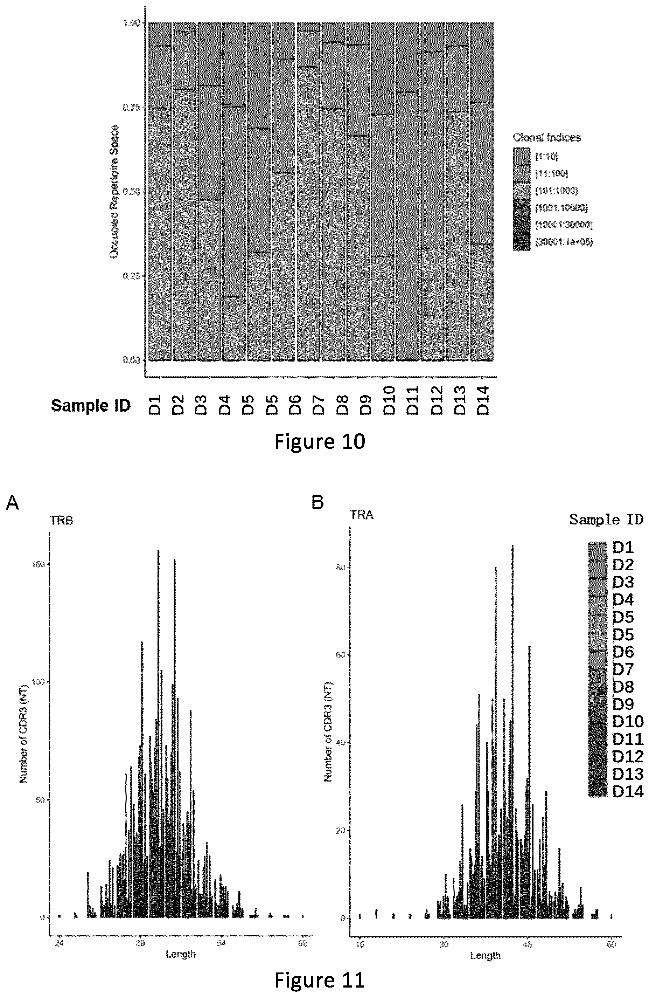

Figures 10 and 11 display the basic information of TCR clones obtained from enriched T cells from peripheral blood samples of 14 individuals in Example 6. Figure 10 shows the distribution of major TCR clones detected in each of the 14 samples, indicating the diversity of TCR clones among different individuals. Figures 11A and 11B respectively show the distribution of detected TRB genes (Figure 11A) and TRA genes (Figure 11B) in the 14 individuals.

### Sequence Information

The information regarding the sequences involved in the present invention is provided in Table 1 below.

**Table 1:**

| SEQ ID NO: | Sequence Description (5'-3') |
|---|---|
| 1 | Transposon Adapter 1 Nucleotide Sequence |
| | CTGTCTCTTATACACATCTC |
| 2 | Transposon Adapter 2 Nucleotide Sequence |
| | GACGTGTGCTCTTCCGATCT[NNNNNN]AGATGTGTATAAGAGACAG |
| 3 | Transposon Tag 1 Nucleotide Sequence |
| | AAAGAA |
| 4 | Transposon Tag 2 Nucleotide Sequence |
| | AACAGC |
| 5 | Transposon Tag 3 Nucleotide Sequence |
| | AACGTG |
| 6 | Transposon Tag 4 Nucleotide Sequence |
| | AAGCCA |
| 7 | Transposon Tag 5 Nucleotide Sequence |
| | AAGTAT |
| 8 | Transposon Tag 6 Nucleotide Sequence |
| | AATTGG |
| 9 | Transposon Tag 7 Nucleotide Sequence |
| | ACAAGG |
| 10 | Transposon Tag 8 Nucleotide Sequence |
| | ACCCAA |
| 11 | Transposon Tag 9 Nucleotide Sequence |
| | ACCTTC |
| 12 | Transposon Tag 10 Nucleotide Sequence |
| | ACGGAC |
| 13 | Transposon Tag 11 Nucleotide Sequence |
| | ACTGCA |
| 14 | Transposon Tag 12 Nucleotide Sequence |
| | AGACCC |
| 15 | Transposon Tag 13 Nucleotide Sequence |
| | AGATGT |
| 16 | Transposon Tag 14 Nucleotide Sequence |
| | AGCACG |
| 17 | Transposon Tag 15 Nucleotide Sequence |
| | AGGTTA |
| 18 | Transposon Tag 16 Nucleotide Sequence |
| | AGTAAA |
| 19 | Transposon Tag 17 Nucleotide Sequence |
| | AGTCTG |
| 20 | Transposon Tag 18 Nucleotide Sequence |
| | ATACTT |
| 21 | Transposon Tag 19 Nucleotide Sequence |
| | ATAGCG |
| 22 | Transposon Tag 20 Nucleotide Sequence |
| | ATATAC |
| 23 | Transposon Tag 21 Nucleotide Sequence |
| | ATCCGG |
| 24 | Transposon Tag 22 Nucleotide Sequence |
| | ATGAAG |
| 25 | Transposon Tag 23 Nucleotide Sequence |
| | ATTAGT |
| 26 | Transposon Tag 24 Nucleotide Sequence |
| | CAACCG |
| 27 | Transposon Tag 25 Nucleotide Sequence |
| | CAAGTC |
| 28 | Transposon Tag 26 Nucleotide Sequence |
| | CACCAC |
| 29 | Transposon Tag 27 Nucleotide Sequence |
| | CACTGT |
| 30 | Transposon Tag 28 Nucleotide Sequence |
| | CAGACT |
| 31 | Transposon Tag 29 Nucleotide Sequence |
| | CAGGAG |
| 32 | Transposon Tag 30 Nucleotide Sequence |
| | CATAGA |
| 33 | Transposon Tag 31 Nucleotide Sequence |
| | CCACGC |
| 34 | Transposon Tag 32 Nucleotide Sequence |
| | CCGATG |
| 35 | Transposon Tag 33 Nucleotide Sequence |
| | CCGTAA |
| 36 | Transposon Tag 34 Nucleotide Sequence |
| | CCTCTA |
| 37 | Transposon Tag 35 Nucleotide Sequence |
| | CGAAAG |
| 38 | Transposon Tag 36 Nucleotide Sequence |
| | CGAGCA |
| 39 | Transposon Tag 37 Nucleotide Sequence |
| | CGCATA |
| 40 | Transposon Tag 38 Nucleotide Sequence |
| | CGGCGT |
| 41 | Transposon Tag 39 Nucleotide Sequence |
| | CGGTCC |
| 42 | Transposon Tag 40 Nucleotide Sequence |
| | CGTTAT |
| 43 | Transposon Tag 41 Nucleotide Sequence |
| | CTAGGT |
| 44 | Transposon Tag 42 Nucleotide Sequence |
| | CTATTA |
| 45 | Transposon Tag 43 Nucleotide Sequence |
| | CTCAAT |
| 46 | Transposon Tag 44 Nucleotide Sequence |
| | CTGTGG |
| 47 | Transposon Tag 45 Nucleotide Sequence |
| | CTTACG |
| 48 | Transposon Tag 46 Nucleotide Sequence |
| | CTTGAA |
| 49 | Transposon Tag 47 Nucleotide Sequence |
| | GAAATA |
| 50 | Transposon Tag 48 Nucleotide Sequence |
| | GAAGGG |
| 51 | Transposon Tag 49 Nucleotide Sequence |
| | GACTCG |
| 52 | Transposon Tag 50 Nucleotide Sequence |
| | GAGCTT |
| 53 | Transposon Tag 51 Nucleotide Sequence |
| | GAGGCC |
| 54 | Transposon Tag 52 Nucleotide Sequence |
| | GAGTGA |
| 55 | Transposon Tag 53 Nucleotide Sequence |
| | GATCAA |
| 56 | Transposon Tag 54 Nucleotide Sequence |
| | GCCAGA |
| 57 | Transposon Tag 55 Nucleotide Sequence |
| | GCCGTT |
| 58 | Transposon Tag 56 Nucleotide Sequence |
| | GCGAAT |
| 59 | Transposon Tag 57 Nucleotide Sequence |
| | GCGCGG |
| 60 | Transposon Tag 58 Nucleotide Sequence |
| | GCTCCC |
| 61 | Transposon Tag 59 Nucleotide Sequence |
| | GCTGAG |
| 62 | Transposon Tag 60 Nucleotide Sequence |
| | GCTTGT |
| 63 | Transposon Tag 61 Nucleotide Sequence |
| | GGACGA |
| 64 | Transposon Tag 62 Nucleotide Sequence |
| | GGATTG |
| 65 | Transposon Tag 63 Nucleotide Sequence |
| | GGCCAT |
| 66 | Transposon Tag 64 Nucleotide Sequence |
| | GGGATC |
| 67 | Transposon Tag 65 Nucleotide Sequence |
| | GGTAGG |
| 68 | Transposon Tag 66 Nucleotide Sequence |
| | GGTGCT |
| 69 | Transposon Tag 67 Nucleotide Sequence |
| | GTACAG |
| 70 | Transposon Tag 68 Nucleotide Sequence |
| | GTCCTA |
| 71 | Transposon Tag 69 Nucleotide Sequence |
| | GTCGGC |
| 72 | Transposon Tag 70 Nucleotide Sequence |
| | GTGGTG |
| 73 | Transposon Tag 71 Nucleotide Sequence |
| | GTTAAC |
| 74 | Transposon Tag 72 Nucleotide Sequence |
| | GTTTCA |
| 75 | Transposon Tag 73 Nucleotide Sequence |
| | TAAGCT |
| 76 | Transposon Tag 74 Nucleotide Sequence |
| | TAATAG |
| 77 | Transposon Tag 75 Nucleotide Sequence |
| | TACCGA |
| 78 | Transposon Tag 76 Nucleotide Sequence |
| | TAGAGG |
| 79 | Transposon Tag 77 Nucleotide Sequence |
| | TATTTC |
| 80 | Transposon Tag 78 Nucleotide Sequence |
| | TCAGTG |
| 81 | Transposon Tag 79 Nucleotide Sequence |
| | TCATCA |
| 82 | Transposon Tag 80 Nucleotide Sequence |
| | TCCAAG |
| 83 | Transposon Tag 81 Nucleotide Sequence |
| | TCGCCT |
| 84 | Transposon Tag 82 Nucleotide Sequence |
| | TCGGGA |
| 85 | Transposon Tag 83 Nucleotide Sequence |
| | TCTAGC |
| 86 | Transposon Tag 84 Nucleotide Sequence |
| | TGAATT |
| 87 | Transposon Tag 85 Nucleotide Sequence |
| | TGAGAC |
| 88 | Transposon Tag 86 Nucleotide Sequence |
| | TGCGGT |
| 89 | Transposon Tag 87 Nucleotide Sequence |
| | TGCTAA |
| 90 | Transposon Tag 88 Nucleotide Sequence |
| | TGGCAG |
| 91 | Transposon Tag 89 Nucleotide Sequence |
| | TGTGTA |
| 92 | Transposon Tag 90 Nucleotide Sequence |
| | TGTTCG |
| 93 | Transposon Tag 91 Nucleotide Sequence |
| | TTAAGA |
| 94 | Transposon Tag 92 Nucleotide Sequence |
| | TTCGCA |
| 95 | Transposon Tag 93 Nucleotide Sequence |
| | TTCTTG |
| 96 | Transposon Tag 94 Nucleotide Sequence |
| | TTGCTC |
| 97 | Transposon Tag 95 Nucleotide Sequence |
| | TTGGAT |
| 98 | Transposon Tag 96 Nucleotide Sequence |
| | TTTGGG |
| 99 | Tn5 Transposase Recognition Sequence |
| | AGATGTGTATAAGAGACAG |
| 100 | Reverse Transcription Primer Nucleotide Sequence (NEB #S1327S) |
| | d(T)23VN |
| 101 | Reverse Transcription Primer Nucleotide Sequence (Thermo Scientific #SO142) |
| | d(NNNNNN), N = G, A, T, or C |
| 102 | S-P5 Primer Nucleotide Sequence |
| | AATGATACGGCGACCACCGAGATCTACACTCTTTCCCTACACGACGCTC |
| 103 | S-bio-P5 Primer Nucleotide Sequence |
| | Bio-AATGATACGGCGACCACCGAGATCTACACTCTTTCCCTACACGACGCTC |
| 104 | S-P7 Primer Nucleotide Sequence |
| | CAAGCAGAAGACGGCATACGAGAT[NNNNNNNN]GTCTCGTGGGCTCGG |
| 105 | S-P7 Primer Tag 1 Nucleotide Sequence |
| | CGAGTAAT |
| 106 | S-P7 Primer Tag 2 Nucleotide Sequence |
| | TCTCCGGA |
| 107 | S-P7 Primer Tag 3 Nucleotide Sequence |
| | AATGAGCG |
| 108 | S-P7 Primer Tag 4 Nucleotide Sequence |
| | GGAATCTC |
| 109 | S-P7 Primer Tag 5 Nucleotide Sequence |
| | TTCTGAAT |
| 110 | S-P7 Primer Tag 6 Nucleotide Sequence |
| | ACGAATTC |
| 111 | S-P7 Primer Tag 7 Nucleotide Sequence |
| | AGCTTCAG |
| 112 | S-P7 Primer Tag 8 Nucleotide Sequence |
| | GCGCATTA |
| 113 | S-P7 Primer Tag 9 Nucleotide Sequence |
| | CATAGCCG |
| 114 | S-P7 Primer Tag 10 Nucleotide Sequence |
| | TTCGCGGA |
| 115 | S-P7 Primer Tag 11 Nucleotide Sequence |
| | GCGCGAGA |
| 116 | S-P7 Primer Tag 12 Nucleotide Sequence |
| | CTATCGCT |
| 117 | Human TCR outer-1 Primer |
| | TGAAGGCGTTTGCACATGCA |
| 118 | Human TCR outer-2 Primer |
| | TCAGGCAGTATCTGGAGTCATTGAG |
| 119 | Human TCR inner-1 Primer |
| | CTGGTTGCTCCAGGCAATGG |
| 120 | Human TCR inner-2 Primer |
| | TGTAGGCCTGAGGGTCCGT |

| | |
|---|---|
| Note: N=A, T, G, or C; "bio" represents biotin modification; V=A, G, or C. | |

### DETAILED DESCRIPTION OF THE INVENTION

The following examples are provided to illustrate the present invention (but not to limit the scope of the invention) with reference to the specific Examples. Unless otherwise specified in the examples, conventional conditions or conditions recommended by manufacturers are used. Reagents or instruments not specified with a manufacturer are standard products available for purchase in the market. Those skilled in the art will appreciate that the examples are illustrative and do not intend to limit the scope of protection sought in this application.

### Example 1: Preparation of Single-End Specific Oligonucleotide-Tagged Tn5 Transposase Complex

The reagents/instruments used in this example are shown in Table 2:

**Table 2 Reagents and Instruments**

| Reagent/Instrument Name | Manufacturer | Catalog Number |
|---|---|---|
| TruePrep^{®} Tagment Enzyme | Vazyme | S601-01 |
| Tris-HCl pH 8.0 | Thermo Fisher Scientific | AM9855G |
| EDTA, 0.5M pH 8.0 | Promega | V4231 |
| Transposase Adapter 1 (SEQ ID NO: 1) : | | |
| [5Phos]-CTGTCTCTTATACACATCT/3ddC/ | | Biotech Synthesis |
| Biotinylated Transposase Adapter 2 ( SEQ ID NO: 2 ) : 5'- | | Custom Synthesis (96 variations synthesized in this example) |
| GACGTGTGCTCTTCCGATCT[NNNNNN]AGATGTGTATAAGAGAC AG-3' | | |
| Where [NNNNNN] represents the biotin label sequence | | |
| 96-well PCR Plate | Eppendorf | 0030129504 |
| 96-well PCR Plate Sealing Film | Axygen | AM-96-PCR-RD |
| 0.2mL Clear Flat Cap Low Binding PCR Tube | Axygen | PCR-02-L-C |
| Mini Centrifuge for Microplates | Shanghai Yiheng Scientific Instrument | SB-YH0461 |
| PCR Machine | BioRad | 1851197 |

| | | |
|---|---|---|
| Note: "5Phos" represents 5'-end phosphorylation modification; "3ddC" represents 3'-end cytidine dideoxynucleotide. | | |

### Experimental Method:

### 1. Transposon Preparation

(1) Dissolve transposon adapter 1 (SEQ ID NO: 1) and labeled transposon adapter 2 (SEQ ID NO: 2) separately in Annealing buffer from the TruePrep^{®} Tagment Enzyme Kit to a final concentration of 100 µM. Mix transposon adapter 1 with 96 different labeled transposon adapters 2 (sequences indicated as SEQ ID Nos: 3-98) in a 1:1 volume ratio. The mixing steps are as follows: take 10 µL of adapter 1 and adapter 2 each, mix thoroughly in a 96-well PCR plate, seal the plate with a silicone cover, and briefly centrifuge in a mini centrifuge to ensure that the solution is collected at the bottom of the tubes.
(2) Place the mixture of transposon adapter 1 and labeled transposon adapter 2 prepared in step (1) in a PCR instrument and perform the following annealing reaction program: (lid temperature: 105°C) 75°C for 15 minutes, 60°C for 10 minutes, 50°C for 10 minutes, 40°C for 10 minutes, 25°C for 30 minutes. The annealed mixture of adapters obtained is the transposon, which is stored at -20°C for later use.

### 2. Encapsulation of Tn5 Transposase Complex

(1) Take a new 96-well PCR plate and prepare the reaction solution as follows using TruePrep^{®} Tagment Enzyme Kit's TruePrep Tagment Enzyme (2 µg/µl) and Coupling Buffer. Each well contains: 2.5 µL TruePrep Tagment Enzyme (2 µg/µL), 8.25 µL Coupling Buffer, and 1.75 µL labeled transposon (obtained from step 1 above).
(2) Gently tap the reaction solution 20 times using a pipette to mix thoroughly. Seal the 96-well PCR plate with a silicone cover, briefly centrifuge, and place it in a PCR instrument. Perform the reaction at 30°C for 1 hour (with a heat lid at 50°C). After the reaction, the single-stranded oligonucleotide-labeled Tn5 transposase complex is obtained and can be stored at -20°C for up to 1 year.

### 3.Transposase Complex Efficiency Assay

(1) The efficiency of the embedded Tn5 transposase complex needs to be assessed for fragmenting before it can be used in subsequent experiments. In this Example, intact mouse genomic DNA is used as the detection target.
(2) Prepare the reaction mixture by combining 4 µl of 5 × Tagment Buffer L from the TruePrep^{®} Tagment Enzyme kit, 1 µl of 100 ng/µl DNA obtained from step 2, and 1 µl of Tn5 transposase complex. Gently pipette the reaction mixture 20 times to ensure thorough mixing, briefly centrifuge, and place it in a PCR instrument at 55°C for 10 minutes (with a heat lid at 105°C). After the sample temperature has cooled to 4°C, remove the PCR tube, add 5 µl of stop reaction buffer (100 mM Tris-HCl pH 8.0, 200 mM EDTA), mix thoroughly, and incubate at room temperature for 5 minutes.
(3) Perform agarose gel electrophoresis on the reaction products from step (2) to validate the fragmenting efficiency of the Tn5 transposase complex.

The experimental results are shown in Figure 2. The results indicate that the Tn5 transposase complex can fragment mouse genomic DNA (23 kb in full length) into bands of 300-600 bp.

### Example 2: Preparation of Single-Cell Nuclei

The reagents and instruments used in this Example are shown in Table 3:

**Table 3: Reagents and Instruments**

| Reagent/Instrument | Manufacturer | Catalog Number |
|---|---|---|
| 1x PBS | Gibco | 14190-094 |
| 1M Tris-HCl pH7.5 | Jena Bioscience | BU-125S |
| 1M MgCl₂ | Merck Millipore | 20-303 |
| 5M NaCl | Biotech Synthesis Company | B548121-0100 |
| Tween-20 | Sigma | P7949-500ML |
| IGEPAL CA-630 | Sigma | I8896-50ML |
| BSA (Bovine Serum Albumin) | Sigma | A8806-5 |
| Digitonin | Abcam | ab141501 |
| SUPERase-In RNase Inhibitor | Thermo Fisher Scientific | AM2696 |
| nuclease-free water | Invitrogen | AM9932 |
| Flowmi Cell Strainer, 40 µM | Bel-Art | H13680-0040 |
| 1.5 mL low-binding DNA tube | Eppendorf | 30108051 |
| HEK293T cell line | Cell Bank of the Chinese Academy of Sciences | GNHu17 |
| Hela cell line | Cell Bank of the Chinese Academy of Sciences | TCHu187 |
| K562 cell line | Cell Bank of the Chinese Academy of Sciences | SCSP-5054 |
| Centrifuge machine | Thermo Fisher Scientific | Micro21R |
| Fully automated fluorescence cell counter | LUNA | LUNA FL |

### Experimental Method:

1. Prepare a fresh lysis buffer (10 mM Tris-HCl, pH 7.4, 10 mM NaCl, 3 mM MgCl2, 0.1% Tween-20, 0.1% IGEPAL CA-630, 1% BSA, 0.01% digitonin, 1% SUPERase-In RNase Inhibitor), wash buffer (10 mM Tris-HCl, pH 7.4, 10 mM NaCl, 3 mM MgCl2, 0.1% Tween-20, 1% BSA, 1% SUPERase-In RNase Inhibitor), and sample dilution buffer (1x PBS with 1% BSA and 1% SUPERase-In RNase Inhibitor), and keep them on ice for pre-cooling.
2. Nuclei extraction can be performed using fresh tissues, fresh cell lines, fresh blood samples, primary cells, cryopreserved cells, or tissues preserved by flash-freezing in liquid nitrogen, depending on experimental requirements. In this Example, HEK293T cell line (obtained from the Cell Bank of the Chinese Academy of Sciences, catalog number GNHu17), Hela cell line (obtained from the Cell Bank of the Chinese Academy of Sciences, catalog number TCHu187), and K562 cell line (obtained from the Cell Bank of the Chinese Academy of Sciences, catalog number SCSP-5054) were used for the experiments. After culturing and obtaining single-cell suspensions of the cell lines, the cells were washed twice with 1X PBS, and then 2 million cells were separately collected into 1.5 mL centrifuge tubes for single-cell nucleus extraction. The steps for nucleus extraction were as follows:
   (1) The cell pellet was resuspended in 500 µL of pre-chilled lysis buffer, gently pipetted up and down 10 times using a 1 mL pipette, ensuring even resuspension of the cells. The tube was then placed on ice and incubated for 5 minutes, followed by the addition of 1 mL of pre-chilled wash buffer and gentle pipetting up and down 5 times. The tube was centrifuged at 4°C, 500g, and the supernatant was removed.
   (2) The pellet obtained from step (1) was resuspended in 50 µL of nucleus dilution buffer. A small amount was taken for microscopic examination, and if cell aggregates were observed, the suspension was filtered through a 40 µm cell strainer. The prepared nuclei were kept on ice, thoroughly mixed, and 1 µL was used for cell counting using an automated fluorescence cell counter. The nucleus extraction process resulted in a loss of approximately 40-50% of the nuclei.

### Example 3: Single-cell Suspension Permeabilization

The reagents/instruments used in this Example are listed in Table 4:

**Table 4: Reagents and Instruments**

| Reagent/Instrument | Manufacturer | Catalog Number |
|---|---|---|
| Methanol | Fisher Chemical | M/4000/17 |
| 1x PBS | Gibco | 14190-094 |
| BSA (Bovine Serum Albumin) | Sigma | A8806-5 |
| SUPERase-In RNase Inhibitor | Thermo Fisher Scientific | AM2696 |
| nuclease-free water | Invitrogen | AM9932 |
| Flowmi Cell Strainer, 40 µm | Bel-Art | H13680-0040 |
| 1.5 mL low-binding DNA tube | Eppendorf | 30108051 |
| Centrifuge | Thermo Fisher Scientific | Micro21R |
| Automated fluorescence cell counter | LUNA | LUNA FL |

### Experimental Method:

1. Prepare a dilution buffer for fresh samples (1x PBS with 1% BSA and 1% SUPERase-In RNase Inhibitor).
2. Depending on the experimental requirements, perform complete single-cell library preparation using fresh tissues, fresh cell lines, fresh blood samples, primary cells, or cryopreserved cell samples. Prior to library preparation, cells need to be permeabilized. In this Example, cell permeabilization was performed on HEK293T cell line, Hela cell line, and K562 cell line. The steps for cell permeabilization are as follows:
   (1) After culturing and obtaining single-cell suspensions of the cell lines, wash the cells twice with 1X PBS. Then, transfer 2 million cells into a 1.5 mL centrifuge tube and resuspend the cells in 1.5 mL of pre-chilled methanol. Mix thoroughly using a 1 mL pipette, and incubate at -20°C for 10 minutes.
   (2) Centrifuge at 4°C, 500g, and remove the supernatant. Wash the cells twice with 1 mL of the dilution buffer, centrifuge at 4°C, 500g, and remove the supernatant. Finally, resuspend the cells in 50 µL of the dilution buffer.
   (3) Take a small amount of the suspension for microscopic examination. If cell aggregates are observed, filter the suspension through a 40 µm cell strainer. Keep the prepared cells on ice, thoroughly mix, and use 1 µL for cell counting using an automated fluorescence cell counter. Cell permeabilization process may result in a loss of approximately 30-40% of the cells.

### Example 4: Preparation of Single-Cell Transcriptome Library

The reagents and instruments used in this example are shown in Table 5 below:

**Table 5: Reagents and Instruments**

| Reagent/Instrument Name | Manufacturer | Product Number |
|---|---|---|
| Maxima H Minus Reverse Transcriptase | Thermo Fisher Scientific | EP0753 |
| RNaseOUT RNase inhibitor | Thermo Fisher Scientific | 10777019 |
| Deoxy-NTP Set | ROCHE | 11969064001 |
| 100 mM DTT | Invitrogen | Y00147 |
| nuclease-free water | Invitrogen | AM9932 |
| Oligo d(T)23 VN | NEB | S1327S |
| Random Hexamer Primer | Thermo Fisher Scientific | SO142 |
| TruePrep ^{®} Tagment Enzyme | Vazyme | S601-01 |
| Tween-20 | Sigma | P7949-500ML |
| Digitonin | Abcam | ab141501 |
| Tris-HCl pH 8.0 | Thermo Fisher Scientific | AM9855G |
| EDTA, 0.5M pH 8.0 | Promega | V4231 |
| Ficoll solution | Sigma | F5415-25ML |
| Single Cell 5 ' Gel Beads | 10X genomics | 220112 |
| Additive A | 10X genomics | 220074 |
| Chip A Single Cell Kit | 10X genomics | 120236 |
| Dynabeads MyOne SILANE | 10X genomics | 2000048 |
| EB | Qiagen | 19086 |
| SPRIselect Reagent Kit | Beckman Coulter | B23318 |
| Dynabeads MyOne Streptavidin C1 | Invitrogen | 65001 |
| NEBNext High-Fidelity 2x PCR Master Mix | NEB | M0541S |
| KAPA HiFi HotStart 2X ReadyMix | Kapa | KK2602 |
| S-bio-P5 primer (SEQ ID NO: 103) : | | Sangon Biotech |
| 5'Bio- | | |
| AATGATACGGCGACCACCGAGATCTACACTCTTTCCCTACACGAC GCTC-3' | | |
| S-P5 primer (SEQ ID NO:102) : | | Sangon Biotech |
| 5- AATGATACGGCGACCACCGAGATCTACACTCTTTCCCTACACGAC GCTC-3' | | |
| S-P7 primer (SEQ ID NO: 104) : 5'- | | Sangon Biotech (12 examples were synthesized in this Example) |
| CAAGCAGAAGACGGCATACGAGAT[NNNNNNNN]GTCTCGTGGGC TCGG-3 | | |
| [NNNNNNNN] represents a concatenated sequence of eight nucleotides | | |
| 10X Chip Holder | 10X Genomics | 330019 |
| 10X Magnetic Separator | 10X Genomics | 120250 |
| 1.5 mL DNA LoBind^{®} tubes | Eppendorf | 30108051 |
| 96-well PCR plate | Eppendorf | 0030129504 |
| Silicone sealing mat for 96-well PCR plate | Axygen | AM-96-PCR-RD |
| 0.2 mL Low-bind PCR tubes with flat caps, thin wall | Axygen | PCR-02-L-C |
| Mini centrifuge for microplates | Shanghai Yiheng Scientific Instrument Co., Ltd. | SB-YH0461 |
| Thermo shaker | Hangzhou Aosheng Instrument Co., Ltd. | MSC-100 |
| PCR machine | Bio-Rad Laboratories | 1851197 |
| Centrifuge | Thermo Fisher Scientific | Micro21R |
| Chromium Controller | 10X genomics | / |

### Experimental Method:

### 1.Reverse Transcription

(1) Count 200,000 single-cell nuclei obtained from Example 2 and 200,000 permeabilized cell samples obtained from Example 3. Transfer each to 200 µL PCR tube and add 3 µL of 25 µM reverse transcription primer (SEQ ID NO: 100). The total reaction volume is 10 µL, and any remaining volume can be filled with nuclease-free water. The reverse transcription reaction system used in this experiment is typically suitable for reverse transcription of 50,000 to 500,000 cells/nuclei. 200,000 cells/nuclei were exemplified in this experiment. However, it is understood that if fewer or more cells/nuclei are required, the volume of the reverse transcription reaction system can be reduced or increased accordingly.
(2) Gently mix with pipette and incubate in PCR machine (with heated lid at 105°C) at 55°C for 5 minutes, then immediately transfer to ice for at least 2 minutes.
(3) Add 30 µL of reverse transcription reaction mixture (8 µL 5x Reverse Transcription Buffer, 2 µL 100 mM DTT, 2 µL 10 mM dNTPs, 2 µL RNaseOUT RNase inhibitor, 2.5 µL Maxima H Minus Reverse Transcriptase, 13.5 µL nuclease-free water). Mix thoroughly with pipette and place in the PCR machine for the following reaction: (with heated lid at 60°C) 50°C for 10 minutes; 3 cycles of [8°C for 12 seconds, 15°C for 45 seconds, 20°C for 45 seconds, 30°C for 30 seconds, 42°C for 2 minutes, 50°C for 3 minutes]; 50°C for 5 minutes, store at 4°C.

### 2. Single-end Transposition Reaction (Loading First Index Sequence)

The cDNA products obtained from Step 1 in Example 1 were subjected to a transposition reaction using the single-end specific oligonucleotide-tagged TN5 transposase complex prepared in Example 1 to load the first index sequence. The experimental steps are as follows:
(1) Prepare the transposition reaction mixture for the reverse-transcribed samples according to the number of cells to be sequenced. For nuclear samples, the reaction mixture per well is as follows: 4 µL of 5x Reaction Buffer (purchased from Vazyme, product number: S601-01), 2000-10000 nuclei/reaction, and add nuclease-free water to a final volume of 18.2 µL. For cell samples, the reaction mixture per well is as follows: 4 µL of 5x Reaction Buffer (purchased from Vazyme, product number: S601-01), 2000-10000 cells/reaction, 0.2 µL of 1% Digitonin, and add nuclease-free water to a final volume of 18.2 µL.
(2) Add the prepared cell reaction mixture to a 96-well plate containing pre-dispensed TN5 transposase complex with single-end specific oligonucleotide tags (1.8 µL per well). Mix thoroughly with pipette, seal the plate with a silicone sealing mat for 96-well PCR plates, centrifuge briefly, and incubate in thermo shaker at 37°C and 1000 rpm for 30 minutes.
(3) After incubation, collect all reaction mixtures from the wells of the 96-well plate into two 1.5 mL centrifuge tubes (approximately 1 mL per tube). Add 200 µL of reaction stop solution (100 mM Tris-HCl pH 8.0, 200 mM EDTA), mix thoroughly, and let it sit at room temperature for 5 minutes. Centrifuge at 4°C, 500g, and remove the supernatant. Wash twice with 200 µL of sample dilution buffer (1x PBS with 1% BSA and 1% SUPERase-In RNase Inhibitor).

### 3. Preparation of Water-in-oil Droplet and Template Switching Reaction (Loading of Second Barcode Sequence)

In this Example, the preparation of water-in-oil droplet and the template switching reaction to load a unique barcode label (second barcode sequence) into each droplet is performed using the 10X Genomics Chromium platform and 10X Single Cell 5' Gel Beads as an example. The oil-encapsulation process and cell barcode labeling using gel beads can be substituted with other platforms. The experimental steps are as follows:
(1) Preparation of Template Switching Reaction Mix: Resuspend the cell or nuclei pellet obtained from the previous step in the Template Switching Reaction Mix (20 µl 5x Reverse Transcription Buffer, 1 µl 100 mM DTT, 10 µl 10 mM dNTPs, 2 µl RNaseOUT RNase inhibitor, 2.4 µl Additive A, 10 µl 20% Ficoll, 5 µl Maxima H Minus Reverse Transcriptase, 49 µl nuclease-free water). The number of cells or nuclei resuspended in this step is approximately 110,000.
(2) Following the instructions in the 10X Chromium Single Cell V(D)J Reagent Kits User Guide, load 90 µl of the cell reaction mix, 40 µl of 10X Single Cell 5' Gel Beads, and 270 µl of partitioning oil onto the 10X Chip A chip (load 50% glycerol in empty wells as required by the user guide). Perform oil encapsulation using the 10X Genomics Chromium instrument, which takes approximately 7 minutes. The number of cells loaded on the chip for oil encapsulation in this step is approximately 100,000.
(3) Carefully collect the water-in-oil droplet products into a 200 µl PCR tube and quickly place it in a PCR machine. Perform the reaction under the following conditions: (with heated lid at 105°C) 25°C for 30 minutes, 42°C for 90 minutes, 53°C for 10 minutes, and store at 4°C temporarily.

### 4. The Water-in-oil Microdroplet Purification

Following the instructions in the 10X Chromium Single Cell V(D)J Reagent Kits User Guide, the water-in-oil microdroplets were purified, and the final elution was performed using 35.5 µL of EB buffer.

### 5. cDNA Amplification

Prepare the PCR amplification reaction system in a 200 µL PCR tube: 50 µL of NEBNext High-Fidelity 2x PCR Master Mix, 0.5 µL of 100 mM S-P5 primer (SEQ ID NO: 102), 35 µL of the purified product obtained from the above step 4, and 11.5 µL of nuclease-free water. Mix well and place it in a PCR machine. The reaction conditions are as follows: (with a heated lid at 105°C) 72°C for 3 minutes, 98°C for 45 seconds, followed by 13 cycles (determined based on the number of cells loaded) of [98°C for 20 seconds, 67°C for 30 seconds, 72°C for 1 minute], and a final extension step at 72°C for 1 minute. Store at 4°C temporarily.

### 6. Purification and Recovery of cDNA Amplification Product

(1) Perform cDNA purification using 0.8x SPRIselect magnetic beads. Add 80 µL of 0.8x SPRIselect magnetic beads to the 100 µL reaction product from step 5 and mix thoroughly using a pipette.
(2) Incubate at room temperature for 5 minutes, then place the tube on magnetic separator. When the magnetic beads are fully immobilized on the magnet and the solution becomes clear, remove the supernatant.
(3) Wash the magnetic beads twice with 200 µL of freshly prepared 80% ethanol, discard the supernatant, and allow the beads to air dry. Resuspend the beads in 41 µL of EB buffer, let it sit at room temperature for 2 minutes, then place the tube on a magnetic separator. When the magnetic beads are fully immobilized on the magnet and the solution becomes clear, collect the supernatant into a new centrifuge tube.
(4) Take 1 µL of the eluted cDNA, measure the concentration using Qubit, and the samples can be stored at -80°C for up to 3 months.

### 7.Pre-amplification of Sequencing Library

(1) Perform PCR amplification on 20 µl of the product from step 6 using the following reaction mixture: 50 µl of KAPA HiFi HotStart 2X ReadyMix, 1 µl of 100 mM S-bio-P5 primer (SEQ ID NO: 103), 4 µl of 25 mM S-P7 primer (SEQ ID NO: 104, take 1 µl from each of the four 25 mM S-P7 primers, whose tag sequences are shown as SEQ ID NOs: 105-108), 20 µl of the product from step 6, and 25 µl of nuclease-free water. Mix well and place the tube in a PCR machine. The reaction conditions are as follows: (with heated lid at 105°C) 98°C for 45 seconds, 8 cycles (the number of cycles can be adjusted based on the concentration of the product from step 6) of [98°C for 20 seconds, 54°C for 30 seconds, 72°C for 20 seconds], 72°C for 1 minute, and store at 4°C temporarily.
(2) Perform specific enrichment of the biotinylated nucleic acid fragments from the previous step using 10 µl of Dynabeads MyOne Streptavidin C1 beads. This pre-amplification helps to further amplify the cDNA fragments from the desired source. After pre-amplification, resuspend the beads in 20.5 µl of EB buffer and set aside.

### 8. Sequencing Library Secondary Amplification

Take 20 µL of the product from step 7 and perform PCR amplification using the following reaction mixture: 50 µL KAPA HiFi HotStart 2X ReadyMix, 1 µL of 100 mM S-P5 primer (SEQ ID NO: 102), 4 µL of 25 mM S-P7 primers (1 µL each from the four 25 mM S-P7 primers), 20 µL of the product from step 6, and 25 µL of nuclease-free water. After thorough mixing, place the reaction mixture in a PCR machine with the following conditions: (with heated lid at 105°C) 98°C for 45 seconds, 8 cycles (the number of cycles can be adjusted based on the concentration of the product from step 7) of [98°C for 20 seconds, 54°C for 30 seconds, 72°C for 20 seconds], 72°C for 1 minute, and store at 4°C.

### 9. Library Purification and Fragment Size Selection

Purify and select fragments of the above product using 0.55X and 0.2X SPRIselect beads. Finally, obtain a sequencing library with fragment sizes around 300-600 bp.

### 10. Sequencing

The constructed library is sequenced using the NovaSeq 6000 system (Illumina, San Diego, CA) with paired-end reads of 150 bp. Each cell is sequenced with 50,000 reads.

### 10.Data Analysis

### 10.1 Pseudo-Single Cell Rate

According to data published by 10X Genomics, during the library preparation process using the 10X Genomics Chromium platform and reagent kits for 5' end transcriptome sequencing, the actual number of cells captured is typically around 57% of the cells loaded onto the machine for droplet generation (i.e., a capture rate of approximately 57%). Furthermore, the pseudo-single cell rate is linearly correlated with the number of cells loaded onto the machine, meaning that higher cell numbers lead to higher pseudo-single cell rates (refer to Figure 3). When the number of cells loaded onto the machine is 100,000, the number of cells captured in the droplets is approximately 57,000, with a pseudo-single cell rate of approximately 45.65% (data source: USER GUIDE of Chromium Next GEM Single Cell V(D)J Reagent Kits v1.1, 10X Genomics). However, the sequencing data generated from these pseudo-single cells consumes significant sequencing costs without providing any useful information, and thus needs to be filtered out. To maintain the pseudo-single cell rate within a reasonable range (e.g., below 8%), 10X Genomics recommends that the number of cells loaded onto the machine should not exceed 10,000.

In this Example, 100,000 cell nuclei or permeabilized cells, pre-loaded with the first tag, were used for droplet generation and construction of the transcriptome library, followed by sequencing. After sequencing, the sequencing data was analyzed using the sequences of the first and second tags. The analysis results showed that during droplet generation, 59,622 cell nuclei were captured (capture rate of 59.62%), and 58,771 permeabilized cells were captured (capture rate of 58.77%), confirming the capture efficiency consistent with the data provided by 10X Genomics. The analysis results also revealed that for experiments using cell nuclei, the percentage of droplets containing only one type of first tag was 65.37%; droplets containing two types of first tags accounted for 25.93%; droplets containing three types of first tags accounted for 6.95%; and droplets containing more than three types of first tags accounted for 1.75% (refer to Figure 4). In other words, for experiments using cell nuclei, the percentage of droplets containing two or more types of first tags was 34.63%. Here, the number of first tags in an individual droplet essentially reflects the number of cell nuclei in that droplet (excluding the case where a single droplet contains two or more cell nuclei with the same first tag). Therefore, in the droplets prepared in this Example, the percentage of droplets containing two or more cells was 34.63%. Similar results were obtained for experiments using permeabilized cells: droplets containing only one type of first tag accounted for 57.85%; droplets containing two types of first tags accounted for 28.71%; droplets containing three types of first tags accounted for 9.82%; and droplets containing more than three types of first tags accounted for 3.62% (refer to Figure 4). In other words, for experiments using permeabilized cells, the percentage of droplets containing two or more types of first tags was 42.15%.

For conventional 10X Genomics transcriptome sequencing protocols, such doublets or multiplets rates (34.63% or 42.15%) are considered unacceptable as they can lead to the generation of a significant amount of non-informative sequencing data. However, in the method described in this Example, since each individual cell or nucleus is loaded with the first barcode prior to encapsulation in oil droplets, sequencing data generated from such droplets containing two or more cells or nuclei can be deconvoluted using the sequence of the first barcode to obtain sequencing data for each individual cell within the droplet. Therefore, by utilizing the method described in this Example, sequencing data derived from doublets or multiplets (containing two or more cells or nuclei) can be used for analyzing individual cells without the need for filtering or removal. This greatly reduces sequencing costs. Additionally, due to the compatibility of the entire method with a higher number of cells (e.g., at least 100,000 cells or nuclei) for library construction, the library preparation cost is significantly reduced.

### 10.2 Data Quality

In this example, three human cell lines, namely HEK293T cells, Hela cells, and K562 cells, were mixed, and libraries were prepared and sequenced. The sequencing results demonstrated that each cell line exhibited unique highly expressed genes. Furthermore, the sequencing data obtained from both permeabilized cell samples (Figure 5A) and cell nucleus samples (Figure 6A) showed a high degree of consistency. Additionally, dimensionality reduction visualization analysis was performed on the expression matrices of the sequencing data. The results revealed that the sequencing data obtained from both permeabilized cell samples (Figure 5B) and cell nucleus samples (Figure 6B) were able to effectively distinguish between the three cell lines (i.e., the three cell lines were clearly separated into three distinct clusters). These results indicate that the method employed in this example is suitable for high-throughput single-cell transcriptome sequencing. It accurately measures the RNA abundance of individual cells and effectively identifies and characterizes transcriptional differences between different cell types.

### Example 5: Influence of Different Reverse Transcription Primers on the Quality of Single-Cell Transcriptome Data from Nuclei and Permeabilized Cells

The reagents and instruments used in this example are the same as those listed in Table 3-5.

### Experimental Method:

This Example's basic steps include the basic steps of Example 2 for single-cell nucleus preparation, Example 3 for single-cell suspension permeabilization, and Example 4 for single-cell transcriptome library preparation. Specific differences are described as follows:

### 1. Preparation of Reverse Transcription Primers

(1) 25 µM poly T primer (SEQ ID NO: 100);
(2) 25 µM random primer (SEQ ID NO: 101);
(3) 25 µM mixed primer, obtained by combining poly T primer and random primer in a 1:1 molar ratio.

### 2. Preparation of Single-Cell Samples:

A specific cell line was selected for testing. In this Example, Hela cell line (obtained from the Cell Bank of the Chinese Academy of Sciences, catalog number TCHu187) was used to extract cell nuclei according to the method described in Example 2 and to permeabilize cells following the method described in Example 3.

### 3. Reverse Transcription Reaction:

The above permeabilized cell samples and cell nucleus samples were separately placed in three 200 µL PCR tubes at a quantity of 50,000 cells per tube. In each of the three tubes, 3 µL of the reverse transcription primer prepared in Step 1 (poly T primer, random primer, or mixed primer) was added. A total of six experiments were performed. The total reaction volume in each tube was 10 µL, and any remaining volume was supplemented with nuclease-free water. After gently mixing with a pipette, the tubes were incubated at 55°C in a PCR machine (with a heated lid at 105°C) for 5 minutes, then immediately transfer to ice for at least 2 minutes. 30 µL of reverse transcription reaction mixture (8 µL 5x Reverse Transcription Buffer, 2 µL 100 mM DTT, 2 µL 10 mM dNTPs, 2 µL RNaseOUT RNase inhibitor, 2.5 µL Maxima H Minus Reverse Transcriptase, 13.5 µL nuclease-free water) was added to each PCR tube. After thorough mixing with a pipette, the tubes were subjected to the following reaction in the PCR machine: (with a heated lid at 60°C) 50°C for 10 minutes; 3 cycles of [8°C for 12 seconds, 15°C for 45 seconds, 20°C for 45 seconds, 30°C for 30 seconds, 42°C for 2 minutes, 50°C for 3 minutes]; 50°C for 5 minutes, and stored at 4°C temporarily.

### 4. Single-end Transposition Reaction (Loading of the First Index Sequence)

The reverse transcription products obtained from the six species described in step 3 were subjected to transposition reaction using the single-end specific oligonucleotide-labeled TN5 transposase complex prepared according to Example 1. The first index sequence was loaded by performing transposition labeling with eight different single-end specific oligonucleotide-labeled TN5 transposase complexes for each reverse transcription product. Refer to Example 4, step 2, for specific experimental procedures of the single-end transposition reaction. Finally, gently resuspend the samples in 20 µL of dilution buffer (1x PBS with 1% BSA and 1% SUPERase-In RNase Inhibitor) and perform cell counting.

### 5. The Water-in-oil Droplet Preparation and Template Switching Reaction (Loading of the Second Index Sequence)

From the cellular and nuclear products obtained in step 4, take 28,000 for each and perform water-in-oil droplet preparation and template switching reaction. Refer to Example 4, step 3, for the remaining experimental procedures and conditions.

### 6. The Water-in-oil Droplet Purification to Library Purification and Fragment Selection

Refer to Example 4, steps 4-9, for specific experimental procedures.

### 7. Sequencing

The constructed libraries were sequenced using NovaSeq 6000 (Illumina, San Diego, CA) with paired-end sequencing of 150 bp read length, resulting in a total of 125 Gb of raw data.

### 8. Data Analysis

The data obtained in this example, as shown in Figure 7, demonstrated that all three reverse transcription primers achieved nucleic acid detection in permeabilized cells or cell nuclei. Additionally, regardless of sequencing depth at 100 Gb or 125 Gb, using the same primer conditions, more genes were detected in permeabilized cell samples compared to cell nuclei samples. For permeabilized cell samples, more genes were detected using the poly-T primer compared to random primers. For cell nuclei samples, a mixed primer containing both poly-T and random primers detected more genes compared to using a single type of primer. These results indicate that all three reverse transcription primers (poly-T primer, random primer, or mixed primer) can achieve nucleic acid detection in permeabilized cells or cell nuclei. Furthermore, the use of a mixed primer containing both poly-T and random primers enhances the detection capability of single-cell transcriptome genes in cell nuclei samples.

### Example 6: Compatibility of this Single-Cell Sequencing Technology with Amplification of Immune Cell VDJ Sequences

The reagents and instrument information used in this example are shown in Table 6, while the rest remains the same as in Tables 3-5:

**Table 6: Reagents and Instruments**

| Reagent/Instrument Name | Manufacturer | Product Number |
|---|---|---|
| Ficoll Paque PLUS | Cytiva | 17144003 |
| APC anti-human CD3 | Biolegend | 317318 |
| 7-AAD | ABCAM | ab228563 |
| Flow Cytometry Tubes | BD | 352054 |
| Chromium Single Cell 5' Library Construction Kit | 10X Genomics | PN-1000020 |
| Chromium i7 Sample Index | 10X Genomics | PN-220103 |
| Plate well | | |
| Flow Cytometer | BD | FACSAria IIIu |
| Human TCR outer-1Primer (SEQ ID NO: 117) TGAAGGCGTTTGCACATGCA | | Sangon Biotech |
| Human TCR outer-2Primer (SEQ ID NO: 118) TCAGGCAGTATCTGGAGTCATTGAG | | Sangon Biotech |
| Humane TCR inner-1Primer (SEQ ID NO: 119) CTGGTTGCTCCAGGCAATGG | | Sangon Biotech |
| Human TCR inner-2 Primer (SEQ ID NO: 120) TGTAGGCCTGAGGGTCCGT | | Sangon Biotech |

This example describes the use of T cells enriched from human peripheral blood as an example to demonstrate the single-cell library preparation method of the present invention. The method allows for the detection of single-cell transcriptome data while enriching the VDJ regions (including the B cell BCR region or T cell TCR region) from the amplified cDNA products.

Although this example specifically focuses on T cells derived from human peripheral blood, the method is also applicable to the enrichment and sequencing of VDJ regions of T cells and B cells, as well as other immune cells, from different sources and species, as well as the capture of target genes for other purposes.

The enrichment method provided in this example involves the design of specific primers for the target gene or fragment based on its characteristics. The target fragments are specifically enriched together with S-P5 primers, resulting in the enrichment of the target fragments coupled with the second label. This enrichment method is simple and straightforward.

### Experimental Method:

1. PBMC Extraction: 3 mL of human peripheral blood was diluted with an equal volume of PBS, and PBMC was isolated and extracted according to the instructions of Ficoll Paque PLUS.
2. T Cell Enrichment:
   (1) CD3 Antibody Incubation: The isolated PBMC was resuspended in 100 µL of wash buffer (1X PBS with 2% BSA), and 5 µL of APC anti-human CD3 antibody was added. The mixture was incubated on ice in the dark for 30 minutes, washed twice with 500 µL of wash buffer, and centrifuged at 350g to remove the supernatant.
   (2) 7-AAD Staining: The CD3 antibody-incubated cells were resuspended in 100 µL of wash buffer, and 5 µL of 7-AAD was added. The mixture was incubated on ice in the dark for 5 minutes. After incubation, the cells were resuspended in 500 µL of wash buffer, passed through a 70 µm cell strainer, transferred to a flow tube, and kept on ice for sorting.
   (3) Flow Sorting: CD3+ and 7-AAD- cells were sorted using a flow cytometer. These cells represent live T cells.
3. T Cell Permeabilization: See Example 3 for the permeabilization process.
4. Preparation of Single-Cell Transcriptome Library for T Cells.

In this example, T cells were enriched and single-cell transcriptome libraries were prepared from peripheral blood samples of 14 individuals, including 2 healthy individuals and 12 cancer patients, using the aforementioned enrichment method. The library preparation procedure is described in Example 4.

In summary, 15,000 cells were taken for each sample, and reverse transcription was performed using Poly T primers. In the single-end tagmentation reaction, 12 different single-end-specific oligonucleotides labeled with TN5 transposase complexes were used for tagmentation in healthy individuals, while 6 different single-end-specific oligonucleotides labeled with TN5 transposase complexes were used for tagmentation in cancer patients. After the single-end tagmentation reaction, a total of 67,000 cells were collected, and all cells were used for water-in-oil microdroplet generation and template switching reaction.

The remaining steps are consistent with the corresponding steps in Example 4 to obtain single-cell transcriptome data.

### 5. T Cell VDJ Enrichment

From the cDNA products obtained during the preparation of single-cell transcriptome libraries, take 5 µl for subsequent amplification and enrichment.
(1) First Round Nested PCR: Prepare the PCR amplification reaction system in a 200 µl PCR tube: 50 µl KAPA HiFi HotStart 2X ReadyMix, 5 µl 10 mM T MIX 1 (a mixture of Human TCR outer-1 primer, SEQ ID NO: 117, and Human TCR outer-2 primer, SEQ ID NO: 118), 5 µl 10 mM S-P5 primer (SEQ ID NO: 102), 5 µl of the cDNA enrichment product from the previous step, and 35 µl nuclease-free water. Mix well and place in a PCR machine. Reaction conditions: (with heated lid at 105°C) 98°C for 45 seconds, 12 cycles (the number of cycles adjusted according to the concentration of the cDNA enrichment product) of [98°C for 20 seconds, 67°C for 30 seconds, 72°C for 60 seconds], 72°C for 1 minute, store at 4°C temporarily.
(2) Purification of Pre-amplification Product: Purify and fragment select the product from the previous step using 0.5X and 0.3X SPRIselect magnetic beads, followed by elution with 25 µl EB. The goal is to enrich fragments around 600-1000 bp.
(3) Second Round Nested PCR: Prepare the PCR amplification reaction system in a 200 µl PCR tube: 50 µl KAPA HiFi HotStart 2X ReadyMix, 5 µl 10 mM T MIX 2 (a mixture of Human TCR inner-1 primer, SEQ ID NO: 119, and Human TCR inner-2 primer, SEQ ID NO: 120), 5 µl 10 mM S-P5 primer (SEQ ID NO: 102), 25 µl of the first round nested PCR product, and 15 µl nuclease-free water. Mix well and place in a PCR machine. Reaction conditions: (with heated lid at 105°C) 98°C for 45 seconds, 10 cycles (the number of cycles adjusted according to the concentration of the first round nested PCR product) of [98°C for 20 seconds, 67°C for 30 seconds, 72°C for 60 seconds], 72°C for 1 minute, store at 4°C temporarily.
(4) Purification of Amplification Product, same as (2) above.

### 6. VDJ Library Construction

The amplified products can be used for library construction using traditional transcriptome library preparation methods. In this Example, the Chromium Single Cell 5' Library Construction Kit was used for library construction.

### (1) Fragmentation of Products

Take 50 ng of amplified products, add nuclease-free water to a total volume of 20 µL, and add fragmentation reaction mix (including 5 µL fragmentation buffer, 15 µL Fragmentation Enzyme Blend, and 15 µL nuclease-free water). Mix thoroughly on ice and place in a PCR machine. Reaction conditions: (with a heated lid at 65°C) 32°C for 2 minutes, 65°C for 30 minutes, and store at 4°C.

### (2) End Repair & Adaptor Ligation

Add end repair and adaptor ligation reaction mix to the fragmented products (including 20 µL Ligation Buffer, 10 µL DNA Ligase, 2.5 µL Adaptor Mix, and 17.5 µL nuclease-free water). Mix thoroughly and place in a PCR machine. Reaction conditions: (with a heated lid at 30°C) 20°C for 15 minutes, and store at 4°C.

### (3) Product Purification

Purify the products from the previous step using 0.8X SPRIselect magnetic beads and elute with 30 µL EB.

### (4) VDJ Sequencing Library Amplification

Add the above products to a 70 µL reaction mix with the following components: 50 µL KAPA HiFi HotStart 2X ReadyMix, 2 µL SI-PCR Primer, 10 µL individual Chromium i7 Sample Index, and 8 µL nuclease-free water. Mix thoroughly and place in a PCR machine. Reaction conditions: (with a heated lid at 105°C) 98°C for 45 seconds, 9 cycles of [98°C for 20 seconds, 54°C for 30 seconds, 72°C for 20 seconds], 72°C for 1 minute, and store at 4°C.

### 7.VDJ Sequencing Library Purification

Purify the products from the previous step using 0.8X SPRIselect magnetic beads.

### 8.VDJ Sequencing

Sequencing of the constructed library was performed using the NovaSeq 6000 system (Illumina, San Diego, CA) with a read length of 150 bp paired-end sequencing, obtaining 12,500 reads per cell.

### 9.Data Analysis

The data obtained in this Example showed that a total of 41,337 cells were detected in the transcriptome data obtained. Out of these, 4,719 single cells with high expression of TCR were enriched at the current sequencing depth. Analysis of the single-cell transcriptome data from T cells enriched from peripheral blood samples of 14 individuals revealed the presence of 12 distinct T cell types, all of which carried TCR information (100% concordance). The visualization results and cell counts for each cell population are shown in Figure 8.

For the 12 T cell types detected in the transcriptome data, corresponding TCR clones were detected in the VDJ library sequencing data. The visualization results and cell counts for different TCR clone populations detected in the VDJ library sequencing data are shown in Figure 9. The results in Figure 9 indicate that the proportion of cells with detected TCR in each cell type is consistent with the proportion of cells detected in the transcriptome data. Furthermore, the distribution of major TCR clones detected in each of the 14 samples was analyzed, demonstrating the diversity of TCR clones among different individuals (see Figure 10). Additionally, the distribution of TRB and TRA genes detected in each sample was analyzed, showing no preference in the distribution of TCR TRB and TRA genes among different samples (see Figure 11A and Figure 11B).

Based on the above results, it is evident that the present invention is capable of enriching and sequencing the single-cell TCR VDJ region.

Although specific Examples of the invention have been described in detail, those skilled in the art will understand that various modifications and changes can be made based on the teachings provided herein, all of which are within the scope of the invention as defined by the appended claims

## Claims

1. A method for generating labeled nucleic acid molecules, comprising the following steps:
(a) providing multiple bead particles comprising coupled oligonucleotide molecules, wherein said oligonucleotide molecules contains a labeling sequence comprising a second label sequence, wherein each bead particle has multiple oligonucleotide molecules, and the multiple oligonucleotide molecules on the same bead particle have the same second label sequence, while oligonucleotide molecules on different bead particles have different second label sequences;
(b) providing multiple cells or cell nuclei comprising RNA;
(c) performing processing on the RNA within the cells or cell nuclei, including a reverse transcription step to form double-stranded nucleic acids containing cDNA chains;
(d) incubating the double-stranded nucleic acids with a transposase complex comprising a transposase and a transposon sequence that the transposase is used to recognize and bind, and is capable of cleaving or breaking the double-stranded nucleic acids, wherein the transposon sequence contains a transfer chain and a non-transfer chain, the transfer chain comprising a transposase recognition sequence, a first label sequence, and a first common sequence, wherein the first label sequence is located upstream of the transposase recognition sequence, and the first common sequence is located upstream of the first label sequence, and wherein the incubation is performed under conditions that allow the double-stranded nucleic acids to be fragmented by the transposase complex into nucleic acid fragments and for the transposon sequence to be connected to the end of the nucleic acid fragments, thereby forming a group of nucleic acid fragments within the cells or cell nuclei, the nucleic acid fragments comprising cDNA fragments wherein the sequences of the transfer chains are connected to the 5' end of the cDNA fragments and, the nucleic acid fragments contain the first common sequence, the first label sequence, the transposase recognition sequence, and the cDNA fragment from the 5' end to the 3' end; and
(e) contacting the processed cells or cell nuclei of step (d) with the bead particles, wherein the nucleic acid fragments and the oligonucleotide molecules generate labeled nucleic acid molecules comprising the sequence of the nucleic acid fragments and the complementary sequence of the labeling sequence from the 5' end to the 3' end, or comprise the labeling sequence and the complementary sequence of the nucleic acid fragments.

2. The method according to claim 1, wherein in step (e), at least 2 cells or cell nuclei are provided.

3. The method according to claim 1, wherein the cells are derived from animals, plants, or microorganisms, or any combination thereof.

4. The method according to claim 1, wherein the RNA is reverse-transcribed using a reverse transcriptase to form hybrid double-stranded nucleic acid comprising RNA and cDNA chains.

5. The method according to claim 1, wherein in step (e), the nucleic acid fragments are brought into contact with the oligonucleotide molecules by means selected from the following:
(e1) releasing the nucleic acid fragments by treating the processed cells or cell nuclei;
(e2) releasing the oligonucleotide molecules from the beads; or
(e3) a combination of (e1) and (e2).

6. A method for constructing a library of nucleic acid molecules comprising:
(i) generating multiple labeled nucleic acid molecules according to claim 1, and
(ii) recovering and/or combining multiple labeled nucleic acid molecules to obtain a library of nucleic acid molecules.

7. The method according to claim 6, wherein the method further comprises: (iii) enriching the labeled nucleic acid molecules.

8. A method for sequencing nucleic acids from cells or cell nuclei, comprising:
constructing a nucleic acid library according to claim 6; and
sequencing the nucleic acid library.

9. A reagent kit comprising: a reverse transcriptase, a transposase, and a transposase recognition sequence that the transposase can recognize and bind to, wherein the transposase and transposase recognition sequences can form a transposase complex, which is capable of cleaving or breaking double-stranded nucleic acid,
the transposase recognition sequence comprises a transfer chain and a non-transfer chain, the transposase recognition sequence comprises a transposase recognition sequence, a first labeling sequence, and a first common sequence, the first labeling sequence is located upstream of the transposase recognition sequence, and the first common sequence is located upstream of the first labeling sequence,
the reagent kit further comprised beads coupled with multiple oligonucleotide molecules, and the oligonucleotide molecules contain a labeling sequence, the labeling sequence comprises a second label sequence, wherein each bead has multiple oligonucleotide molecules, and the multiple oligonucleotide molecules on the same bead have the same second label sequence, while the oligonucleotide molecules on different beads have different second label sequences;
wherein the reagent kit includes at least 2 transposase recognition sequences and each transposase recognition sequence has a different first label sequence.

10. The kit according to claim 9, further comprising reagents for constructing transcriptome sequencing libraries.

11. The method according to claim 1 for constructing nucleic acid libraries or for conducting transcriptome sequencing.

## Patentansprüche

1. Verfahren zur Erzeugung markierter Nukleinsäuremoleküle, umfassend die folgenden Schritte:
(a) Bereitstellen mehrerer Beads, die gekoppelte Oligonukleotidmoleküle umfassen, wobei die Oligonukleotidmoleküle eine Markierungssequenz enthalten, die eine zweite Markierungssequenz umfasst, wobei jedes Bead mehrere Oligonukleotidmoleküle aufweist, und wobei die mehreren Oligonukleotidmoleküle auf demselben Bead dieselbe zweite Markierungssequenz aufweisen, während Oligonukleotidmoleküle auf unterschiedlichen Beads unterschiedliche zweite Markierungssequenzen aufweisen;
(b) Bereitstellen mehrerer Zellen oder Zellkerne, die RNA enthalten;
(c) Verarbeiten der RNA innerhalb der Zellen oder Zellkerne, einschließlich eines Schrittes der reversen Transkription zur Bildung doppelsträngiger Nukleinsäuren, die cDNA-Stränge enthalten;
(d) Inkubieren der doppelsträngigen Nukleinsäuren mit einem Transposase-Komplex, umfassend eine Transposase und eine Transposon-Sequenz, die von der Transposase erkannt und gebunden wird und die doppelsträngigen Nukleinsäuren schneiden oder spalten kann, wobei die Transposon-Sequenz einen Transferstrang und einen Nicht-Transferstrang umfasst, wobei der Transferstrang eine Transposase-Erkennungssequenz, eine erste Markierungssequenz und eine erste gemeinsame Sequenz umfasst, wobei die erste Markierungssequenz stromaufwärts der Transposase-Erkennungssequenz angeordnet ist und die erste gemeinsame Sequenz stromaufwärts der ersten Markierungssequenz angeordnet ist, und wobei die Inkubation unter Bedingungen erfolgt, die es ermöglichen, dass die doppelsträngigen Nukleinsäuren durch den Transposase-Komplex in Nukleinsäurefragmente fragmentiert werden und dass die Transposon-Sequenz mit den Enden der Nukleinsäurefragmente verbunden wird, wodurch eine Gruppe von Nukleinsäurefragmenten innerhalb der Zellen oder Zellkerne gebildet wird, wobei die Nukleinsäurefragmente cDNA-Fragmente umfassen, wobei die Sequenzen der Transferstränge mit dem 5'-Ende der cDNA-Fragmente verbunden sind, und wobei die Nukleinsäurefragmente von ihrem 5'-Ende zum 3'-Ende die erste gemeinsame Sequenz, die erste Markierungssequenz, die Transposase-Erkennungssequenz und das cDNA-Fragment umfassen; und
(e) In Kontakt bringen der in Schritt (d) verarbeiteten Zellen oder Zellkerne mit den Beads, wobei die Nukleinsäurefragmente und die Oligonukleotidmoleküle markierte Nukleinsäuremoleküle erzeugen, die von ihrem 5'-Ende zum 3'-Ende die Sequenz der Nukleinsäurefragmente und die komplementäre Sequenz der Markierungssequenz umfassen oder die Markierungssequenz und die komplementäre Sequenz der Nukleinsäurefragmente umfassen.

2. Verfahren nach Anspruch 1, wobei in Schritt (e) mindestens zwei Zellen oder Zellkerne bereitgestellt werden.

3. Verfahren nach Anspruch 1, wobei die Zellen von Tieren, Pflanzen oder Mikroorganismen stammen oder eine beliebige Kombination davon sind.

4. Verfahren nach Anspruch 1, wobei die RNA unter Verwendung einer reversen Transkriptase revers transkribiert wird, um eine hybride doppelsträngige Nukleinsäure zu bilden, die RNA- und cDNA-Stränge umfasst.

5. Verfahren nach Anspruch 1, wobei in Schritt (e) die Nukleinsäurefragmente mittels eines der folgenden Mittel mit den Oligonukleotidmolekülen in Kontakt gebracht werden:
(e1) Freisetzen der Nukleinsäurefragmente durch Behandeln der verarbeiteten Zellen oder Zellkerne;
(e2) Freisetzen der Oligonukleotidmoleküle von den Beads; oder
(e3) eine Kombination von (e1) und (e2).

6. Verfahren zur Herstellung einer Nukleinsäurebibliothek, umfassend:
(i) Erzeugen mehrerer markierter Nukleinsäuremoleküle nach Anspruch 1; und
(ii) Gewinnen und/oder Zusammenführen mehrerer markierter Nukleinsäuremoleküle zur Herstellung einer Nukleinsäurebibliothek.

7. Verfahren nach Anspruch 6, ferner umfassend: (iii) Anreichern der markierten Nukleinsäuremoleküle.

8. Verfahren zur Sequenzierung von Nukleinsäuren aus Zellen oder Zellkernen, umfassend:
Herstellen einer Nukleinsäurebibliothek nach Anspruch 6; und
Sequenzieren der Nukleinsäurebibliothek.

9. Reagenzkit, umfassend: eine reverse Transkriptase, eine Transposase und eine Transposase-Erkennungssequenz, die von der Transposase erkannt und gebunden werden kann, wobei die Transposase und die Transposase-Erkennungssequenz einen Transposase-Komplex bilden können, der in der Lage ist, doppelsträngige Nukleinsäuren zu schneiden oder zu spalten,
wobei die Transposon-Sequenz einen Transferstrang und einen Nicht-Transferstrang umfasst, wobei der Transferstrang eine Transposase-Erkennungssequenz, eine erste Markierungssequenz und eine erste gemeinsame Sequenz umfasst, wobei die erste Markierungssequenz stromaufwärts der Transposase-Erkennungssequenz angeordnet ist und die erste gemeinsame Sequenz stromaufwärts der ersten Markierungssequenz angeordnet ist,
wobei das Reagenzkit ferner Beads umfasst, die mit mehreren Oligonukleotidmolekülen gekoppelt sind, wobei die Oligonukleotidmoleküle eine Markierungssequenz enthalten, die eine zweite Markierungssequenz umfasst, wobei jedes Bead mehrere Oligonukleotidmoleküle aufweist, und wobei die mehreren Oligonukleotidmoleküle auf demselben Bead dieselbe zweite Markierungssequenz aufweisen, während Oligonukleotidmoleküle auf unterschiedlichen Beads unterschiedliche zweite Markierungssequenzen aufweisen;
wobei das Reagenzkit mindestens zwei Transposase-Erkennungssequenzen umfasst und jede Transposase-Erkennungssequenz eine unterschiedliche erste Markierungssequenz aufweist.

10. Kit nach Anspruch 9, ferner umfassend Reagenzien zur Herstellung von Transkriptom-Sequenzierbibliotheken.

11. Verfahren nach Anspruch 1 zur Herstellung von Nukleinsäurebibliotheken oder zur Durchführung einer Transkriptom-Sequenzierung.

## Revendications

1. Procédé de génération de molécules d'acide nucléique marquées, comprenant les étapes suivantes:
(a) la fourniture de multiples particules de billes comprenant des molécules oligonucléotidiques couplées, dans lequel lesdites molécules oligonucléotidiques contiennent une séquence de marquage comprenant une deuxième séquence d'étiquette, dans lequel chaque particule de bille comporte de multiples molécules oligonucléotidiques, et les multiples molécules oligonucléotidiques sur une même particule de bille ont la même deuxième séquence d'étiquette, tandis que les molécules oligonucléotidiques sur différentes particules de billes ont des deuxièmes séquences d'étiquette différentes;
(b) la fourniture de multiples cellules ou noyaux cellulaires comprenant de l'ARN;
(c) la réalisation d'un traitement sur l'ARN à l'intérieur des cellules ou des noyaux cellulaires, comprenant une étape de transcription inverse pour former des acides nucléiques double brin contenant des chaînes d'ADNc;
(d) l'incubation des acides nucléiques double brin avec un complexe de transposase comprenant une transposase et une séquence transposon que la transposase est utilisée pour reconnaître et lier, et étant capable de cliver ou de couper les acides nucléiques double brin, dans lequel la séquence transposon contient un brin transféré et un brin non transféré, le brin transféré comprenant une séquence de reconnaissance de transposase, une première séquence d'étiquette et une première séquence commune, dans lequel la première séquence d'étiquette est située en amont de la séquence de reconnaissance de transposase, et la première séquence commune est située en amont de la première séquence d'étiquette, et dans lequel l'incubation est réalisée dans des conditions qui permettent aux acides nucléiques double brin d'être fragmentés par le complexe de transposase en fragments d'acide nucléique et à la séquence transposon d'être connectée à l'extrémité des fragments d'acide nucléique, formant ainsi un groupe de fragments d'acide nucléique à l'intérieur des cellules ou des noyaux cellulaires, les fragments d'acide nucléique comprenant des fragments d'ADNc dans lesquels les séquences des brins transférés sont connectées à l'extrémité 5' des fragments d'ADNc et les fragments d'acide nucléique contiennent, de l'extrémité 5' à l'extrémité 3', la première séquence commune, la première séquence d'étiquette, la séquence de reconnaissance de transposase et le fragment d'ADNc ; et
(e) la mise en contact des cellules ou noyaux cellulaires traités de l'étape (d) avec les particules de billes, dans laquelle les fragments d'acide nucléique et les molécules oligonucléotidiques génèrent des molécules d'acide nucléique marquées comprenant, de l'extrémité 5' à l'extrémité 3', la séquence des fragments d'acide nucléique et la séquence complémentaire de la séquence de marquage, ou comprenant la séquence de marquage et la séquence complémentaire des fragments d'acide nucléique.

2. Procédé selon la revendication 1, dans lequel, à l'étape (e), au moins 2 cellules ou noyaux cellulaires sont fournis.

3. Procédé selon la revendication 1, dans lequel les cellules sont dérivées d'animaux, de plantes ou de micro-organismes, ou de toute combinaison de ceux-ci.

4. Procédé selon la revendication 1, dans lequel l'ARN est soumis à une transcription inverse à l'aide d'une transcriptase inverse pour former un acide nucléique double brin hybride comprenant des chaînes d'ARN et d'ADNc.

5. Procédé selon la revendication 1, dans lequel, à l'étape (e), les fragments d'acide nucléique sont mis en contact avec les molécules oligonucléotidiques par des moyens choisis parmi les suivants:
(e1) la libération des fragments d'acide nucléique par traitement des cellules ou noyaux cellulaires traités;
(e2) la libération des molécules oligonucléotidiques des billes ; ou
(e3) une combinaison de (e1) et (e2).

6. Procédé de construction d'une banque d'acides nucléiques comprenant:
(i) la génération de multiples molécules d'acide nucléique marquées selon la revendication 1, et
(ii) la récupération et/ou la combinaison de multiples molécules d'acide nucléique marquées pour obtenir une banque de molécules d'acide nucléique.

7. Procédé selon la revendication 6, dans lequel le procédé comprend en outre : (iii) l'enrichissement des molécules d'acide nucléique marquées.

8. Procédé de séquençage d'acides nucléiques à partir de cellules ou de noyaux cellulaires, comprenant:
la construction d'une banque d'acides nucléiques selon la revendication 6; et
le séquençage de la banque d'acides nucléiques.

9. Kit réactif comprenant: une transcriptase inverse, une transposase, et une séquence de reconnaissance de transposase que la transposase peut reconnaître et à laquelle elle peut se lier, dans lequel la transposase et la séquence de reconnaissance de transposase peuvent former un complexe de transposase, lequel est capable de cliver ou de couper un acide nucléique double brin,
la séquence transposon contient un brin transféré et un brin non transféré, le brin transféré comprenant une séquence de reconnaissance de transposase, une première séquence d'étiquette et une première séquence commune, une première séquence d'étiquette et une première séquence commune, la première séquence d'étiquette étant située en amont de la séquence de reconnaissance de transposase, et la première séquence commune étant située en amont de la première séquence d'étiquette,
kit réactif comprend en outre des billes couplées à de multiples molécules oligonucléotidiques, et les molécules oligonucléotidiques contiennent une séquence de marquage, la séquence de marquage comprenant une deuxième séquence d'étiquette, dans lequel chaque bille comporte de multiples molécules oligonucléotidiques, et les multiples molécules oligonucléotidiques sur une même bille ont la même deuxième séquence d'étiquette, tandis que les molécules oligonucléotidiques sur différentes billes ont des deuxièmes séquences d'étiquette différentes;
dans lequel le kit réactif comprend au moins 2 séquences de reconnaissance de transposase et chaque séquence de reconnaissance de transposase a une première séquence d'étiquette différente.

10. Kit selon la revendication 9, comprenant en outre des réactifs pour la construction de banques de séquençage du transcriptome.

11. Procédé selon la revendication 1 pour la construction de banques d'acides nucléiques ou pour la réalisation d'un séquençage du transcriptome.
